(19) Europäisches Patentamt
European Patent Office
Office européen des brevets

(11) **EP 2 335 599 A1**

(12) **EUROPEAN PATENT APPLICATION**
published in accordance with Art. 153(4) EPC

(43) Date of publication:
**22.06.2011 Bulletin 2011/25**

(21) Application number: **09773162.4**

(22) Date of filing: **29.06.2009**

(51) Int Cl.:
*A61B 10/00* (2006.01)       *A61B 5/00* (2006.01)
*G06Q 50/00* (2006.01)

(86) International application number:
**PCT/JP2009/003002**

(87) International publication number:
**WO 2010/001584 (07.01.2010 Gazette 2010/01)**

(84) Designated Contracting States:
**AT BE BG CH CY CZ DE DK EE ES FI FR GB GR HR HU IE IS IT LI LT LU LV MC MK MT NL NO PL PT RO SE SI SK TR**
Designated Extension States:
**AL BA RS**

(30) Priority: **30.06.2008 JP 2008171810**

(71) Applicant: **Sysmex Corporation**
**Kobe-shi, Hyogo 651-0073 (JP)**

(72) Inventor: **TAKAHATA, Takayuki**
**Kobe-shi**
**Hyogo 651-0073 (JP)**

(74) Representative: **HOFFMANN EITLE**
**Patent- und Rechtsanwälte**
**Arabellastraße 4**
**81925 München (DE)**

(54) **SUPPORTING SYSTEM FOR INSULIN RESISTANCE EVALUATION, METHOD FOR SUPPORTING INSULIN RESISTANCE EVALUATION AND COMPUTER PROGRAM**

(57)     The present invention provides an insulin resistance evaluation supporting system, an insulin resistance evaluation supporting method, and a computer program that can reduce the burden on a subject necessary to estimate biological information relating to the insulin resistance compared with that in conventional examples. An insulin resistance evaluation supporting system 1 includes an input/output interface 11f that receives input of information relating to a glucose concentration, an insulin concentration, and a free fatty acid concentration in blood obtained by measuring the subject, a CPU 11a that estimates a glucose uptake rate of the subject based on the information relating to the glucose concentration, the insulin concentration, and the free fatty acid concentration in the blood whose input has been received, and an image output interface 11g that outputs the estimated glucose uptake rate.

FIG. 1

EP 2 335 599 A1

# EP 2 335 599 A1

**Description**

Technical Field

The present invention relates to an insulin resistance evaluation supporting system that supports evaluation of the insulin resistance of a subject, a method thereof, and a computer program for causing a computer to function as the insulin resistance evaluation supporting system.

Background Art

Insulin resistance is one of the disease states of diabetes, and is known as an important background factor of metabolic syndrome. Conventionally, a glucose clamp test is used for evaluating insulin resistance. The glucose clamp test is a test that injects insulin to a vein of a subject using an artificial pancreas, and regulates the rate of glucose injected so as to keep the blood glucose level constant. This test method is highly invasive to a subject and places a heavy burden on the subject.
Patent Document 1 discloses a diagnosis supporting system that receives "fasting insulin level", "blood glucose level at 2 hours after metal", "HOMA-IR", and "insulin OGTT peak level" as input values, performs a peripheral insulin resistance determining process for obtaining scores by comparing these respective input values with pre-set determination reference values, and thus analyzes the risk of contracting diabetes and metabolic syndrome.

Citation List

Patent Document

Patent Document 1: JP 2006-304833A

Summary of Invention

Problem to be Solved by the Invention

However, the diagnosis supporting system described in Patent Document 1 requires test results of an oral glucose tolerance test (OGTT: Oral Glucose Tolerance Test) as input information. The OGTT is a test that causes a subject to orally ingest dextrose, and collects blood several times after a predetermined period of time to measure the blood glucose level and the blood insulin concentration, that is, the burden of this test on a subject is lighter than that of glucose clamp, but the test takes several hours.
The present invention was made in view of these circumstances, and it is a primary object thereof to provide an insulin resistance evaluation supporting system, an insulin resistance evaluation supporting method, and a computer program that can solve the above-described problems.

Means for Solving Problem

In order to solve the above-described problems, an aspect of the present invention is directed to an insulin resistance evaluation supporting system for supporting evaluation of insulin resistance of a subject, comprising: an input means that receives input of information relating to a glucose concentration, an insulin concentration, and a free fatty acid concentration in blood obtained by measuring the subject; and an estimating means that estimates a glucose uptake rate of the subject based on the information relating to the glucose concentration, the insulin concentration, and the free fatty acid concentration in the blood whose input has been received by the input means.
In this case, it is preferable that the estimating means comprises: a first estimating means that estimates a value according to a glucose transporter appearance amount based on the information relating to the insulin concentration and the free fatty acid concentration in the blood; and a second estimating means that estimates a glucose uptake rate based on the value according to the glucose transporter appearance amount estimated by the first estimating means and the glucose concentration in the blood.
Furthermore, in this case, it is preferable that the first estimating means comprise a third estimating means that estimates a fatty acyl-coenzyme A complex concentration based on the information relating to the free fatty acid concentration in the blood; and a fourth estimating means that estimates the value according to the glucose transporter appearance amount based on the insulin concentration in the blood and the fatty acyl-coenzyme A complex concentration estimated by the first estimating means.
Furthermore, in this case, it is preferable that the estimating means further comprises a fifth estimating means

2

that estimates an intracellular pyruvic acid concentration based on the glucose uptake rate estimated by the second estimating means; a sixth estimating means that estimates an intracellular acetyl coenzyme concentration based on the fatty acyl-coenzyme A complex concentration estimated by the third estimating means and the pyruvic acid concentration estimated by the fifth estimating means and an adjusting means that adjusts a production rate of an acetyl coenzyme produced from a fatty acyl-coenzyme A complex based on the acetyl coenzyme concentration estimated by the sixth estimating means; and the sixth estimating means again estimates the acetyl coenzyme concentration based on the production rate of the acetyl coenzyme after the adjustment by the adjusting means.

Furthermore, in this case, it is preferable that the input means further receives input of information relating to a muscle amount of the subject, the second estimating means estimates a glucose uptake rate per unit muscle amount of the subject based on the value according to the glucose transporter appearance amount estimated by the first estimating means and the glucose concentration in the blood, and the estimating means estimates a glucose uptake rate per unit weight of the subject based on the glucose uptake rate per unit muscle amount of the subject estimated by the second estimating means and the information relating to the muscle amount of the subject whose input has been received by the input means.

Furthermore, in this case, it is preferable that the estimating means repeats a process that, in production of a plurality of substances relating to a change from glucose to pyruvic acid in a living body, acquires amounts of the substances produced in a specific period of time based on concentrations of the substances before the production, acquires concentrations of the substances after the specific period of time by reflecting the amounts of the respective substances produced on the concentrations of the substances before the production, and estimates the glucose uptake rate based on the acquired concentrations of the substances.

Furthermore, in this case, it is preferable that the estimating means determines whether or not the glucose uptake rate reaches a steady state, and repeats the process that acquires the concentrations of the substances until it is determined that the glucose uptake rate reaches a steady state.

Furthermore, in this case, it is preferable that the input means receives input of information relating to a glucose concentration, an insulin concentration, and a free fatty acid concentration in blood in a fasted state of the subject, and the estimating means comprises: a first glucose uptake rate estimating means that acquires concentrations of the substances in the fasted state based on the information relating to the glucose concentration, the insulin concentration, and the free fatty acid concentration in the blood whose input has been received by the input means, and estimates a glucose uptake rate in the fasted state based on the acquired concentrations of the substances, and a second glucose uptake rate estimating means that acquires concentrations of the substances when the insulin concentration is at a predetermined value, based on the concentrations of the substances in the fasted state acquired by the first glucose uptake rate estimating means, and estimates a glucose uptake rate when the insulin concentration is at the predetermined value, based on the acquired concentrations of the substances.

Furthermore, in this case, it is preferable that the insulin resistance evaluation supporting system further comprises an output means that outputs the glucose uptake rate estimated by the estimating means, or that the insulin resistance evaluation supporting system further comprises: an insulin resistance estimating means that estimates insulin resistance of the subject based on information relating to the glucose uptake rate estimated by the estimating means; and an output means that outputs a result of the estimation by the insulin resistance estimating means.

Moreover, an aspect of the present invention is directed to an insulin resistance evaluation supporting method for supporting evaluation of insulin resistance of a subject using a computer provided with an input device, comprising the steps of receiving, using the input device, input of information relating to a glucose concentration, an insulin concentration, and a free fatty acid concentration in blood obtained by measuring the subject; and estimating, using the computer, a glucose uptake rate of the subject based on the information relating to the glucose concentration, the insulin concentration, and the free fatty acid concentration in the blood whose input has been received using the input device.

In this case, it is preferable that the step of estimating a glucose uptake rate of the subject comprises the steps of: estimating, using the calculating portion, a value according to a glucose transporter appearance amount based on the information relating to the insulin concentration and the free fatty acid concentration in the blood; and estimating, using the computer, a glucose uptake rate based on the estimated value according to the glucose transporter appearance amount and the glucose concentration in the blood.

Moreover, an aspect of the present invention is directed to a computer program for causing a computer provided with an input device to execute the steps of receiving, using the input device, input of information relating to a glucose concentration, an insulin concentration, and a free fatty acid concentration in blood obtained by measuring a subject; and estimating a glucose uptake rate of the subject based on the glucose concentration, the insulin concentration, and the free fatty acid concentration in the blood whose input has been received using the input means and the glucose concentration in the blood of the subject.

In this case, it is preferable that, in the step of estimating a glucose uptake rate of the subject, the computer is further caused to execute the steps of: estimating a value according to a glucose transporter appearance amount based on the information relating to the insulin concentration and the free fatty acid concentration in the blood; and estimating a glucose uptake rate based on the estimated value according to the glucose transporter appearance amount and the glucose concentration in the blood.

Effect of the Invention

With the insulin resistance evaluation supporting system, the insulin resistance evaluation supporting method, and the computer program according to the present invention, the burden on a subject necessary to estimate biological information relating to the insulin resistance can be made lighter than that in conventional techniques.

Brief Description of Drawings

FIG. 1 is a block diagram showing the configuration of an insulin resistance evaluation supporting system according to Embodiment 1.
FIG. 2 is a conceptual diagram showing a functional configuration of the insulin resistance evaluation supporting system according to Embodiment 1.
FIG. 3 is a conceptual diagram showing a virtual substance reaction flow in the insulin resistance evaluation supporting system according to Embodiment 1.
FIG. 4 is a flowchart illustrating a processing flow of an insulin resistance evaluation supporting program according to Embodiment 1.
FIG. 5 is a schematic diagram showing an exemplary input screen of the insulin resistance evaluation supporting system according to Embodiment 1.
FIG. 6 is a flowchart illustrating the procedure or a first glucose uptake rate estimating process in the insulin resistance evaluation supporting system according to Embodiment 1.
FIG. 7 is a flowchart illustrating the procedure of a second glucose uptake rate estimating process in the insulin resistance evaluation supporting system according to Embodiment 1.
FIG. 8 is a schematic diagram showing an exemplary output screen in the insulin resistance evaluation supporting system according to Embodiment 1.
FIG. 9 is a block diagram showing the configuration of an insulin resistance evaluation supporting system according to Embodiment 2.
FIG. 10 is a conceptual diagram showing a virtual substance reaction flow in the insulin resistance evaluation supporting system according to Embodiment 2.
FIG. 11 is a flowchart illustrating a processing flow of an insulin resistance evaluation supporting program according to Embodiment 2.
FIG. 12 is a schematic diagram showing an exemplary input screen in the insulin resistance evaluation supporting system according to Embodiment 2.
FIG. 13 is a flowchart illustrating the procedure of a first glucose uptake rate estimating process in the insulin resistance evaluation supporting system according to Embodiment 2.
FIG. 14 is a flowchart illustrating the procedure of a second glucose uptake rate estimating process in the insulin resistance evaluation supporting system according to Embodiment 2.
FIG. 15 is a diagram showing a simulation result display screen in an evaluation experiment by the insulin resistance evaluation supporting system according to Embodiment 2.
FIG. 16 is a graph showing simulation results of the blood glucose concentration in the evaluation experiment by the insulin resistance evaluation supporting system according to Embodiment 2.
FIG. 17 is a graph showing simulation results of the blood insulin concentration in the evaluation experiment by the insulin resistance evaluation supporting system according to Embodiment 2.
FIG. 18 is a graph showing simulation results of the blood fatty acid concentration in the evaluation experiment by the insulin resistance evaluation supporting system according to Embodiment 2.
FIG. 19 is a graph showing simulation results of the glucose uptake rate in the evaluation experiment by the insulin resistance evaluation supporting system according to Embodiment 2.
FIG. 20 is a graph showing a result of comparison between measured values in a document and simulation results.

Description of Embodiments

Hereinafter, preferred embodiments of the present invention will be described with reference to the drawings.

Embodiment 1

This embodiment relates to an insulin resistance evaluation supporting system that receives input of information relating to a glucose concentration, an insulin concentration, and a free fatty acid concentration in blood obtained by measuring a subject, and an amount of oxygen consumed and an amount of carbon dioxide produced per unit time in skeletal muscle, estimates a glucose uptake rate of the subject based on the information relating to the glucose concentration, the insulin concentration, and the free fatty acid concentration in the blood, and the amount of oxygen consumed and the amount of carbon dioxide produced per unit time in the skeletal muscle whose input has been received, estimates insulin resistance of the subject based on the estimated glucose uptake rate, and outputs a result of the estimation.

Configuration of the Insulin Resistance Evaluation Supporting System

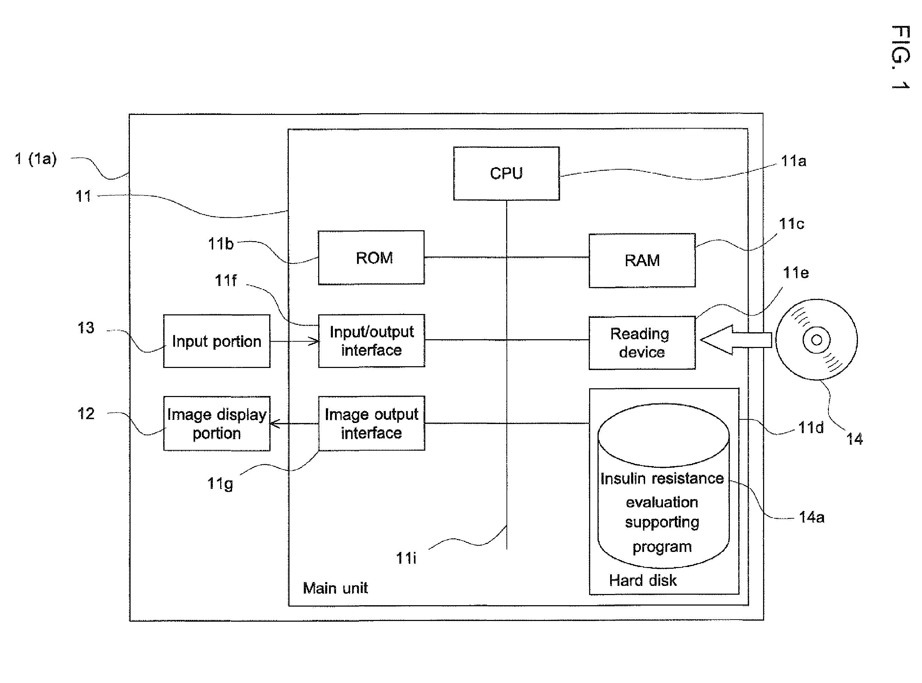

FIG. 1 is a block diagram showing the configuration of an insulin resistance evaluation supporting system according to this embodiment. An insulin resistance evaluation supporting system 1 according to this embodiment is realized as a computer 1a. As shown in FIG. 1, the computer 1a is provided with a main unit 11, an image display portion 12, and an input portion 13. The main unit 11 is provided with a CPU 11a a ROM 11b, a RAM 11c, a hard disk 11d, a reading device 11e, an input/output interface 11f, and an image output interface 11g. The CPU 11a, the ROM 11b, the RAM 11c, the hard disk 11d, the reading device 11c, the input/output interface 11f, and the image output interface 11g are connected to each other via a bus 11i.

The CPU 11a can execute computer programs loaded into the RAM 11c. Execution of an insulin resistance evaluation supporting program 14a as described later by the CPU 11a allows the computer 1a to function as the insulin resistance evaluation supporting system 1.

The ROM 11b is configured from a mask ROM, a PROM, an EPROM, an EEPROM, or the like, and stores computer programs that are to be executed by the CPU 11a, data used for the execution, and the like.

The RAM 11c is configured from an SRAM, a DRAM, or the like. The RAM 11c is used to read the insulin resistance evaluation supporting program 14a stored in the hard disk 11d. Furthermore, the RAM 11c is used as a work area of the CPU 11a when the CPU 11a executes a computer program.

On the hard disk 11d, various computer programs (e.g., operating systems and application programs) that are to be executed by the CPU 11a and data used for the execution of the computer programs are installed. The insulin resistance evaluation supporting program 14a described later is also installed on the hard disk 11d.

The reading device 11e is configured from a flexible disk drive, a CD-ROM drive, a DVD-ROM drive, or the like, and can read computer programs or data stored in a portable storage medium 14. Furthermore, the portable storage medium 14 stores the insulin resistance evaluation supporting program 14a for causing a computer to function as an insulin resistance evaluation supporting system. The computer 1a can read the insulin resistance evaluation supporting program 14a from the portable storage medium 14, and install the insulin resistance evaluation supporting program 14a on the hard disk 11d.

Here, the insulin resistance evaluation supporting program 14a does not necessarily have to be provided by the portable storage medium 14, and can be provided through an electric telecommunication line (it may be either wired or wireless) from an external apparatus communicably connected via the electric telecommunication line to the computer 1a. For example, it is possible that the insulin resistance evaluation supporting program 14a is stored in a hard disk of a server computer on the Internet, and the computer 1a accesses the server computer to download and install the computer program on the hard disk 11d.

Furthermore, on the hard disk 11d, for example, a multi-task operating system such as Windows (registered trademark) manufactured and marketed by Microsoft Corporation (U.S.) is installed. In the following description, it is assumed that the insulin resistance evaluation supporting program 14a according to this embodiment operates on the operating system. The configuration of the insulin resistance evaluation supporting program 14a will be described later in detail.

The input/output interface 11 is configured from, for example, a serial interface such as a USB, an IEEE 1394, or an RS-232C, a parallel interface such as an SCSI, an IDE, or an IEEE 1284, an analog interface such as a D/A converter or an AD converter. The input/output interface 11f is connected to the input portion 13 including a keyboard and a mouse, and a user can input data to the computer 1a using the input portion 13.

The image output interface 11g is connected to the image display portion 12 configured from an LCD, a CRT, or the like, and outputs video signals according to image data given from the CPU 11a to the image display portion 12. The image display portion 12 displays images (screens) according to the input video signals.

Functional Configuration of the Insulin Resistance Evaluation Supporting System

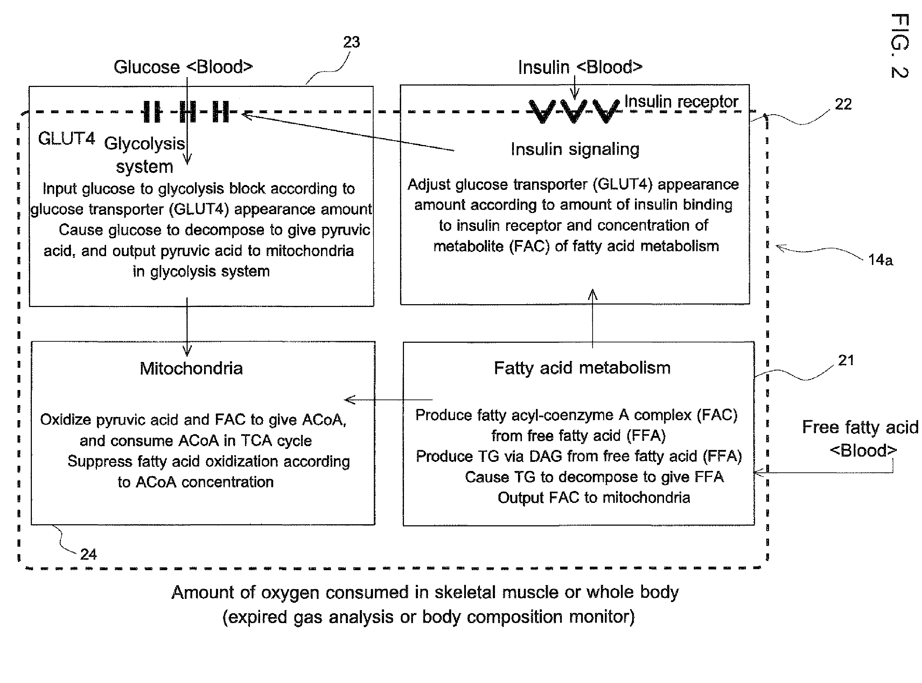

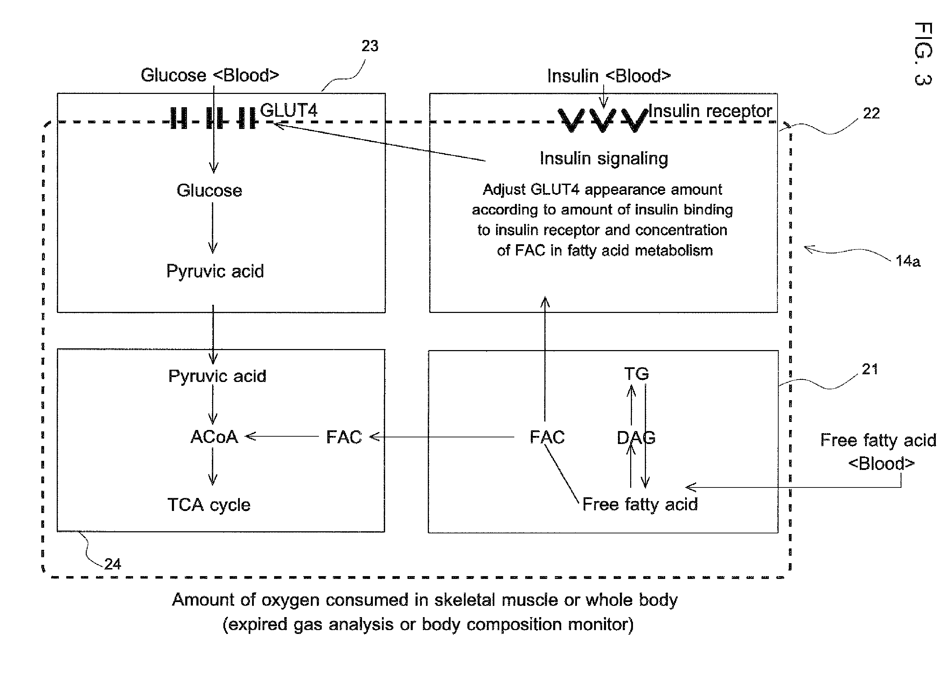

Next, the insulin resistance evaluation supporting program 14a will be described in more detail. FIG. 2 is a conceptual diagram showing a functional configuration of the insulin resistance evaluation supporting system realized by the insulin resistance evaluation supporting program 14a, and FIG. 3 is a conceptual diagram showing a virtual substance reaction flow in the insulin resistance evaluation supporting system. The insulin resistance evaluation supporting program 14a receives input of biological information including the body weight of a subject, the amount of oxygen consumed per unit time in the skeletal muscle, the amount of carbon dioxide produced, the skeletal muscle percentage, and the fasting blood glucose concentration, plasma insulin concentration, and blood free fatty acid concentration, and outputs an estimated glucose uptake rate (glucose uptake) in the peripheral tissues (the skeletal muscle) of the subject. The insulin resistance evaluation supporting program 14a includes four blocks 21 to 24 that virtually reproduce functions obtained by dividing a living organ (functions) relating to glucose uptake in the body according to functions. Each of the blocks 21 to 24 includes a plurality of parameters, and is configured so as to calculate the reaction rates of the production of substances relating to glucose uptake. Furthermore, the insulin resistance evaluation supporting program 14a is configured so as to calculate the production rates (the amounts produced in a specific period of time) of substances relating to glucose uptake from the reaction rates, and to calculate the substance concentrations after the specific period of time reflecting the amounts produced after the specific period of time obtained by this calculation.

Hereinafter, the configuration of each block will be described in detail, but, prior to this description, the concept of an ordinary metabolism rate functioning as the basis of the calculation in each block will be described. An ordinarily irreversible enzyme reaction in a living organ is represented by the following formula.

$$X + Y + E_1 \rightarrow V + W + E_2 \dots (1)$$

In the formula, X and Y refer to the concentrations of substrates metabolized, V and W refer to the concentrations of substrates produced, and $E_1$ and $E_2$ refer two at and ADP, or ADP and ATP, and/or NADH and NAD, or NAD and NADH.

The enzyme reaction represented by Formula (1) can also be represented by Formula (2) below.

$$X+Y \underset{}{\overset{E_1 \quad E_2}{\rightleftharpoons}} V+W \qquad \cdots (2)$$

The reaction rate $f_{X+Y \rightarrow V+W}$ in the above-described enzyme reaction can be obtained by Formula (3) below.

$$f_{X+Y \rightarrow V+W} = V_{X+Y \rightarrow V+W} \cdot \frac{PS^{\pm}}{\mu^{\pm} + PS^{\pm}} \cdot \frac{RS^{\pm}}{v^{\pm} + RS^{\pm}} \cdot \frac{C_X/K_X \cdot C_Y/K_Y}{1 + C_X/K_X + C_Y/K_Y + C_X/K_X \cdot C_Y/K_Y} \qquad \cdots (3)$$

In the formula, $V_{X+Y \rightarrow V+W}$ refers to the maximum saturation rate, Cx and $C_Y$ refer to the concentrations of the substrates X and Y, $PS^+$ refers to ATP/ADP, $PS^-$ refers to ADP/ATP, $RS^+$ refers to NADH/NAD, $RS^-$ refers to NAD/NADH, and Kx, $K_Y$, $\mu^{\pm}$, and $v^{\pm}$ refer to a Michaelis-Menten constant or a model parameter relating to metabolic control in the reaction process.

Here, it is assumed that the reaction rate, the production rate, and the concentration of a substance in the following description of the blocks 21 to 24 and the supplemental calculation process are the reaction rate (mmol/kg/min), the production rate (mmol/kg/min), and the concentration (mM) per unit weight, respectively, unless otherwise indicated.

Fatty Acid Metabolism Block

The fatty acid metabolism block 21 is a functional block virtually reproducing a function of metabolizing fatty acid in a living organ. The fatty acid metabolism function of the body causes blood free fatty acid (FFA) to be taken up into the cell, produces triglyceride (TG) via diacylglycerol (DAG), and produces a fatty acyl-coenzyme A complex (FAC) from the free fatty acid. The fatty acid metabolism block 21 represents such a fatty acid metabolism function of the body. Execution of the fatty acid metabolism block 21 by the CPU 11a allows the reaction rate $f_{FFA \rightarrow TG}$ of converting free fatty acid into triglyceride, the reaction rate $f_{FFA \rightarrow FAC}$ of producing a fatty acyl-coenzyme A complex from free fatty acid, and the reaction rate $f_{TG \to FFA}$ of producing free fatty acid from triglyceride to be calculated based on the intracellular free fatty acid concentration, and the intracellular free fatty acid concentration, triglyceride concentration, and fatty acyl-coenzyme A complex concentration after a specific period of time to be calculated.

Hereinafter, a chemical reaction relating to the fatty acid metabolism function of the living organ and a specific calculation process of the fatty acid metabolism block 21 based thereon will be described. Here, the intracellular concentrations of free fatty acid, triglyceride, ATP, ADP a fatty acyl-coenzyme A complex, and phosphoric acid in the following formulae are each provided with predetermined initial values. The initial value is used in the first calculation, and an updated value is used in the subsequent calculations. First, blood free fatty acid is taken up into a cell, and the passive inflow rate $f_{FFA}$ of free fatty acid from blood into a tissue (cell) at that time is represented by Formula (4) below.

$$f_{FFA} = \frac{f_{O2} + f_{CO2}}{7} \quad \cdots (4)$$

In the formula, $f_{O2}$ refers to the rate of oxygen consumed in a tissue, and is obtained from the amount of oxygen consumed per unit time in the skeletal muscle and the skeletal muscle percentage that have been input. Furthermore, $f_{CO2}$ refers to the rate of carbon dioxide produced in the tissue, and is obtained from the amount of carbon dioxide produced per unit time in the skeletal muscle and the skeletal muscle percentage that have been input.

The above-described inflow rate (rate of uptake into a tissue) $f_{FFA}$ of free fatty acid can also be represented by Formula (5) below.

$$f_{FFA} = \lambda_{FFA} \left( C_{FFAb} + \sigma_{FFA} \cdot C_{FFA} \right) \quad \cdots (5)$$

In the formula, $C_{FFAb}$ refers to the blood free fatty acid concentration, $C_{FFA}$ refers to the intracellular free fatty acid concentration, $\lambda_{FFA}$ refers to a membrane transport coefficient of FFA having membrane permeability, and $\sigma_{FFA}$ refers to a distribution coefficient relating to FFA. If the CPU 11a applies $f_{FFA}$ obtained by Formula (4) and the input blood free fatty acid concentration to Formula (5), the intracellular free fatty acid concentration FFA is calculated.

Next, the free fatty acid (FFA) taken up into the cell is converted via diacylglycerol to triglyceride (TG).

$$3FFA + 6ATP \to TG + 6ADP \dots (6)$$

The rate of this reaction is represented by Formula (7) below.

$$f_{FFA \to TG} = V_{FFA \to TG} \cdot \frac{PS^+}{\mu^+ + PS^+} \cdot \frac{C_{FFA}/K_{FFA}}{1 + C_{FFA}/K_{FFA}} \quad \cdots (7)$$

Furthermore, a fatty acyl-coenzyme A complex (FAC) is produced from free fatty acid (FFA) and coenzyme A (CoA).

$$FFA + CoA + 2ATP \to FAC + 2ADP + 2Pi \dots (8)$$

The rate of this reaction is represented by Formula (9) below.

$$f_{FFA \to FAC} = V_{FFA \to FAC} \cdot \frac{PS^+}{\mu^+ + PS^+} \cdot \frac{C_{FFA}/K_{FFA} \cdot C_{CoA}/K_{CoA}}{1 + C_{FFA}/K_{FFA} + C_{CoA}/K_{CoA} + C_{FFA}/K_{FFA} \cdot C_{CoA}/K_{CoA}} \quad \cdots (9)$$

Moreover, triglyceride decomposes to give free fatty acid as shown in Formula (10) below.

$$TG \rightarrow 3FFA \dots (10)$$

The rate of this reaction is represented by Formula (11) below.

$$f_{TG \rightarrow FFA} = V_{TG \rightarrow FFA} \cdot \frac{C_{TG}/K_{TG}}{1 + C_{TG}/K_{TG}} \qquad \cdots (11)$$

The fatty acyl-coenzyme A complex produced by the reaction of Formula (8) above is given to mitochondria. Furthermore, the concentration information of the fatty acyl-coenzyme A complex is updated as below.
In the fatty acid metabolism block 21, the CPU 11a calculates the reaction rates $f_{FFA \rightarrow TG}$, $f_{FFA \rightarrow FAC}$, and $f_{TG \rightarrow FFA}$ represented by Formulae (7), (9), and (11) above, respectively.
Furthermore, in the fatty acid metabolism block 21, the CPU 11a calculates the production rate of free fatty acid represented by Formula (12) below, the production rate of triglyceride represented by Formula (13) below, and the production rate of fatty acyl-coenzyme A complex represented by Formula (14) below.

$$V \cdot \frac{dC_{FFA}}{dt} = f_{FFA} - f_{FFA \rightarrow FAC} - f_{FFA \rightarrow TG} + f_{TG \rightarrow FFA} \qquad \cdots (12)$$

$$V \cdot \frac{dC_{TG}}{dt} = f_{FFA \rightarrow TG} - f_{TG \rightarrow FFA} \qquad \cdots (13)$$

$$V \cdot \frac{dC_{FAC}}{dt} = f_{FFA \rightarrow FAC} - f_{FAC \rightarrow ACbA} \qquad \cdots (14)$$

In Formula (12), the reaction rate $f_{FAC \rightarrow ACoA}$ is represented by Formula (30) described later, and calculated in the mitochondria block 24. If no calculation has been performed in the mitochondria block 24, the initial value of the reaction rate $f_{FAC \rightarrow ACoA}$ is used.
Furthermore, in the fatty acid metabolism block 21, the CPU 11a calculates the amounts of free fatty acid, triglyceride, and fatty acyl-coenzyme A complex produced in a specific period of time respectively from the thus obtained rates of free fatty acid, triglyceride, and fatty acyl-coenzyme A complex produced, and reflects these amounts on the free fatty acid concentration, the triglyceride concentration, and the fatty acyl-coenzyme A complex concentration at that time, thereby calculating the intracellular free fatty acid concentration, triglyceride concentration, and fatty acyl-coenzyme A complex concentration after the specific period of time. Here, in this embodiment, the specific period of time is a constant, but this value may be set by the user.
Furthermore, as described later, the fatty acid metabolism block 21 is used both in a first glucose uptake rate estimating process and in a second glucose uptake rate estimating process. In the first glucose uptake rate estimating process, the fatty acid metabolism block 21 is used to perform a process that calculates the intracellular free fatty acid concentration, triglyceride, concentration, and fatty acyl-coenzyme A complex concentration in a fasted state. Furthermore, in the second glucose uptake rate calculating process, the fatty acid metabolism block 21 is used to perform a process that calculates the intracellular concentrations of the substances in a hyperinsulinemic state.

Insulin Signaling Block

The insulin signaling block 22 is a functional block virtually reproducing a function of adjusting the glucose transporter (GLUT4) appearance amount in a living organ. The function of adjusting the GLUT4 appearance amount in a living body adjusts the GLUT4 appearance amount according to the amount of insulin binding to insulin receptors and the concentrations of fatty acyl-coenzyme A complex and diacylglycerol formed as metabolites of the fatty acid metabolism. The insulin signaling block 22 according to this embodiment represents such a function of adjusting the GLUT4 appearance amount in the body. Execution of the insulin signaling block 22 by the CPU 11a allows a value according to the GLUT4 appearance amount to be calculated based on the plasma insulin concentration (PI) and the FAC concentration.

Hereinafter, a specific calculation process of the insulin signaling block 22 based on the function of adjusting the GLUT4 appearance amount in the living organ will be described. The function of adjusting the GLUT4 appearance amount in the living organ is characterized in that, as the plasma insulin concentration is increased, the GLUT4 appearance amount is increased, and, as the FAC concentration and the DAG concentration are increased, the GLUT4 appearance amount is suppressed. In consideration of these characteristics, in the insulin signaling block 22, a glucose uptake (GLUT), which is a value according to the GUT4 appearance amount, is calculated following Formula (15) below using the input plasma insulin concentration and the FAC concentration obtained through the calculation in the fatty acid metabolism block 21. Here, in this embodiment, the glucose uptake is calculated without consideration of the DAG concentration.

$$GLUT = V_{\max} \cdot \frac{C_{PI}^{nPI}}{K_{PI}^{nPI} + C_{PI}^{nPI}} \cdot \frac{K_{FAC}^{nFAC}}{K_{FAC}^{nFAC} + C_{FAC}^{nFAC}} \qquad \cdots \ (15)$$

In the formula, $V_{\max}$ refers to a predetermines coefficient, and nPI and nFAC refer to constants. Here, the glucose uptake GLUT is a real number proportional to the GLUT4 appearance amount.

Furthermore, as described later, the insulin signaling block 22 is used both in the first glucose uptake rate estimating process and in the second glucose uptake rate estimating process. In the first glucose uptake rate estimating process, the insulin signaling block 22 is used to perform a process that calculates the glucose uptake GLUT in a fasted state. Furthermore, in the second glucose uptake rate calculating process, the insulin signaling block 22 is used to perform a process that calculates the glucose uptake GLUT in a hyperinsulinemic state.

Glycolysis Block

The glycolysis block 23 is a functional block virtually reproducing a function of causing glucose to decompose in a living organ. The glucose decomposition function of the body causes glucose to be taken up into the cell according to the GLUT4 appearance amount and causes the glucose in the cell to decompose to give pyruvic acid via G6P (glucose 6-phosphate) and GA3P (glyceraldehyde 3-phosphate). The glycolysis block 23 represents such a glucose decomposition function of the body. Execution of the glycolysis block 23 by the CPU 11a allows the glucose uptake rate to be calculated based on the glucose uptake GLUT obtained in the insulin signaling block 22 and the rate of oxygen consumed and the rate of carbon dioxide produced in the tissue, and the reaction rate $f_{GLU \to G6P}$ of converting glucose into G6P, the reaction rate $f_{G6P \to GA3P}$ of converting G6P into GA3P, and the reaction rate $f_{GA3P \to PYR}$ of converting GA3P into pyruvic acid to be calculated. Furthermore, the glucose concentration, the G6P concentration, the GA3P concentration, and the pyruvic acid concentration after a specific period of time are calculated based on these reaction rates.

Hereinafter, a chemical reaction relating to the glucose decomposition function of the living organ and a specific calculation process of the glycolysis block 23 based thereon will be described. Here, the intracellular concentrations of glucose, G6P, GA3P, pyruvic acid, NAD, and NADH, in the following formulae are each provided with predetermined initial values. The initial value is used in the first calculation, and an updated value is used in the subsequent calculations. First, blood glucose is taken up into the cell according to the GLUT4 appearance amount (appearance amount on the cell surface). This uptake rate $f_{GLU}$ is represented by Formula (16) below.

$$f_{GLU} = GLUT \left( C_{GLUb} + \sigma_{GLU} \cdot C_{GLU} \right) \qquad \cdots \ (16)$$

In the formulae, $C_{GLUb}$ refers to the input blood glucose concentration, $C_{GLU}$ refers to the intracellular glucose concentration, and $\sigma_{GLU}$ refers to a distribution coefficient relating to glucose.

The glucose (GLU) taken up into the cell is phosphorylated to give G6P.

$$GLU + ATP \rightarrow G6P + ADP \ldots (17)$$

The rate of this reaction is represented by Formula (18) below.

$$f_{GLU \rightarrow G6P} = V_{GLU \rightarrow G6P} \cdot \frac{PS^+}{\mu^+ + PS^+} \cdot \frac{C_{GLU}/K_{GLU}}{1 + C_{GLU}/K_{GLU}} \qquad \cdots (18)$$

Furthermore, the G6P produced by the conversion reacts withATP to give GA3P and ADP as shown in the following formula.

$$G6P + ATP \rightarrow 2GA3P + ADP \ldots (19)$$

The rate of this reaction is represented by Formula (20) below.

$$f_{G6P \rightarrow GA3P} = V_{G6P \rightarrow GA3P} \cdot \frac{(PS^+)^\gamma}{(\mu^+)^\gamma + (PS^+)^\gamma} \cdot \frac{C_{G6P}/K_{G6P}}{1 + C_{G6P}/K_{G6P}} \qquad \cdots (20)$$

The GA3P produced by the conversion is converted to pyruvic acid (PYX) according to the following formula.

$$GA3P + Pi + NAD + 2ADP \rightarrow PYR + NADH + 2ATP \ldots (21)$$

The reaction rate of the conversion to PYR is represented by Formula (22) below.

$$f_{GA3P \rightarrow PYR} = V_{GA3P \rightarrow PYR} \cdot \frac{RS^-}{\nu^- + RS^-} \cdot \frac{C_{GA3P}/K_{GA3P} \cdot C_{Pi}/K_{Pi}}{1 + C_{GA3P}/K_{GA3P} + C_{Pi}/K_{Pi} + C_{GA3P}/K_{GA3P} \cdot C_{Pi}/K_{Pi}} \qquad \cdots (22)$$

In the glycolysis block 23, the CPU 11a calculates the reaction rates $f_{GLU \rightarrow G6P}$, $f_{G6P \rightarrow GA3P}$, and $f_{GA3P \rightarrow PYR}$ represented by Formulae (18), (20), and (22) above, respectively

Furthermore, in the glycolysis block 23, the CPU 11a calculates the production rate of glucose represented by Formula (23) below, the production rate of G6P represented by Formula (24) below, the production rate of GA3P represented by Formula (25) below, and the production rate of pyruvic acid represented by Formula (26) below.

$$V \cdot \frac{dC_{GLU}}{dt} = f_{GLU} - f_{GLU \rightarrow G6P} \qquad \cdots (23)$$

$$V \cdot \frac{dC_{G6P}}{dt} = f_{GLU \rightarrow G6P} - f_{G6P \rightarrow GA3P} \qquad \cdots (24)$$

$$V \cdot \frac{dC_{GA3P}}{dt} = 2f_{G6P \rightarrow GA3P} - f_{GA3P \rightarrow PYR} \qquad \cdots (25)$$

$$V \cdot \frac{dC_{PYR}}{dt} = f_{GA3P \rightarrow PYR} - f_{PYR \rightarrow ACoA} \qquad \cdots (26)$$

In Formula (26), the reaction rate $f_{PYR \rightarrow ACoA}$ is represented by Formula (28) described later, and calculated in the mitochondria block 24. If no calculation has been performed in the mitochondria block 24, the initial value of the reaction rate $f_{PYR \rightarrow ACoA}$ is used.

Furthermore, in the glycolysis block 23, the CPU 11a calculates the amounts of glucose, G6P, GA3P, and pyruvic acid produced in a specific period of time respectively from the thus obtained rates of glucose, G6P, GA3P, and pyruvic acid produced, and reflects these amounts on the intracellular glucose concentration, G6P concentration, GA3P concentration, and pyruvic acid concentration at that time, thereby calculating the intracellular glucose concentration, G6P concentration, GA3P concentration, and pyruvic acid concentration after the specific period of time.

Furthermore, as described later, the glycolysis block 23 is used both in the first glucose uptake rate estimating process and in the second glucose uptake rate estimating process. In the first glucose uptake rate estimating process, the glycolysis block 23 is used to perform a process that calculates the intracellular glucose concentration, G6P concentration, GA3P concentration, and pyruvic acid concentration in a fasted state. Furthermore, in the second glucose uptake rate calculating process, the glycolysis block 23 is used to perform a process that calculates the intracellular concentrations of the substances in a hyperinsulinemic state.

Mitochondria Block

The mitochondria block 24 is a functional block virtually reproducing a function of mitochondria in a living organ. The mitochondria produce acetyl coenzyme A (ACoA) by oxidizing the pyruvic acid and the fatty acyl-coenzyme A complex, and consume the acetyl coenzyme A in the TCA cycle. Fatty acid oxidization is suppressed according to the acetyl coenzyme A concentration. The mitochondria block 24 represents such a mitochondria function. Execution of the mitochondria block 24 by the CPU 11a allows the reaction rate $f_{PYR \rightarrow ACoA}$ of producing acetyl coenzyme A from the pyruvic acid obtained in the glycolysis block 23, the reaction rate $f_{FAC \rightarrow ACoA}$ of producing acetyl coenzyme A from the fatty acyl-coenzyme A complex obtained in the fatty acid metabolism block 21, the reaction rate $f_{ACoA \rightarrow CO2}$ of producing carbon dioxide from the acetyl coenzyme A, and the reaction rate $f_{O2 \rightarrow H2O}$ of consuming oxygen and producing water to be calculated. Furthermore, the intracellular concentrations of acetyl coenzyme A, coenzyme A, oxygen, and carbon dioxide after a specific period of time are calculated based on these reaction rates.

Hereinafter, a chemical reaction relating to the mitochondria and a specific calculation process of the mitochondria block 24 based thereon will be described. Here, the intracellular concentrations of acetyl coenzyme A, coenzyme A, oxygen, and carbon dioxide in the following formulae are each provided with predetermined initial values. The initial value is used in the first calculation, and an updated value is used in the subsequent calculations.

First, intracellular pyruvic acid is oxidized to give acetyl coenzyme A.

$$\mathrm{PYR + CoA + NAD \rightarrow ACoA + NADH + CO_2} \dots (27)$$

The rate of this reaction is represented by Formula (28) below.

$$f_{PYR \rightarrow ACoA} = V_{PYR \rightarrow ACoA} \cdot \frac{RS^-}{v^- + RS^-} \cdot \frac{C_{PYR}/K_{PYR} \cdot C_{CoA}/K_{CoA}}{1 + C_{PYR}/K_{PYR} + C_{CoA}/K_{CoA} + C_{PYR}/K_{PYR} \cdot C_{CoA}/K_{CoA}} \qquad \cdots (28)$$

Furthermore, the fatty acyl-coenzyme A complex produced by the fatty acid metabolism function is oxidized to give acetyl coenzyme A as shown in Formula (29) below. This route is called β-oxidization.

$$\mathrm{FAC + 7CoA + \frac{35}{3}NAD \rightarrow 8ACoA + \frac{35}{3}NADH} \qquad \cdots (29)$$

The rate of this reaction is represented by Formula (30) below.

$$f_{FAC \to ACoA} = V_{FAC \to ACoA} \cdot \frac{RS^-}{v^- + RS^-} \cdot \frac{K_{MCoA}^{nMCoA}}{K_{MCoA}^{nMCoA} + C_{MCoA}^{nMCoA}} \cdot \frac{C_{FAC}/K_{FAC} \cdot C_{CoA}/K_{CoA}}{1 + C_{FAC}/K_{FAC} + C_{CoA}/K_{CoA} + C_{FAC}/K_{FAC} \cdot C_{CoA}/K_{CoA} + C_{ACoA}/K_{ACoA}}$$

$$\cdots (30)$$

In the formula, $C_{MCoA}$ refers to the malonyl-CoA concentration, and is obtained by multiplying the ACoA concentration $C_{ACoA}$ by a predetermined coefficient. In this manner, in the β-oxidization, the rate of acetyl coenzyme A produced is suppressed due to the malonyl-CoA concentration. Accordingly, the rate of acetyl-CoA produced in the β-oxidization is adjusted due to the malonyl-CoA concentration, Here, in the first calculation of Formula (30), a predetermined initial value of $C_{MCoA}$ is used, and, in the second and subsequent calculations, a value of $C_{MCoA}$ obtained through the calculation is used.

Furthermore, the fatty acyl-coenzyme A complex is metabolized in the TCA cycle, and ATP and NADH are newly produced.

$$ACoA + ADP + Pi + 4NAD \to 2CO_2 + CoA + ATP + 4NADH \ldots (31)$$

The rate of this reaction is represented by Formula (32) below.

$$f_{ACoA \to CO2} = V_{ACoA \to CO2} \cdot \frac{PS^-}{\mu^- + PS^-} \cdot \frac{RS^-}{v^- + RS^-} \cdot \frac{C_{ACoA}/K_{ACoA} \cdot C_{Pi}/K_{Pi}}{1 + C_{ACoA}/K_{ACoA} + C_{Pi}/K_{Pi} + C_{ACoA}/K_{ACoA} \cdot C_{Pi}/K_{Pi}} \quad \cdots (32)$$

Meanwhile, the relationship between the consumption of NADH, oxygen, and ADP and the synthesis of ATP in the mitochondria is represented by Formula (33) below.

$$O_2 + 6ADP + 6Pi + 2NADH \to 2H_2O + 6ATP + 2NAD \ldots (33)$$

The rate of this reaction is represented by Formula (34) below.

$$f_{O2 \to H2O} = V_{O2 \to H2O} \cdot \frac{PS^-}{\mu^- + PS^-} \cdot \frac{RS^+}{v^+ + RS^+} \cdot \frac{C_{O2}/K_{O2} \cdot C_{Pi}/K_{Pi}}{1 + C_{O2}/K_{O2} + C_{Pi}/K_{Pi} + C_{O2}/K_{O2} \cdot C_{Pi}/K_{Pi}} \quad \cdots (34)$$

In the mitochondria block 24, the CPU 11a calculates the reaction rates $f_{PYR \to ACoA}$, $f_{FAC \to ACoA}$, $f_{ACoA \to CO2}$, and $f_{O2 \to H2O}$ represented by Formulae (28), (30), (32), and (34) above, respectively.

As described above, the rate $f_{O2}$ of oxygen consumed in the cell is determined by the amount of oxygen consumed per unit time in the skeletal muscle and the skeletal muscle percentage that have been input, but can also be represented by Formula (35) below.

$$f_{O2} = \lambda_{O2}(C_{O2b} + \sigma_{O2} \cdot C_{O2}) \quad \cdots (35)$$

In the formula, $C_{O2b}$ refers to the blood oxygen concentration (constant), $C_{O2}$ refers to the intracellular oxygen concentration, $\lambda_{O2}$ refers to a membrane transport coefficient of oxygen having membrane permeability, and $\sigma_{O2}$ refers to a distribution coefficient relating to oxygen. The CPU 11a can obtain the intracellular oxygen concentration from Formula (35) above.

In a similar manner, as described above, the rate $f_{CO2}$ of carbon dioxide produced in the cell is determined by the amount of carbon dioxide produced per unit time in the skeletal muscle and the skeletal muscle percentage that have been input, but can also be represented by the following formula.

$$f_{CO2} = \lambda_{CO2}\left(C_{CO2b} + \sigma_{CO2} \cdot C_{CO2}\right) \qquad \cdots \ (36)$$

In the formula, $C_{CO2b}$ refers to the blood carbon dioxide concentration (constant), $C_{CO2}$ refers to the intracellular carbon dioxide concentration, $\lambda_{CO2}$ refers to a membrane transport coefficient of carbon dioxide having membrane permeability, and $\sigma_{CO2}$ refers to a distribution coefficient relating to carbon dioxide. The CPU 11a obtains the intracellular carbon dioxide concentration from Formula (36) above.

Furthermore, in the mitochondria block 24, the CPU 11a calculates the production rate of acetyl coenzyme A represented by Formula (37) below and the production rate of coenzyme A represented by Formula (38) below.

$$V \cdot \frac{dC_{ACoA}}{dt} = f_{PYR \to ACoA} + f_{FAC \to ACoA} - f_{ACoA \to CO2} \qquad \cdots \ (37)$$

$$V \cdot \frac{dC_{CoA}}{dt} = f_{ACoA \to CO2} - f_{PYR \to ACoA} - 8f_{FAC \to ACoA} \qquad \cdots \ (38)$$

Furthermore, in the mitochondria block 24, the CPU 11a calculates the amounts of oxygen, carbon dioxide, acetyl coenzyme A, and coenzyme A produced (consumed) in a specific period of time respectively from the thus obtained rates of oxygen, carbon dioxide, acetyl coenzyme A, and coenzyme A produced (consumed), and reflects these amounts on the free fatty acid concentration, the triglyceride concentration, and the fatty acyl-coenzyme A complex concentration at that time, thereby calculating the intracellular oxygen concentration, carbon dioxide concentration, acetyl coenzyme A concentration, and coenzyme A concentration after the specific period of time.

Furthermore, as described later, the mitochondria block 24 is used both in the first glucose uptake rate estimating process and in the second glucose uptake rate estimating process. In the first glucose uptake rate estimating process, the mitochondria block 24 is used to perform a process that calculates the intracellular oxygen concentration, carbon dioxide concentration, acetyl coenzyme A concentration, and coenzyme A concentration in a fasted state. Furthermore, in the second glucose uptake rate calculating process, the mitochondria block 24 is used to perform a process that calculates the intracellular concentrations of the substances in a hyperinsulinemic state.

Supplemental Calculation Process

Furthermore, the CPU 11a performs a supplemental calculation process as described below.

When the ADP concentration is high, phosphocreatine (PCr) and ADP react with each other to give creatine (Cr) and ATP.

$$PCr + ADP \to Cr + ATP \ldots (39)$$

The rate of this reaction is represented by Formula (40) below.

$$f_{PCr \to Cr} = V_{PCr \to Cr} \cdot \frac{PS^-}{\mu^- + PS^-} \cdot \frac{C_{PCr}/K_{PCr}}{1 + C_{PCr}/K_{PCr}} \qquad \cdots \ (40)$$

Meanwhile, as the ATP concentration is increased, creatine and ATP react with each other to give phosphocreatine and ADP.

$$Cr + ATP \to PCr + ADP \ldots (41)$$

The rate of this reaction is represented by Formula (42) below.

$$f_{G \to PCr} = V_{G \to PCr} \cdot \frac{PS^+}{\mu^+ + PS^+} \cdot \frac{C_G/K_G}{1 + C_G/K_G} \qquad \cdots (42)$$

Furthermore, ATP is hydrolyzed and converted to ADP according to the following formula.

$$ATP \to ADP + Pi \dots (43)$$

The rate of this reaction is represented by Formula (44) below.

$$f_{ATP \to ADP} = V_{ATP \to ADP} \cdot \frac{C_{ATP}/K_{ATP}}{1 + C_{ATP}/K_{ATP}} \qquad \cdots (44)$$

In the supplemental calculation process, the CPU 11a calculates the reaction rates $f_{PCr \to Cr}$, $f_{Cr \to PCr}$, and $f_{ATP \to ADP}$ represented by Formulae (40), (42), and (44) above, respectively.
Furthermore, the CPU 11a calculates the production rates of NAD, NADH, ATP, ADP, Pi, PCr, and Cr according to Formulae (45) to (51) below.

$$V \cdot \frac{dC_{NAD}}{dt} = 2f_{O2 \to H2O} - f_{GA3P \to PYR} - f_{PYR \to ACoA} - 4f_{ACoA \to CO2} - \frac{35}{3} f_{FFA \to ACoA} \qquad \cdots (45)$$

$$V \cdot \frac{dC_{NADH}}{dt} = f_{GA3P \to PYR} + f_{PYR \to ACoA} + 4f_{ACoA \to CO2} + \frac{35}{3} f_{FFA \to ACoA} - 2f_{O2 \to H2O} \qquad \cdots (46)$$

$$V \cdot \frac{dC_{ATP}}{dt} = 2f_{GA3P \to PYR} + f_{ACoA \to CO2} + 6f_{O2 \to H2O} + f_{PCr \to Cr} - f_{GLU \to G6P} - f_{G6P \to GA3P}$$
$$- 2f_{FFA \to FAC} - 2f_{FFA \to TG} - f_{Cr \to PCr} - f_{ATP \to ADP} \qquad \cdots (47)$$

$$V \cdot \frac{dC_{ADP}}{dt} = f_{GLU \to G6P} + f_{G6P \to GA3P} + 2f_{FFA \to FAC} + 2f_{FFA \to TG} + f_{Cr \to PCr} + f_{ATP \to ADP}$$
$$- 2f_{GA3P \to PYR} - f_{ACoA \to CO2} - 6f_{O2 \to H2O} - f_{PCr \to Cr} \qquad \cdots (48)$$

$$V \cdot \frac{dC_{Pi}}{dt} = 2f_{FFA \to FAC} + \frac{7}{3} f_{FFA \to TG} + f_{ATP \to ADP} - f_{GA3P \to PYR} - f_{ACoA \to CO2} - 6f_{O2 \to H2O} \qquad \cdots (49)$$

$$V \cdot \frac{dC_{PCr}}{dt} = f_{Cr \to PCr} - f_{PCr \to Cr} \qquad \cdots (50)$$

$$V \cdot \frac{dC_{O}}{dt} = f_{PO \to O} - f_{O \to PO} \qquad \cdots (51)$$

Furthermore, the CPU 11a calculates the amounts of NAD, NADH, ATP, ADP, Pi, PCr, and Cr produced in a specific period of time respectively from the thus obtained rates of NAD, NADH, ATP, ADP Pi, PCr, and Cr produced, and reflects these amounts on the intracellular concentrations of NAD, NADH, ATP, ADP, Pi, PCr, and Cr at that time, thereby calculating the intracellular concentrations of NAD, NADH, ATP, ADP, Pi, PCr, and Cr after the specific period of time.

Furthermore, as described later, the supplemental calculation process is used both in the first glucose uptake rate estimating process and in the second glucose uptake rate estimating process. In the first glucose uptake rate estimating process, the supplemental calculation process is executed to perform a process that calculates the intracellular concentrations of NAD, NADH, ATP, ADP, Pi, PCr, and Cr in a fasted state. Furthermore, in the second glucose uptake rate calculating process, the supplemental calculation process is executed to perform a process that calculates the intracellular concentrations of the substances in a hyperinsulinemic state.

Operation of the Insulin Resistance Evaluation Supporting System

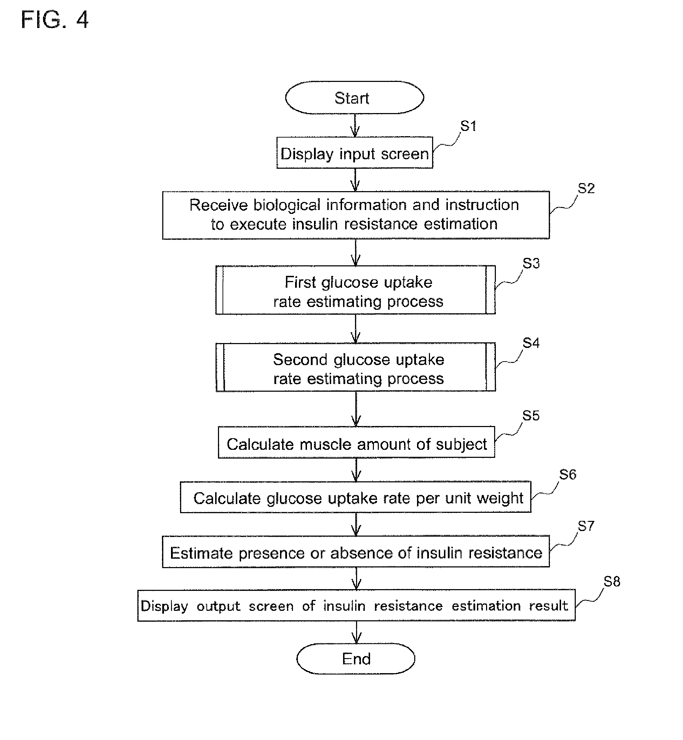

Next, an operation of the insulin resistance evaluation supporting system 1 according to this embodiment will be described. FIG. 4 is a flowchart illustrating a processing flow of the insulin resistance evaluation supporting program according to this embodiment. The computer 1a operates as below by executing the insulin resistance evaluation supporting program 14a. First, the body weight of a subject is weighed in advance with a scale, the amount of oxygen consumed per unit time in the skeletal muscle is measured with a body composition monitor or expired gas analysis, the skeletal muscle percentage is measured with the body composition monitor, and a blood test and an expired gas test are performed. Thus, the biological information of the subject including the body weight, the amount of oxygen consumed and the amount of carbon dioxide produced per unit time in the skeletal muscle (hereinafter, referred to as an "amount of oxygen consumed" and an "amount of carbon dioxide produced"), the skeletal muscle percentage, the blood glucose concentration, the plasma insulin concentration, and the blood free fatty acid concentration is obtained. Here, the blood glucose concentration, the plasma insulin concentration, and the blood free fatty acid concentration all measured in a fasted state are used. The biological information is given in advance to a user such as a doctor or an operator who uses the insulin resistance evaluation supporting system.

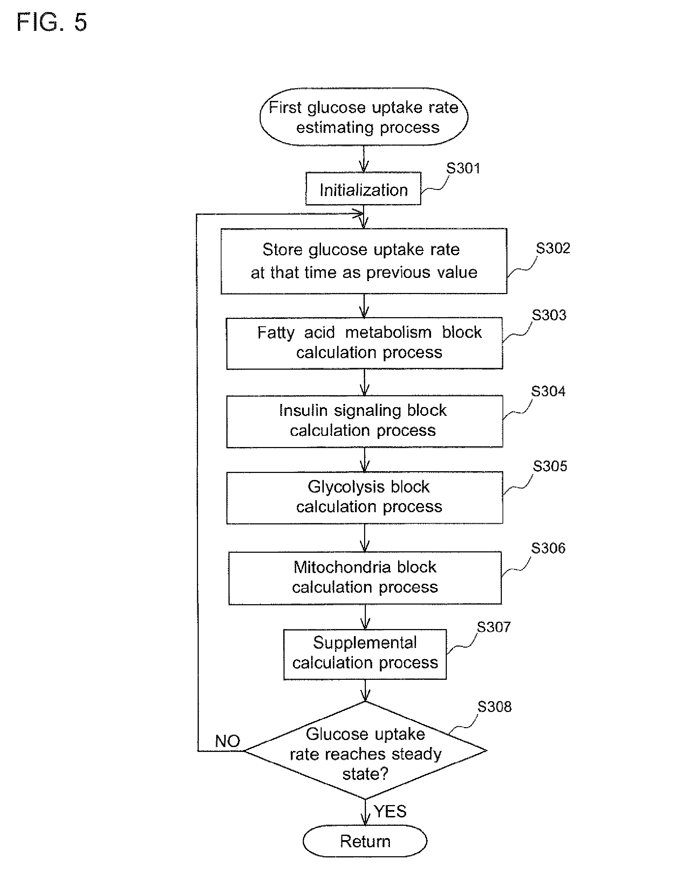

After the insulin resistance evaluation supporting program 14a is started, first, the CPU 11a displays an input screen for prompting the user to input the body weight, the amount of oxygen consumed, the amount of carbon dioxide produced, the skeletal muscle percentage, the blood glucose concentration, the plasma insulin concentration, and the blood free fatty acid concentration of the subject (step S1). FIG. 5 is a schematic diagram showing an exemplary input screen. As shown in FIG. 5, an input screen 3 includes input areas 31 to 37 in which the body weight, the amount of oxygen consumed, the amount of carbon dioxide produced, the skeletal muscle percentage, the blood glucose concentration, the plasma insulin concentration, and the blood free fatty acid concentration of the subject are to be input, and an Execute button 38 with which an instruction is to be given to execute estimation of the insulin resistance after the biological information is input to the input areas 31 to 37. The user operates the input portion 13 to input the biological information including the body weight, the amount of oxygen consumed, the amount of carbon dioxide produced, the skeletal muscle percentage, the blood glucose concentration, the plasma insulin concentration, and the blood free fatty acid concentration of the subject to the input areas 31 to 37, and selects (clicks on) the Execute button 38 to give an instruction to execute estimation of the insulin resistance. The CPU 11a receives input of the biological information and the execution instruction from the user (step S2). When such biological information is input, the CPU 11a is interrupted, and the processes in step S3 and subsequent steps are called.

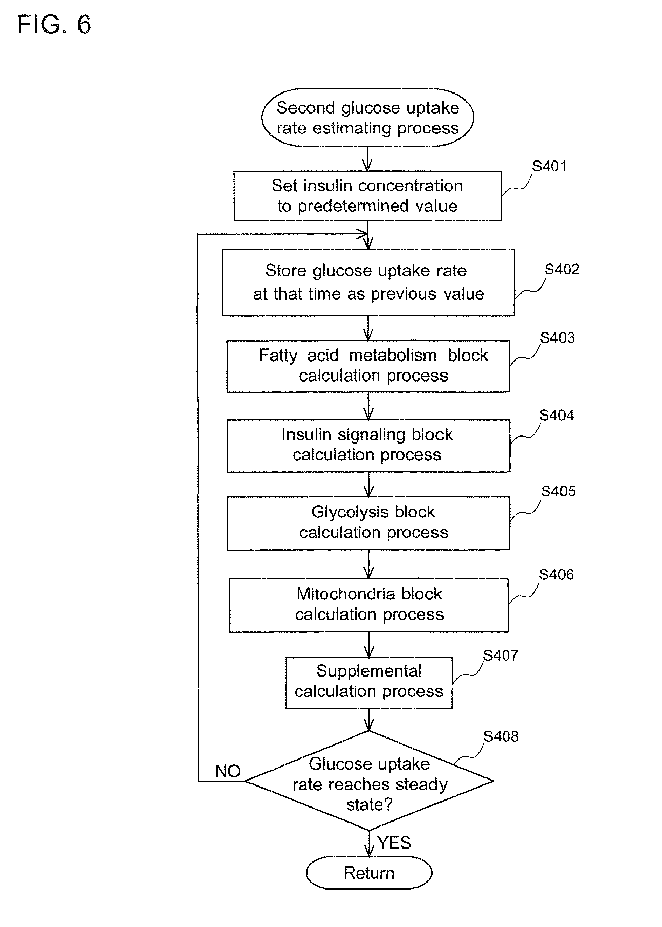

Once input of the biological information and the execution instruction is received from the user, the CPU 11a executes a first glucose uptake rate estimating process (step S3). FIG. 6 is a flowchart illustrating the procedure of the first glucose uptake rate estimating process. The first glucose uptake rate estimating process is a process that sequentially repeats steps S301 to S308, thereby estimating the fasting glucose uptake rate. First, the CPU 11a sets (initializes) the initial value to a variable (step S301). In this process, the initial values of the intracellular concentrations and the reaction rates of the above-described substances in a fasted state are stored in the RAM 11c, and the initial value 0 is stored in the RAM 11c as the glucose uptake rate. Next, the CPU 11a stores the glucose uptake rate at that time in the RAM 11c as the previous glucose uptake rate (step S302). Then, the CPU 11a sequentially executes a fatty acid metabolism block calculation process that is the calculation process in the fatty acid metabolism block 21 (step S303), an insulin signaling block calculation process that is the calculation process in the insulin signaling block 22 (step S304), a glycolysis block calculation process that is the calculation process in the glycolysis block 23 (step S305), a mitochondria block calculation process that is the calculation process in the mitochondria block 24 (step S306), and a supplemental calculation process (step S307). The substance concentrations, the reaction rates, the value according to the GLUT4 appearance amount, and the glucose uptake rate obtained in steps S303 to 307 are values in a fasted state.

Next, the CPU 11a determines whether or not the fasting glucose uptake rate obtained in the above-described process reaches a steady state (step S308). In this embodiment, this process is performed by obtaining a difference between the glucose uptake rate obtained in the current calculation (turn) and the glucose uptake rate in the previous calculation (turn) stored in the RAM 11c, and determining whether or not the difference is less than a first reference value (e.g., 0.1) for determining whether or not the glucose uptake rate reaches a steady state. If the difference between the current glucose uptake rate and the previous glucose uptake rate is less than the first reference value (YES in step S308), the CPU 11a returns the process to the call address of the first glucose uptake rate estimating process in the main routine, and, if the difference is at least the first reference value (NO in step S308), the CPU 11a repeats the processes in step S302 and subsequent steps.

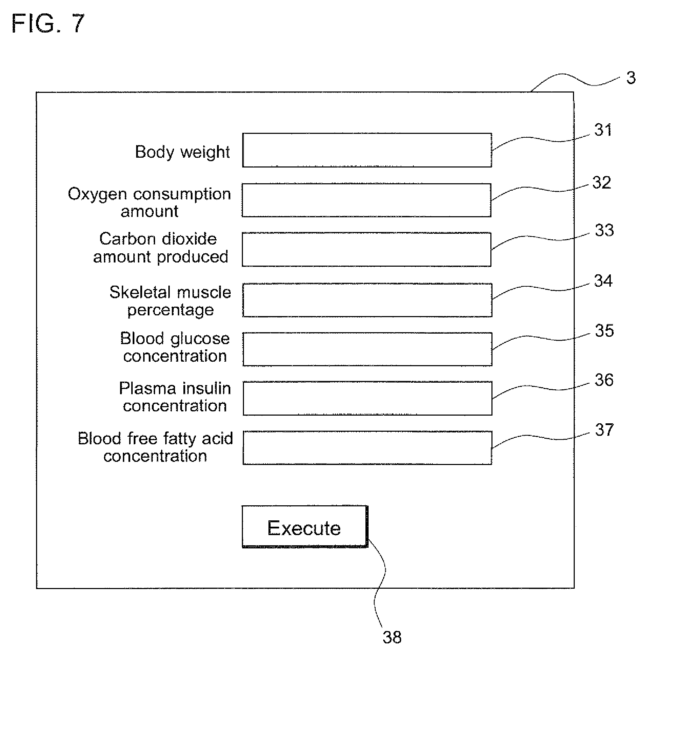

Next, the CPU 11a executes a second glucose uptake rate estimating process (step S4). FIG. 7 is a flowchart illustrating the procedure of the second glucose uptake rate estimating process. The second glucose uptake rate estimating process is a process that sequentially repeats steps S401 to S408, thereby estimating the glucose uptake rate in a hyperinsulinemic state. First, the CPU 11a sets the insulin concentration to a predetermined value (insulin concentration in a hyperinsulinemic state) (step S401). Here, in the second glucose uptake rate estimating process, as initial values of the fasting intracellular substance concentrations other than the insulin concentration, the reaction rates, and the glucose uptake (GLUT), the values finally obtained in the first glucose uptake rate estimating process, that is, the substance concentrations, the reaction rates, and the glucose uptake when the glucose uptake rate reaches the steady state are used as they are. As for the blood glucose concentration, the fasting glucose concentration used in the first glucose uptake rate estimating process is used as it is. Furthermore, the initial value 0 is stored in the RAM 11c as the glucose uptake rate. Next, the CPU 11a stores the glucose uptake rate at that time in the RAM 11c as the previous glucose uptake rate (step S402). Then, the CPU 11a sequentially executes a fatty acid metabolism block calculation process that is the calculation process in the fatty acid metabolism block 21 (step S403), an insulin signaling block calculation process that is the calculation process in the insulin signaling block 22 (step S404), a glycolysis block calculation process that is the calculation process in the glycolysis block 23 (step S405), a mitochondria block calculation process that is the calculation process in the mitochondria block 24 (step S406), and a supplemental calculation process (step S407). The substance concentrations, the reaction rates, the value according to the GLUT4 appearance amount, and the glucose uptake rate obtained in steps S403 to 407 are values in a hyperinsulinemic state.

Next, the CPU 11a determines whether or not the glucose uptake rate in a hyperinsulinemic state obtained in the above-described process reaches a steady state (step S408). In this embodiment, this process is performed by obtaining a difference between the glucose uptake rate obtained in the current calculation (turn) and the glucose uptake rate in the previous calculation (turn) stored in the RAM 11c, and determining whether or not the difference is less than the first reference value (e.g., 0.1) for determining whether or not the glucose uptake rate reaches a steady state. Here, the configuration is adopted in which, in the first glucose uptake rate estimating process and the second glucose uptake rate estimating process, the same first reference value is used to determine whether or not the glucose uptake rate reaches the steady state, but this is not a limitation, and different reference values may be used in these respective processes. Then, if the difference between the current glucose uptake rate and the previous glucose uptake rate is less than the first reference value (YES in step S408), the CPU 11a returns the process to the call address of the second glucose uptake rate estimating process in the main routine, and, if the difference is at least the first preference value (NO in step S408), the CPU 11a repeats the processes in step S402 and subsequent steps.

Next, the CPU 11a calculates the muscle amount of the subject from the input body weight and skeletal muscle percentage of the subject (step S5), and calculates the glucose uptake rate per unit weight in a hyperinsulinemic state by multiplying this muscle amount, the glucose distribution amount (constant), and the glucose concentration obtained in the second glucose uptake rate estimating process by the glucose uptake (GLUT) obtained in the second glucose uptake rate estimating process (step S6). Next, the CPU 11a estimates the presence or absence of the insulin resistance (step S7). This process is performed by determining whether or not the glucose uptake rate (estimated value) per unit weight obtained in step S6 is at least a second reference value (e.g., 12 mg/kg/min) for estimating the presence or absence of the insulin resistance. Accordingly, if the estimated glucose uptake rate is at least the second reference value, it is possible to estimate that the insulin resistance is not present, that is, the insulin sensitivity is present. Furthermore, if the estimated glucose uptake rate is less than the second reference value, it is possible to estimate that the insulin resistance is present, that is, the insulin sensitivity is not present. In this manner, the CPU 11a estimates the insulin resistance.

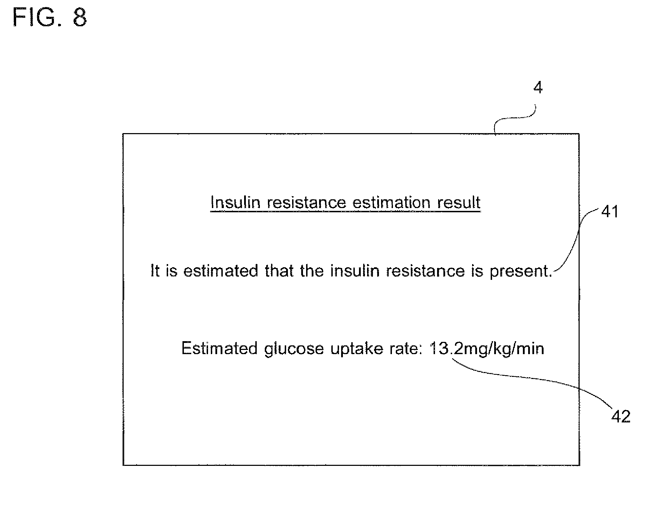

Next, the CPU 11a displays an output screen for outputting the estimation results of the insulin resistance (step S4). FIG. 8 is a schematic diagram showing an exemplary output screen. As shown in FIG. 8, an output screen 4 includes an insulin resistance estimation result 41 obtained in the above-described process and a finally obtained estimated glucose uptake rate per unit weight 42. With the output screen 4, the user is notified of the insulin resistance estimation result and the estimated glucose uptake rate. As the insulin resistance estimation result and the estimated glucose uptake rate are provided to the user in this manner, the user can use the information to perform evaluation of the insulin resistance.

With this sort of configuration, it is possible to estimate the presence or absence of the insulin resistance using biological information including "body weight", "amount of oxygen consumed per unit time in the skeletal muscle", "amount of carbon dioxide produced per unit time in skeletal muscle", "skeletal muscle percentage", "fasting blood glucose concentration", "fasting plasma insulin concentration", and "fasting blood free fatty acid concentration" that can be obtained with a simple test without placing a heavy burden on the subject, instead of requiring test results of a glucose clamp test and an oral glucose tolerance test that place a heavy burden on the subject. Among these pieces of input information, regarding the fasting blood glucose concentration and plasma insulin concentration, test values easily obtained with a blood test can be used as described above, but test values obtained with a glucose clamp test or an oral glucose tolerance test may also be used as the input information. However, this system is useful in that results of tests such as a glucose clamp test and an oral glucose tolerance test that place a heavy burden on the subject are not always required and results of a simple blood test can also be used instead of such test results, and in that information used as the input information can be obtained from any of the blood test, the glucose clamp test, and the oral glucose tolerance test.

Furthermore, suppression of the rate of acetyl-CoA produced in the $\beta$-oxidization due to the malonyl-CoA concentration is taken into consideration, and the rate of acetyl-CoA produced in the $\beta$-oxidization is adjusted due to the malonyl-CoA obtained based on the acetyl-CoA concentration, and the glucose metabolizing function in an actual living body is precisely reproduced. Also from this aspect, it can be expected to obtain estimated values precisely reflecting the actual glucose uptake rate.

Furthermore, based on the above-described biological information, the glucose metabolizing function in the actual living organ is virtually reproduced, and an estimated glucose uptake rate is obtained. Thus, it can be expected to obtain estimated values precisely reflecting the actual glucose uptake rate. Furthermore, in this embodiment, the glucose uptake rate in a hyperinsulinemic state is estimated, and this estimated value is output. In a glucose clamp test, glucose and insulin are injected to the subject such that the hyperinsulinemic state is maintained. Furthermore, the glucose uptake rates disclosed in documents and the like are normally values in a hyperinsulinemic state. Accordingly, by estimating a glucose uptake rate in a hyperinsulinemic state as described above and outputting this estimated value, the user can compare the estimated value with results of a glucose clamp test or values reported in documents and the like, and the insulin resistance of that subject can be easily evaluated.

Furthermore, in this embodiment, the glucose uptake rate per unit weight is estimated, and this estimated value is output. In a glucose clamp test, the glucose uptake rates per unit weight are obtained. Furthermore, the glucose uptake rates disclosed in documents and the like are normally values per unit weight. Accordingly, by estimating a glucose uptake rate per unit weight as described above and outputting this estimated value, the user can compare the estimated value with results of a glucose clamp test or values reported in documents and the like, and the insulin resistance of that subject can be easily evaluated.

Embodiment 2

This embodiment relates to an insulin resistance evaluation supporting system that receives input of information relating to a glucose concentration, an insulin concentration, and a free fatty acid concentration in blood obtained by measuring a subject, estimates a glucose uptake rate of the subject based on the information relating to the glucose concentration, the insulin concentration, and the free fatty acid concentration in the blood whose input has been received, estimates insulin resistance of the subject based on the estimated glucose uptake rate, and outputs a result of the estimation.

Configuration of the Insulin Resistance Evaluation Supporting System

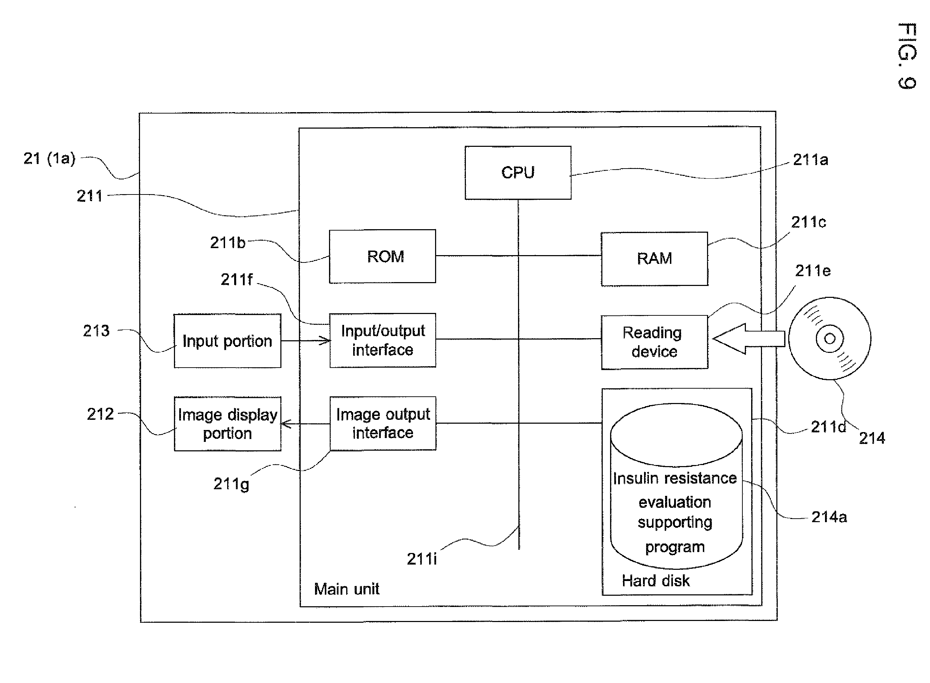

FIG. 9 is a block diagram showing the configuration of an insulin resistance evaluation supporting system according to this embodiment. An insulin resistance evaluation supporting system 201 according to this embodiment is realized as a computer 201a. As shown in FIG. 9, the computer 201a is provided with a main unit 211, an image display portion 212, and an input portion 213. The main unit 211 is provided with a CPU 211a, a ROM 211b, a RAM 211c, a hard disk 211d, a reading device 211e, an input/output interface 211f, and an image output interface 211g. The CPU 211a, the ROM 211b, the RAM 211c, the hard disk 211d, the reading device 211e, the input/output interface 211f, and the image output interface 211g are connected to each other via a bus 211i.

The CPU 211a can execute computer programs loaded into the RAM 211c. Execution of an insulin resistance evaluation supporting program 214a as described later by the CPU 211a allows the computer 201a to function as the insulin resistance evaluation supporting system 201.

The RAM 11c is configured from an SRAM, a DRAM, or the like. The RAM 11c is used to read the insulin resistance evaluation supporting program 214a stored in the hard disk 211d. Furthermore, the RAM 11c is used as a work area of the CPU 211a when the CPU 211a executes a computer program.

On the hard disk 211d, various computer programs (e.g., operating systems and application programs) that are to be executed by the CPU 21a and data used for the execution of the computer programs are installed. The insulin resistance evaluation supporting program 214a described later is also installed on the hard disk 211d.

The reading device 211e is configured from a flexible disk drive, a CD-ROM drive, a DVD-ROM drive, or the like, and can read computer programs or data stored in a portable storage medium 214. Furthermore, the portable storage medium 214 stores the insulin resistance evaluation supporting program 214a for causing a computer to function as an insulin resistance evaluation supporting system. The computer 201a can read the insulin resistance evaluation supporting program 214a from the portable storage medium 214, and install the insulin resistance evaluation supporting program 214a on the hard disk 211d.

Here, the insulin resistance evaluation supporting program 214a does not necessarily have to be provided by the portable storage medium 214, and can be provided through an electric telecommunication line (it may be either wired or wireless) from an external apparatus communicably connected via the electric telecommunication line to the computer 201a. For example, it is possible that the insulin resistance evaluation supporting program 214a is stored in a hard disk of a server computer on the Internet, and the computer 201a accesses the server computer to download and install the computer program on the hard disk 211d.

Furthermore, on the hard disk 211d, for example, a multi-task operating system such as Windows (registered trademark) manufactured and marketed by Microsoft Corporation (U.S.) is installed. In the following description, it is assumed that the insulin resistance evaluation supporting program 214a according to this embodiment operates on the operating system. The configuration of the insulin resistance evaluation supporting program 214a will be described later in detail.

Here, the other portions of the configuration of the computer 201a according to Embodiment 2 are the same as those of the configuration of the computer 1a according to Embodiment 1, and, thus, a description thereof is omitted.

Functional Configuration of the Insulin Resistance Evaluation Supporting System

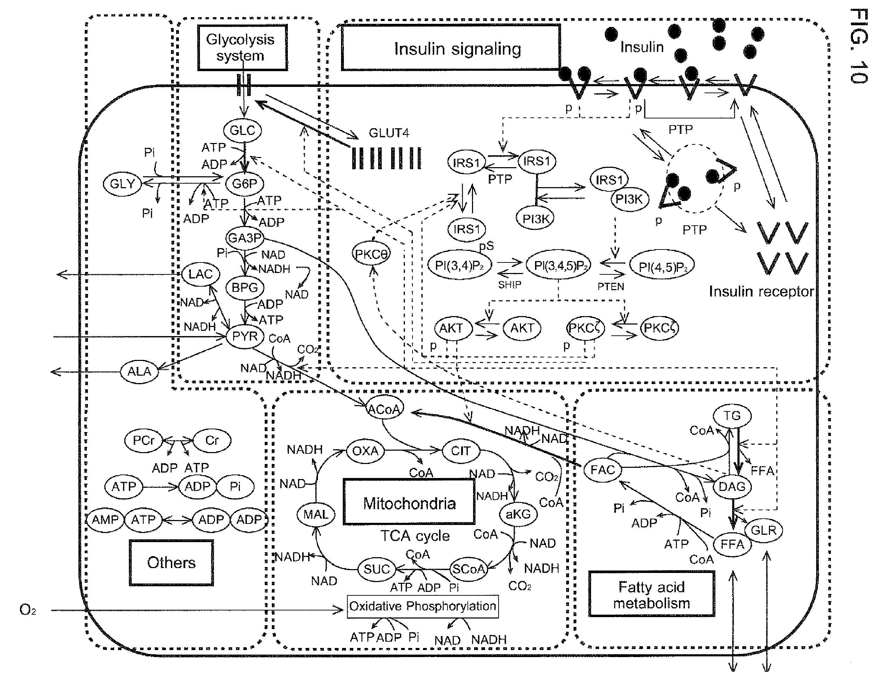

Next, the insulin resistance evaluation supporting program 214a will be described in more detail. FIG. 10 is a conceptual diagram showing a virtual substance reaction flow in the insulin resistance evaluation supporting system according to this embodiment. The insulin resistance evaluation supporting program 214a receives input of biological information including the body weight of a subject, the skeletal muscle percentage, and the fasting blood glucose concentration, plasma insulin concentration, and blood free fatty acid concentration, and outputs an estimated glucose uptake rate (glucose uptake) in the peripheral tissues (the skeletal muscle) of the subject. The insulin resistance evaluation supporting program 214a includes four blocks 221 to 224 that virtually reproduce functions obtained by dividing a living organ (functions) relating to glucose uptake in the body according to functions. Each of the blocks 221 to 224 includes a plurality of parameters, and is configured so as to calculate the reaction rates of the production of substances relating to glucose uptake. Furthermore, the insulin resistance evaluation supporting program 214a is configured so as to calculate the production rates (the amounts produced in a specific period of time) of substances relating to glucose uptake from the reaction rates, and to calculate the substance concentrations after the specific period of time reflecting the amounts produced after the specific period of time obtained by this calculation. Hereinafter, the configuration of each of the blocks 221 to 224 will be described in detail.

Fatty Acid Metabolism Block

The fatty acid metabolism block 221 is a functional block virtually reproducing a function of metabolizing fatty acid in a living organ such as muscle tissues or adipose tissues. The fatty acid metabolism function of the body causes blood free fatty acid to be taken up into the cell, produces fatty acyl-coenzyzne A complex (FAC) from the free fatty acid (FFA), and produces triglyceride (TG) via diacylglycerol (DG). The fatty acid metabolism block 221 represents such a fatty acid metabolism function of the body. Execution of the fatty acid metabolism block 221 by the CPU 11a allows the reaction rate $f_{GA3P \to DG}$ of producing DG from FAC and D-glyceraldehyde 3-phosphate (GA3P), the reaction rate $f_{DG \to TG}$ of producing TG from DG and FAC, the reaction rate $f_{TG \to DG}$ of producing DG and FFA from TG, the reaction rate $f_{DG \to FFA}$ of producing FFA and glycerol (GLR) from DG, and the reaction rate $f_{FFA \to FAC}$ of producing FAC from FFA to be calculated, and the intracellular concentrations of FFA, GLR, DG, TG, FAC and FAC after a specific period of time to be calculated.

Hereinafter, a chemical reaction relating to the fatty acid metabolism function of the living organ such as muscle tissues or adipose tissues and a specific calculation process of the fatty acid metabolism block 221 based thereon will be described. Here, the intracellular concentrations of FFA, FAC, DG, TG, GA3P, GLR, ATP, ADP, NAD, NADH, and inorganic phosphate (Pi) in the following formulae are each provided with predetermined initial values. The initial value is used in the first calculation, and an updated value is used in the subsequent calculations.

First, blood fatty acid (FFA) is taken up into a cell The uptake rate $f_{FFA}$ is represented by Formula (52) below.

$$f_{FFA} = Q \cdot \left( C_{FFA,b} - \sigma_{FFA} \cdot C_{FFA} \right) \quad \cdots (52)$$

In the formula, $C_{FFA,b}$ refers to the input blood fatty acid concentration, $C_{FFA}$ refers to the intracellular fatty acid concentration, $\sigma_{FFA}$ refers to a distribution coefficient relating to fatty acid, and Q refers to the blood flow rate in the muscle tissue.

Next, blood glycerol (GLR) is taken up into the cell. The uptake rate $f_{GLC}$ is represented by Formula (53) below.

$$f_{GLR} = Q \cdot \left( C_{GLR,b} - \sigma_{GLR} \cdot C_{GLR} \right) \quad \cdots (53)$$

In the formula, $C_{GLR,b}$ refers to the input blood glycerol concentration, $C_{GLR}$ refers to the intracellular glycerol concentration, and $\sigma_{GLC}$ refers to a distribution coefficient relating to glycerol.

Then, FAC is synthesized using ATP from the fatty acid (FFA) flown into the cell and CoA according to Formula (8) of Embodiment 1. The rate of this reaction is represented by Formula (54) below.

$$f_{FFA \to FAC} = V_{FFA \to FAC} \left( \frac{\frac{C_{ATP}}{C_{ADP}}}{K_{\frac{ATP}{ADP}} + \frac{C_{ATP}}{C_{ADP}}} \right) \left( \frac{\frac{C_{FFA}}{K_{FFA}} \cdot \frac{C_{CoA}}{K_{CoA}}}{1 + \frac{C_{FFA}}{K_{FFA}} + \frac{C_{CoA}}{K_{CoA}} + \frac{C_{FFA}}{K_{FFA}} \cdot \frac{C_{CoA}}{K_{CoA}} + \frac{C_{FAC}}{K_{FAC}} + \frac{C_{Pi}}{K_{Pi}} + \frac{C_{FAC}}{K_{FAC}} \cdot \frac{C_{Pi}}{K_{Pi}}} \right) \quad \cdots (54)$$

$$K_{FFA} = 0.57$$

$$K_{CoA} = 0.026$$

$$K_{FAC} = 0.0035$$

$$K_{Pi} = 2.7$$

$$K_{\frac{ATP}{ADP}} = 315$$

$$V_{FFA \to FAC} = 0.337312$$

Next, DG is synthesized via a plurality of reactions from the GA3P in the glycolysis block and the produced FAC.

$$GA3P + 2FAC + NADH \to DG + 2CoA + Pi + NAD \ldots (55)$$

The rate of this reaction is represented by Formula (56) below.

$$f_{GA3P \to DG} = V_{GA3P \to DG} \left( \dfrac{\dfrac{C_{NADH}}{C_{NAD}}}{K_{\frac{NADH}{NAD}} + \dfrac{C_{NADH}}{C_{NAD}}} \right) \left( \dfrac{\dfrac{C_{GA3P}}{K_{GA3P}} \cdot \dfrac{C_{FAC}}{K_{FAC}}}{1 + \dfrac{C_{GA3P}}{K_{GA3P}} + \dfrac{C_{FAC}}{K_{FAC}} + \dfrac{C_{GA3P}}{K_{GA3P}} \cdot \dfrac{C_{FAC}}{K_{FAC}} + \dfrac{C_{DG}}{K_{DG}} + \dfrac{C_{CoA}}{K_{CoA}} + \dfrac{C_{Pi}}{K_{Pi}} + \dfrac{C_{DG}}{K_{DG}} \cdot \dfrac{C_{CoA}}{K_{CoA}} \cdot \dfrac{C_{Pi}}{K_{Pi}}} \right) \cdots (56)$$

$$K_{GA3P} = 0.8$$

$$K_{CoA} = 0.026$$

$$K_{FAC} = 0.0035$$

$$K_{Pi} = 2.7$$

$$K_{\frac{NAD}{NADH}} = 81.0$$

$$V_{GA3P \to DG} = 3.931049$$

Next, TG is produced from DG and FAC.

$$DG + FAC \to TG + CoA \ldots (57)$$

The rate of this reaction is represented by Formula (58) below.

$$f_{DG \to TG} = V_{DG \to FAC} \left( \dfrac{\dfrac{C_{DG}}{K_{DG}} \cdot \dfrac{C_{FAC}}{K_{FAC}}}{1 + \dfrac{C_{DG}}{K_{DG}} + \dfrac{C_{FAC}}{K_{FAC}} + \dfrac{C_{DG}}{K_{DG}} \cdot \dfrac{C_{FAC}}{K_{FAC}} + \dfrac{C_{TG}}{K_{TG}} + \dfrac{C_{CoA}}{K_{CoA}} + \dfrac{C_{TG}}{K_{TG}} \cdot \dfrac{C_{CoA}}{K_{CoA}}} \right) \cdots (58)$$

$$K_{DG} = 0.329$$

$$K_{TG} = 14.8$$

$$K_{FAC} = 0.0035$$

$$K_{CoA} = 0.026$$

$$V_{DG \to TGL} = 0.044387$$

As shown in the following formula, hormone-sensitive lipase (HSL) or the like causes the triglyceride (TG) to decompose into DG and FFA.

$$TG \rightarrow DG + FFA \dots (59)$$

The rate of this reaction is represented by Formula (60) below.

$$f_{TG \rightarrow DG} = V_{TG \rightarrow DG} \left( \frac{\dfrac{C_{TG}}{K_{TG}}}{1 + \dfrac{C_{TG}}{K_{TG}} + \dfrac{C_{DG}}{K_{DG}} + \dfrac{C_{FFA}}{K_{FFA}} + \dfrac{C_{DG}}{K_{DG}} \cdot \dfrac{C_{FFA}}{K_{FFA}}} \right) \quad \cdots (60)$$

$$K_{TG} = 14.8$$

$$K_{DG} = 0.329$$

$$K_{FFA} = 0.57$$

$$V_{TG \rightarrow DG} = 0.032714 \cdot HSL(insulin)$$

Here, HSL(insulin) is calculated in the insulin signaling block 222.
HSL or the like causes the DG to decompose into FFA and GLR.

$$DG \rightarrow GLR + 2FFA \dots (61)$$

The rate of this reaction is represented by Formula (62) below.

$$f_{DG \rightarrow GLR} = V_{DG \rightarrow GLR} \left( \frac{\dfrac{C_{DG}}{K_{DG}}}{1 + \dfrac{C_{DG}}{K_{DG}} + \dfrac{C_{GLR}}{K_{GLR}} + \dfrac{C_{FFA}}{K_{FFA}} + \dfrac{C_{GLR}}{K_{GLR}} \cdot \dfrac{C_{FFA}}{K_{FFA}}} \right) \quad \cdots (62)$$

$$K_{DG} = 0.329$$

$$K_{FFA} = 0.57$$

$$K_{GLR} = 0.062$$

$$V_{DG \rightarrow GLR} = 0.032714 \cdot HSL(insulin)$$

In the fatty acid metabolism block 221, the CPU 211a calculates the reaction rates $f_{FFA}$, $f_{GLC}$, $f_{FFA \to FAC}$, $f_{GA3P \to DG}$, $f_{DG \to TG}$, $f_{PG \to DG}$, and $f_{DG \to GLR}$ represented by Formulae (52), (53), (54), (56), (58), (60), and (62) above, respectively.

Furthermore, in the fatty acid metabolism block 221, the CPU 211a calculates the production rate of FFA represented by Formula (63) below, the production rate of FAC represented by Formula (64) below, the production rate of DG represented by Formula (65) below, the production rate of TG represented by Formula (66) below, and the production rate of CLC represented by Formula (67) below.

$$V \cdot \frac{dC_{FFA}}{dt} = f_{FFA} - f_{FFA \to FAC} + f_{TG \to DG} + 2f_{DG \to FFA} \quad \cdots(63)$$

$$V \cdot \frac{dC_{FAC}}{dt} = f_{FFA \to FAC} - 2f_{GA3P \to DG} - f_{DG \to TG} - f_{FAC \to ACoA} \quad \cdots(64)$$

$$V \cdot \frac{dC_{DG}}{dt} = f_{GA3P \to DG} - f_{DG \to TG} + f_{TG \to DG} - f_{DG \to FFA} \quad \cdots(65)$$

$$V \cdot \frac{dC_{TG}}{dt} = f_{DG \to TG} - f_{TG \to DG} \quad \cdots(66)$$

$$V \cdot \frac{dC_{GLR}}{dt} = f_{GLR} + f_{DG \to FFA} \quad \cdots(67)$$

In Formula (64), the reaction rate $f_{FAC \to ACoA}$ is represented by Formula (152) described later, and calculated in the mitochondria block 224. If no calculation has been performed in the mitochondria block 224, the initial value of the reaction rate $f_{FAC \to ACoA}$ is used.

Furthermore, in the fatty acid metabolism block 221, the CPU 211a calculates the amounts of FFA, FAC, DG, TG, and GLR produced in a specific period of time respectively from the thus obtained rates of FFA, FAC, DG, TG, and GLR produced, and reflects these amounts on the intracellular FFA concentration, FAC concentration, DG concentration, TG concentration, and GLR concentration at that time, thereby calculating the intracellular FFA concentration, FAC concentration, DG concentration, TG concentration, and GLR concentration after the specific period of time.

Furthermore, as described later, the fatty acid metabolism block 221 is used both in the first glucose uptake rate estimating process and in the second glucose uptake rate estimating process. In the first glucose uptake rate estimating process, the fatty acid metabolism block 221 is used to perform a process that calculates the intracellular glucose concentration, FFAconcentration, FAC concentration, DG concentration, TG concentration, and GLC acid concentration in a fasted state. Furthermore, in the second glucose uptake rate calculating process, the fatty acid metabolism block 221 is used to perform a process that calculates the intracellular concentrations of the substances in a hyperinsulinemic state.

Insulin Signaling Block

The insulin signaling block 222 is a functional block virtually reproducing a function of adjusting the appearance amount of glucose transporter (GLUT4) that is known to appear in a living organ, in particular, muscle tissues and adipose tissues. The function of adjusting the GLUT4 appearance amount in a living body adjusts the ratio of glucose transporter (GLUT4) occupying the cell membrane according to the amount of insulin binding to insulin receptors and the concentration of diacylglycerol (DG) formed as a metabolite of the fatty acid metabolism. The insulin signaling block 222 according to this embodiment represents such a function of adjusting the ratio of GLUT4 occupying the cell membrane in the body, and the enzymes including hexokinase (HK), glycogen synthase (GS), pyruvate dehydrogenase (PDH), and hormone-sensitive lipase (HSL), and fatty acid oxidization on which insulin is known to act. Execution of the insulin signaling block 222 by the CPU 211a allows a value according to the GLUT4 appearance amount, and the activation of the enzymes HK, GS, PDH, and HSL and the fatty acid oxidization to be calculated based on the plasma insulin concentration (PI) and the DG concentration.

Hereinafter, a specific calculation process of the insulin signaling block 222 based on the function of adjusting the GLUT4 appearance amount on the cell membrane in the living organ will be described. The function of adjusting the GLUT4 appearance amount in the living organ is characterized in that, as the plasma insulin concentration is increased, the GLUT4 appearance amount is increased, and, as the FAC concentration and the DG concentration are increased, the GLUT4 appearance amount is suppressed. In consideration of these characteristics, in the insulin signaling block 222, a glucose uptake (GLUT), which is a value according to the GLUT4 appearance amount, is calculated following Formulae (68) to (131) below using the input plasma insulin concentration and the DG concentration obtained through the calculation in the fatty acid metabolism block 21.

The transition rate $f_{x2 \to x3}$ from insulin receptors on the cell membrane binding to no insulin to insulin receptors on the cell membrane binding to one insulin molecule is determined by the plasma insulin concentration $x_1$, the concentration $x2$ of the insulin receptors on the cell membrane binding to no insulin, and the reaction rate constant $k_1$ as shown in Formula (68) below.

$$f_{x2 \to x3} = k_1 x_1 x_2 \quad \cdots (68)$$

$$k_1 = 6 \times 10^7 M^{-1} \cdot \min^{-1}$$

The transition rate $f_{x3 \to x2}$ from insulin receptors on the cell membrane binding to one insulin molecule to insulin receptors on the cell membrane binding to no insulin is determined by the concentration $x3$ of the insulin receptors on the cell membrane binding to one insulin molecule and the reaction rate constant $K_{-1}$ as shown in Formula (69) below.

$$f_{x3 \to x2} = k_{-1} x_3 \quad \cdots (69)$$

$$k_{-1} = 0.20 \min^{-1}$$

The transition rate $f_{x3 \to x5}$ from insulin receptors on the cell membrane binding to one insulin molecule to insulin receptors on the cell membrane binding to one insulin molecule and activated by phosphorylation is determined by the concentration $x_3$ of the insulin receptors on the cell membrane binding to one insulin molecule and the reaction rate constant $k_3$ as shown in Formula (70) below.

$$f_{x3 \to x5} = k_3 x_3 \quad \cdots (70)$$

$$k_3 = 2500 \min^{-1}$$

The transition rate $f_{x5 \to x2}$ from insulin receptors on the cell membrane binding to one insulin molecule and activated by phosphorylation to insulin receptors on the cell membrane binding to no insulin is determined by the concentration $x_5$ of the insulin receptors on the cell membrane binding to one insulin molecule and activated by phosphorylation, the protein tyrosine phosphatase (PTP), and the reaction rate constant $k_{-3}$ as shown in Formula (71) below.

$$f_{x5 \to x2} = k_{-3} [PTP] x_5 \quad \cdots (71)$$

$$k_{-3} = k_{-1}$$

$$[PTP] = 1.00\left(1 - 0.25\frac{x_{17}}{100/11}\right)$$

if $x_{17} \leq 400/11$

$$[PTP] = 1.00$$

otherwise

The transition rate $f_{x5 \rightarrow x4}$ from insulin receptors on the cell membrane binding to one insulin molecule and activated by phosphorylation to insulin receptors on the cell membrane binding to two insulin molecules and activated by phosphorylation is determined by the concentration $x_5$ of the insulin receptors on the cell membrane binding to one insulin, molecule and activated by phosphorylation, and the reaction rate constant $k_2$ as shown in Formula (72) below.

$$f_{x5 \rightarrow x4} = k_2 x_5 \quad \cdots (72)$$

$$k_2 = k_1$$

The transition rate $f_{x4 \rightarrow x5}$ from insulin receptors on the cell membrane binding to two insulin molecules and activated by phosphorylation to insulin receptors on the cell membrane binding to one insulin molecule and activated by phosphorylation is determined by the concentration $x_4$ of the insulin receptors on the cell membrane binding to one insulin molecule and activated by phosphorylation and the reaction rate constant $k_{-2}$ as shown in Formula (73) below.

$$f_{x4 \rightarrow x5} = k_{-2} x_4 \quad \cdots (73)$$

$$k_{-2} = 100 k_1$$

The transition rate $f_{x2 \rightarrow x6}$ from insulin receptors on the cell membrane binding to no insulin to insulin receptors binding to no insulin in the cell is determined by the concentration $x_2$ of the insulin receptors on the cell membrane binding to no insulin and the reaction rate constant $k_4$ as shown in Formula (74) below.

$$f_{x2 \rightarrow x6} = k_4 x_2 \quad \cdots (74)$$

$$k_4 = \frac{k_{-4}}{9}$$

The transition rate $f_{x6 \rightarrow x2}$ from insulin receptors binding to no insulin in the cell to insulin receptors on the cell membrane binding to one insulin molecule is determined by the concentration $x_6$ of the insulin receptors binding to no insulin in the cell and the reaction rate constant $k_{-4}$ as shown in Formula (75) below.

$$f_{x6 \rightarrow x2} = k_{-4} x_6 \quad \cdots (75)$$

$$k_{-4} = 0.003 \, \text{min}^{-1}$$

The transition rate $f_{x5 \to x8}$ from insulin receptors on the cell membrane binding to one insulin molecule and activated by phosphorylation to insulin receptors binding to one insulin molecule in the cell and activated by phosphorylation is determined by the concentration $x_5$ of the insulin receptors on the cell membrane binding to one insulin molecule and activated by phosphorylation and the reaction rate constant $k_{4'}$ as shown in Formula (76) below.

$$f_{x5 \to x8} = k_{4'} x_5 \quad \cdots (76)$$

$$k_{4'} = 2.1 \times 10^{-3} \, \text{min}^{-1}$$

The transition rate $f_{x8 \to x5}$ from insulin receptors binding to one insulin molecule in the cell and activated by phosphorylation to insulin receptors on the cell membrane binding to one insulin molecule and activated by phosphorylation is determined by the concentration $x_8$ of the insulin receptors binding to one insulin molecule in the cell and activated by phosphorylation and the reaction rate constant $k_{-4'}$ as shown in Formula (77) below.

$$f_{x8 \to x5} = k_{-4'} x_8 \quad \cdots (77)$$

$$k_{-4'} = 2.1 \times 10^{-4} \, \text{min}^{-1}$$

The transition rate $f_{x4 \to x7}$ from insulin receptors on the cell membrane binding to two insulin molecules and activated by phosphorylation to insulin receptors binding to two insulin molecules in the cell and activated by phosphorylation is determined by the concentration $x_4$ of the insulin receptors on the cell membrane binding to two insulin molecules and activated by phosphorylation and the reaction rate constant $k_{4'}$ as shown in Formula (78) below.

$$f_{x4 \to x7} = k_{4'} x_4 \quad \cdots (78)$$

The transition rate $f_{x7 \to x4}$ from insulin receptors binding to two insulin molecules in the cell and activated by phosphorylation to insulin receptors on the cell membrane binding to two insulin molecules and activated by phosphorylation is determined by the concentration $x_7$ of the insulin receptors binding to two insulin molecules in the cell and activated by phosphorylation and the reaction rate constant $k_{-4'}$ as shown in Formula (79) below.

$$f_{x7 \to x4} = k_{-4'} x_7 \quad \cdots (79)$$

The transition rate $f_{x7 \to x6}$ from insulin receptors binding to two insulin molecules in the cell and activated by phosphorylation to insulin receptors binding to no insulin in the cell is determined by the concentration $x_7$ of the insulin receptors binding to two insulin molecules in the cell and activated by phosphorylation, the protein tyrosine phosphatase (PTP), and the reaction rate constant $k_6$ as shown in Formula (80) below.

$$f_{x7 \to x6} = k_6 [PTP] x_7 \quad \cdots (80)$$

$$k_6 = 0.461 \, \text{min}^{-1}$$

$$[PTP] = 1.00\left(1 - 0.25\frac{x_{17}}{100/11}\right)$$

if $x_{17} \leq 400/11$

$$[PTP] = 1.00$$

otherwise

The transition rate $f_{x8 \to x6}$ from insulin receptors binding to one insulin molecule in the cell and activated by phosphorylation to insulin receptors binding to no insulin in the cell is determined by the concentration $x_8$ of the insulin receptors binding to one insulin molecule in the cell and activated by phosphorylation, the protein tyrosine phosphatase (PTP), and the reaction rate constant $k_6$ as shown in Formula (81) below.

$$f_{x8 \to x6} = k_6[PTP]x_8 \quad \cdots(81)$$

$$k_6 = 0.461\,\text{min}^{-1}$$

$$[PTP] = 1.00\left(1 - 0.25\frac{x_{17}}{100/11}\right)$$

if $x_{17} \leq 400/11$

$$[PTP] = 1.00$$

otherwise

It is assumed that the rate of producing an insulin receptor in the cell is a constant value indicated as a constant k5 as shown in Formula (82) below.

$$f_{\to x6} = k_5 \quad \cdots(82)$$

$$k_5 = 10k_{-5}M \cdot \text{min}^{-1}$$

if $(x_6+x_7+x_8) > 1 \times 10^{-13}$

$$k_5 = 60k_{-5}M \cdot \text{min}^{-1}$$

if $(x_6+x_7+x_8) \leq 1 \times 10^{-13}$

The decomposition rate $fx_{6 \to}$ of the insulin receptors binding to no insulin in the cell is determined by the concentration $x_6$ of the insulin receptors binding to no insulin in the cell and the reaction rate constant $k_{-5}$ as shown in Formula (83) below.

$$f_{x6\to} = k_{-5}x_6 \quad \cdots(83)$$

$$k_{-5} = 1.67 \times 10^{-18} \, \text{min}^{-1}$$

The transition rate $f_{x9\to x10}$ from an unphosphorylated insulin receptor substrate 1 (IRS1) to a tyrosine-phosphorylated insulin receptor substrate 1 is determined by the concentration $x_9$ of the unphosphorylated insulin receptor substrate 1, $x_4$ of the insulin receptors on the cell membrane binding to two insulin molecules and activated by phosphorylation, $x_5$ of the insulin receptors on the cell membrane binding to one insulin molecule and activated by phosphorylation, and the reaction rate constant $k_7$ of tyrosine phosphorylation as shown in Formula (84) below.

$$f_{x9\to x10} = k_7 x_9 (x_4 + x_5)/IR_p \quad \cdots(84)$$

$$k_7 = 4.16 \, \text{min}^{-1}$$

In the formula, IRp refers to the phosphorylated insulin receptor concentration on the cell membrane under the maximum insulin stimulation.

The transition rate $f_{x10\to 9}$ from a tyrosine-phosphorylated insulin receptor substrate 1 to an unphosphorylated insulin receptor substrate 1 (IRS1) is determined by $x_{10}$ of the tyrosine-phosphorylated insulin receptor substrate 1, the protein tyrosine phosphatase (PTP), and the reaction rate constant $k_{-7}$ of the dephosphorylation as shown in Formula (85) below.

$$f_{x10\to x9} = k_{-7}[PTP]x_{10} \quad \cdots(85)$$

$$k_{-7} = \frac{2.5}{7.45}k_7 \, \text{min}^{-1}$$

$$[PTP] = 1.00\left(1 - 0.25\frac{x_{17}}{100/11}\right)$$

if $x_{17} \le 400/11$

$$[PTP] = 1.00$$

otherwise

The transition rate $f_{x9\to 10a}$ from an unphosphorylated insulin receptor substrate 1 (IRS1) to a serine-phosphorylated insulin receptor substrate 1 is determined by the concentration $x_9$ of the unphosphorylated insulin receptor substrate 1, the protein kinase C (PKC), and the reaction rate constant $k_7$ of the serine phosphorylation as shown in Formula (86) below.

$$f_{x9\to x10a} = k_{7'}[PKC]x_9 \quad \cdots(86)$$

$$k_{7'} = \ln(2)/2\,\text{min}^{-1}$$

$$[PKC] = [PKC\zeta] + [PKC\theta]$$

$$[PKC\zeta] = \frac{V_{max}(x_{19}(t-\tau))^n}{K_d^n + (x_{19}(t-\tau))^n}$$

$$V_{max} = 20$$

$$K_d = 12$$

$$n = 4$$

$$\tau = 1.5$$

$$PKC\theta = 20 \cdot DG^3$$

The transition rate $f_{x10a \to x9}$ from a serine-phosphorylated insulin receptor substrate 1 to an unphosphorylated insulin receptor substrate 1 (IRS1) is determined by $x_{10a}$ of the serine-phosphorylated insulin receptor substrate and the reaction rate constant $k_{-7'}$ of the dephosphorylation as shown in Formula (87) below.

$$f_{x10a \to x9} = k_{-7'}x_{10a} \quad \cdots(87)$$

$$k_{-7'} = k_{7'}\frac{(2.5/7.45)(3.70 \times 10^{-13})}{(6.27 \times 10^{-13}) - (2.5/7.45)(3.70 \times 10^{-13})}$$

The rate $f_{x10x \to 12}$ of producing a complex of a serine-phosphorylated insulin receptor substrate 1 and a phosphatidylinositol 3-kinase (PI3K) from a serine-phosphorylated insulin receptor substrate and a phosphatidylinositol 3-kinase (PI3K) is determined by the concentration $x_{10}$ of the serine-phosphorylated insulin receptor substrate 1, the concentration $x_{11}$ of the phosphatidylinositol 3-kinase (PI3K), and the rate constant $k_8$ as shown in Formula (88) below.

$$f_{x10 \to x12} = k_8 x_{10} x_{11} \quad \cdots(88)$$

$$k_8 = k_{-8}\frac{5}{70.775} \times 10^{12}\,\text{min}^{-1}$$

The rate $f_{x12 \to x10}$ of producing a serine-phosphorylated insulin receptor substrate 1 and a phosphatidylinositol 3-kinase (PI3K) complex of a serine-phosphorylated insulin receptor substrate 1 and a phosphatidylinositol 3-kinase (PI3K) is determined by the concentration $x_{12}$ of the complex of a serine-phosphorylated insulin receptor substrate 1 and a phosphatidylinositol 3-kinase (PI3K) and the rate constant $k_{-8}$ as shown in Formula (89) below.

$$f_{x12 \to x10} = k_{-8} x_{12} \quad \cdots (89)$$

$$k_{-8} = 10.0 \, \text{min}^{-1}$$

The rate $f_{x13 \to x15}$ of dephosphorylation from phosphatidylinositol (3,4,5)-trisphosphate into phosphatidylinositol (3,4)-bisphosphate using 5'-lipid phosphatase such as Src homology 2 containing inositol 5' phosphatase (SHIP) is determined by the ratio $x_{13}$ of the phosphatidylinositol (3,4,5)-trisphosphate, the SHIP concentration, and the rate constant $k_{-10}$ as shown in Formula (90) below.

$$f_{x13 \to x15} = k_{-10} [SHIP] x_{13} \quad \cdots (90)$$

$$k_{-10} = 2.77 \, \text{min}^{-1}$$

$$[SHIP] = 1.00$$

The rate $f_{x15 \to x13}$ of phosphorylation from phosphatidylinositol (3,4)-bisphosphate into phosphatidylinositol (3,4,5)-trisphosphate is determined by the ratio $x_{15}$ of the phosphatidylinositol (3,4)-bisphosphate and the rate constant $k_{10}$ as shown in Formula (91) below.

$$f_{x15 \to x13} = k_{10} x_{15} \quad \cdots (91)$$

$$k_{10} = \frac{3.1}{2.9} k_{-10}$$

The rate $f_{x13 \to x14}$ of dephosphorylation from phosphatidylinositol (3,4,5)-trisphosphate into phosphatidylinositol (4,5)-bisphosphate using 3'-lipid phosphatase such as phosphatase and tensin homolog deleted from chromosome 10 (PTEN) is determined by the ratio $x_{13}$ of the phosphatidylinositol (3,4,5)-trisphosphate, the PTEN concentration, and the rate constant $k_{-9}$ as shown in Formula (92) below.

$$f_{x13 \to x14} = k_{-9} [PTEN] x_{13} \quad \cdots (92)$$

$$k_{9(stimulated)} = 1.39 \, \text{min}^{-1}$$

$$k_{-9} = \frac{94}{3.1} k_{9(stimulated)}$$

$$[PTEN] = 1.00$$

In the formula, $k_9$(stimulated) refers to a constant relating to the rate $f_{x14 \to x13}$ of phosphorylation from phosphatidylinositol (4,5)-bisphosphate into phosphatidylinositol (3,4,5)-trisphosphate, and relates to a component depending on the complex of a serine-phosphorylated insulin receptor substrate 1 and a phosphatidylinositol 3-kinase (PI3K) at that rate.

The rate $f_{x14 \to 13}$ of phosphorylation from phosphatidylinositol (4,5)-bisphosphate into phosphatidylinositol (3,4,5)-trisphosphate is determined by the ratio $x_{14}$ of the phosphatidylinositol (4,5)-bisphosphate and the rate constant $k_9$ as shown in Formula (93) below.

$$f_{x14 \to x13} = k_9 x_{14} \quad \cdots (93)$$

$$k_9 = \left( k_{9(stimulated)} - k_{9(basal)} \right) \left( \frac{x_{12}}{PI3K} \right) + k_{9(basal)}$$

$$k_{9(basal)} = \frac{0.31}{99.4} k_{-9}$$

$$PI3K = \frac{k_8 \left( 3.70 \times 10^{-13} \right) \left( 1 \times 10^{-13} \right)}{k_8 \left( 3.70 \times 10^{-13} \right) + k_{-8}}$$

In the formula, $k_9$(basal) refers to a constant relating to the rate $f_{x10 \to x13}$ of phosphorylation from phosphatidylinositol (4,5)-bisphosphate into phosphatidylinositol (3,4,5)-trisphosphate, and relates to a component not depending on the complex of a serine-phosphorylated insulin receptor substrate 1 and a phosphatidylinositol 3-kinase (PI3K) at that rate.

The rate $f_{x16 \to x17}$ of phosphorylation from unphosphorylatedAKT into phosphorylatedAKT is determined by the uphosphorylated AKT ratio $x_{16}$ and the rate constant $k_{11}$ as shown in Formula (94) below.

$$f_{x16 \to x17} = k_{11} x_{16} \quad \cdots (94)$$

$$k_{11} = \frac{0.1 k_{-11} \left( x_{13} - 0.31 \right)}{3.1 - 0.31}$$

The rate $f_{x17 \to 16}$ of dephosphorylation from phosphorylated AKT into unphosphorylated AKT is determined by the phosphorylated AKT ratio $x_{17}$ and the rate constant $k_{-11}$ as shown in Formula (95) below.

$$f_{x17 \to x16} = k_{-11} x_{17} \quad \cdots (95)$$

$$k_{-11} = 10 \ln(2) \min^{-1}$$

The rate $f_{x18 \to x19}$ of phosphorylation from unphosphorylated PKC into phosphorylated PKC is determined by the unphosphorylated PKC ratio $x_{18}$ and the rate constant $k_{12}$ as shown in Formula (96) below.

$$f_{x18 \to x19} = k_{12} x_{18} \quad \cdots (96)$$

$$k_{12} = \frac{0.1 k_{-12} (x_{13} - 0.31)}{3.1 - 0.31}$$

The rate $f_{x19 \to x18}$ of dephosphorylation from phosphorylated PKC into unphosphorylated PKC is determined by the phosphorylated PKC ratio $x_{19}$ and the rate constant $k_{-12}$ as shown in Formula (97) below.

$$f_{x19 \to x18} = k_{-12} x_{19} \quad \cdots (97)$$

$$k_{-12} = 10 \ln(2) \, \text{min}^{-1}$$

The rate $f_{\to x20}$ of producing GLUT4 in the cell is determined by the production rate $k_{14}$ as shown in Formula (98) below.

$$f_{\to x20} = k_{14} \quad \cdots (98)$$

$$k_{14} = 96 k_{-14}$$

The decomposition rate $f_{x20 \to}$ of GLUT4 in the cell is determined by the ratio $x_{20}$ of the GLUT4 in the cell and the rate constant $k_{-14}$ as shown in Formula (99) below.

$$f_{x20 \to} = k_{-14} x_{20} \quad \cdots (99)$$

$$k_{-14} = 0.001155 \, \text{min}^{-1}$$

The translocation rate $f_{x20 \to x21}$ from glucose transporter GLUT4 in the cell to GLUT4 on the cell membrane is determined by the ratio $x_{20}$ 0f the GLUT4 in the cell and the rate constants $k_{13}$ and $k_{13'}$ as shown in Formula (100) below.

$$f_{x20 \to x21} = (k_{13} + k_{13'}) x_{20} \quad \cdots (100)$$

$$k_{13} = \frac{4}{96} k_{-13}$$

$$k_{13'} = \left(\frac{40}{60} - \frac{4}{96}\right) \cdot k_{-13} \cdot \left(Effect\right)$$

$$Effect = \frac{\left(0.2x_{17} + 0.8x_{19}\right)}{AP_{equil}}$$

$$AP_{equil} = \frac{100}{11}$$

The translocation rate $f_{x21 \to x20}$ from glucose transporter GLUT4 on the cell membrane to GLUT4 in the cell is determined by the ratio $x_{21}$ of the GLUT4 on the cell membrane and the rate constant $k_{-13}$ as shown in Formula (101) below.

$$f_{x21 \to x20} = k_{-13}x_{21} \quad \cdots (101)$$

$$k_{-13} = 0.167\,\mathrm{min}^{-1}$$

The activation rate $f_{x22 \to x23}$ from inactive enzymes into activated enzymes is determined, by the enzyme activity auxiliary variable $x_{22}$ and the rate constant $k_{15}$ as shown in Formula (102) below.

$$f_{x22 \to x23} = k_{15}x_{22} \quad \cdots (102)$$

$$k_{15} = 0.01 \cdot \left(Effect2\right)\mathrm{min}^{-1}$$

$$Effect2 = \frac{x_{17}}{AP_{equil}}$$

The inactivation rate $f_{x23 \to x22}$ from activated enzymes into inactive enzymes is determined by the enzyme activity auxiliary variable $x_{23}$ and the rate constant $k_{-15}$ as shown in Formula (103) below.

$$f_{x23 \to x22} = k_{-15}x_{23} \quad \cdots (103)$$

$$k_{-15} = 0.01\,\mathrm{min}^{-1}$$

Hexokinase Activity

The value of activated hexokinase (HK) is calculated using Formula (104) below.

$$HK(insulin) = 0.2339x_{23} \quad \cdots(104)$$

Glycogen Synthase Activity

The value of activated glycogen synthase (GS) is calculated using Formula (105) below.

$$GS(insulin) = 0.52x_{23} + 0.134817 \quad \cdots(105)$$

Pyruvate Dehydrogenase Activity

The value of activated pyruvate dehydrogenase (PDH) is calculated using Formula (106) below.

$$PDH(insulin) = 0.37643x_{23} + 0.56191 \quad \cdots(106)$$

Hormone-sensitive Lipase Activity

The value of activated hormone-sensitive lipase (HSL) is calculated using Formula (107) below,

$$HSL(insulin) = 1.0 - 0.12174x_{23} \quad \cdots(107)$$

Fatty Acid Oxidization (Beta-oxidization) Activity

The value of activated hydroxyacyl dehydrogenase (HAD) is calculated using Formulae (108) below.

$$HAD(insulin) = 1.0 - 0.12174x_{23} \quad \cdots(108)$$

Furthermore, in the insulin signaling block 222, the production rate of insulin receptors on the cell membrane binding to no insulin represented by Formula (109) below, the production rate of insulin receptors on the cell membrane binding to one insulin molecule represented by Formula (110) below, the production rate of insulin receptors on the cell membrane binding to one insulin molecule and activated by phosphorylation represented by Formula (111) below, the production rate of insulin receptors on the cell membrane binding to two insulin molecules and activated by phosphorylation represented by Formula (112) below, the production rate of insulin receptors binding to no insulin in the cell represented by Formula (113) below, the production rate of insulin receptors binding to one insulin molecule in the cell and activated by phosphorylation represented by Formula (114) below, the production rate of insulin receptors binding to two insulin molecules in the cell and activated by phosphorylation represented by Formula (115) below, the production rate of an unphosphorylated insulin receptor substrate 1 (IRS1) represented by Formula (116) below, the production rate of a tyrosine-phosphorylated insulin receptor substrate 1 represented by Formula (117) below, the production rate of a serine-phosphorylated insulin receptor substrate 1 represented by Formula (118) below, the production rate of a phosphatidylinositol 3-kinase (PI3K) represented by Formula (119) below, the production rate of a complex of a serine-phosphorylated insulin receptor substrate 1 and a phosphatidylinositol 3-kinase (PI3K) represented by Formula. (120) below, the production rate of phosphatidylinositol (3,4,5)-trisphosphate represented by Formula (121) below, the production rate of phosphatidylinositol (3,4)-bisphosphate represented by Formula (122) below, the production rate of phosphatidylinositol (4,5)-bisphosphate represented by Formula (123) below, the production rate of unphosphorylated AKT represented by Formula (124) below, the production rate of phosphorylated AKT represented by Formula (125) below, the production rate of unphosphorylated PKC represented by Formula (126) below, the production rate of phosphorylated PKC represented by Formula (127) below, the production rate of intracellular glucose transporter (GLUT4) represented by Formula (128) below, the production rate of glucose transporter (GLUT4) on the cell membrane represented by Formula (129) below, the enzyme activity auxiliary variable $x_{22}$ production rate represented by Formula (130) below, and the enzyme activity auxiliary variable $x_{23}$ production rate represented by Formula (131) below are calculated.

$$\frac{dx_2}{dt} = f_{x6 \to x2} + f_{x3 \to x2} + f_{x5 \to x2} - f_{x2 \to x3} - f_{x2 \to x6} \quad \cdots(109)$$

$$\frac{dx_3}{dt} = f_{x2 \to x3} - f_{x3 \to x2} - f_{x2 \to x5} \quad \cdots(110)$$

$$\frac{dx_4}{dt} = f_{x5 \to x4} + f_{x7 \to x4} - f_{x4 \to x5} - f_{x4 \to x7} \quad \cdots(111)$$

$$\frac{dx_5}{dt} = f_{x3 \to x5} + f_{x4 \to x5} + f_{x8 \to x5} - f_{x5 \to x2} - f_{x5 \to x4} - f_{x5 \to x8} \quad \cdots(112)$$

$$\frac{dx_6}{dt} = f_{\to x6} + f_{x7 \to x6} + f_{x8 \to x6} + f_{x2 \to x6} - f_{x6 \to x2} - f_{x6 \to} \quad \cdots(113)$$

$$\frac{dx_7}{dt} = f_{x4 \to x7} - f_{x7 \to x4} - f_{x7 \to x6} \quad \cdots(114)$$

$$\frac{dx_8}{dt} = f_{x5 \to x8} - f_{x8 \to x5} - f_{x8 \to x6} \quad \cdots(115)$$

$$\frac{dx_9}{dt} = f_{x10 \to x9} + f_{x10a \to x9} - f_{x9 \to x10} - f_{x9 \to x10a} \quad \cdots(116)$$

$$\frac{dx_{10}}{dt} = f_{x9 \to x10} + f_{x12 \to x10} - f_{x10 \to x9} - f_{x10 \to x12} \quad \cdots(117)$$

$$\frac{dx_{10a}}{dt} = f_{x9 \to x10a} - f_{x10a \to x9} \quad \cdots(118)$$

$$\frac{dx_{11}}{dt} = f_{x12 \to x10} - f_{x10 \to x12} \quad \cdots (119)$$

$$\frac{dx_{12}}{dt} = f_{x10 \to x12} - f_{x12 \to x10} \quad \cdots (120)$$

$$\frac{dx_{13}}{dt} = f_{x14 \to x13} + f_{x15 \to x13} - f_{x13 \to x14} - f_{x13 \to x15} \quad \cdots (121)$$

$$\frac{dx_{14}}{dt} = f_{x13 \to x14} - f_{x14 \to x13} \quad \cdots (122)$$

$$\frac{dx_{15}}{dt} = f_{x13 \to x15} - f_{x15 \to x13} \quad \cdots (123)$$

$$\frac{dx_{16}}{dt} = f_{x17 \to x16} - f_{x16 \to x17} \quad \cdots (124)$$

$$\frac{dx_{17}}{dt} = f_{x16 \to x17} - f_{x17 \to x16} \quad \cdots (125)$$

$$\frac{dx_{18}}{dt} = f_{x19 \to x18} - f_{x18 \to x19} \quad \cdots (126)$$

$$\frac{dx_{19}}{dt} = f_{x18 \to x19} - f_{x19 \to x18} \quad \cdots (127)$$

$$\frac{dx_{20}}{dt} = f_{\to x20} + f_{x21 \to x20} - f_{x20 \to x21} - f_{x20 \to} \quad \cdots (128)$$

$$\frac{dx_{21}}{dt} = f_{x20 \to x21} - f_{x21 \to x20} \quad \cdots (129)$$

$$\frac{dx_{22}}{dt} = f_{x23 \rightarrow x22} - f_{x22 \rightarrow x23} \quad \cdots(130)$$

$$\frac{dx_{23}}{dt} = f_{x22 \rightarrow x23} - f_{x23 \rightarrow x22} \quad \cdots(131)$$

Furthermore, as described later, the insulin signaling block 222 is used both in the first glucose uptake rate estimating process and in the second glucose uptake rate estimating process. In the first glucose uptake rate estimating process, the insulin signaling block 222 is used to perform a process that calculates the glucose uptake GLUT in a fasted state. Furthermore, in the second glucose uptake rate calculating process, the insulin signaling block 222 is used to perform a process that calculates the glucose uptake GLUT in a hyperinsulinemic state.

Glycolysis Block

The glycolysis block 223 is a functional block virtually reproducing a function of causing glucose to decompose in a living organ. The glucose decomposition function of the body causes glucose to be taken up into the cell according to the ratio of glucose transporter (GLUT4) occupying the cell membrane and causes the glucose in the cell to decompose to give pyruvic acid (PYR) via glucose 6-phosphate (G6P), D-glyceraldehyde 3-phosphate (GA3P), and 1,3-bisphosphoglycerate (BPG). Furthermore, this function produces lactic acid (LAC) from pyruvic acid (PYR). The glycolysis block 223 represents such a glucose decomposition function of the body. Execution of the glycolysis block 223 by the CPU 211a allows the glucose uptake rate to be calculated based on the glucose uptake GLUT obtained in the insulin signaling block 22 and the rate of oxygen consumed and the rate of carbon dioxide produced in the tissue, and the reaction rate $f_{GLU \rightarrow G6P}$ of converting glucose into G6P, the reaction rate $f_{G6P \rightarrow GA3P}$ of converting G6P into GA3P, the reaction rate $f_{GA3P \rightarrow BPG}$ of converting GA3P into BPG, the reaction rate $f_{BPG \rightarrow PYR}$ of converting BPG into PYR, the reaction rate $f_{PYR \rightarrow LAC}$ of converting PYR into LAC, and the reaction rate $f_{LAC \rightarrow PYR}$ of converting LAC into PYR to be calculated. Furthermore, the glucose (GLU) concentration, the G6P concentration, the GA3P concentration, the BPG concentration, the PYR concentration, and the LAC concentration after a specific period of time are calculated based on these reaction rates.

Hereinafter, a chemical reaction relating to the glucose decomposition function of the living organ and a specific calculation process of the glycolysis block 223 based thereon will be described. Here, the intracellular concentrations of glucose, G6P, GA3P, BPG, pyruvic acid, LAC, NAD, and NADH in the following formulae are each provided with predetermined initial values. The initial value is used in the first calculation, and an updated value is used in the subsequent calculations.

First, blood glucose is taken up into the cell according to the GLUT4 appearance amount (appearance amount on the cell surface). This uptake rate $f_{GLU}$ is represented by Formula (132) below.

$$f_{GLU} = Q \cdot GLUT \cdot \left( C_{GLU,b} - \sigma_{GLU} \cdot C_{GLU} \right) \quad \cdots(132)$$

In the formula, $C_{GLU,b}$ refers to the input blood glucose concentration, $C_{GLU}$ refers to the intracellular glucose concentration, and $\sigma_{GLU}$ refers to a distribution coefficient relating to glucose.

Blood lactic acid (LAC) is taken up into the cell. The uptake rate $f_{LAC}$ is represented by Formula (133) below.

$$f_{LAC} = Q \cdot \left( C_{LAC,b} - \sigma_{LAC} \cdot C_{LAC} \right) \quad \cdots(133)$$

In the formula, $C_{LAC,b}$ refers to the input blood lactic acid concentration, $C_{LAC}$ refers to the intracellular lactic acid concentration, and $\sigma_{LAC}$ refers to a distribution coefficient relating to lactic acid.

Blood pyruvic acid (PYR) is taken up into the cell. The uptake rate $f_{PYR}$ is represented by Formula (134) below.

$$f_{PYR} = Q \cdot \left( C_{PYR,b} - \sigma_{PYR} \cdot C_{PYR} \right) \quad \cdots (134)$$

In the formula, $C_{PYR,b}$ refers to the input blood pyruvic acid concentration, $C_{PYR}$ refers to the intracellular pyruvic acid concentration, and $\sigma_{PYR}$ refers to a distribution coefficient relating to pyruvic acid.

The glucose (GLU) taken up into the cell is phosphorylated by hexokinase to give G6P (Formula (17)). The rate of this reaction is represented by Formula (135) below.

$$f_{GLU \to G6P} = V_{GLU \to G6P} \left( \frac{\dfrac{C_{ATP}}{C_{ADP}}}{K_{\frac{ATP}{ADP}} + \dfrac{C_{ATP}}{C_{ADP}}} \right) \left( \frac{\dfrac{C_{GLU}}{K_{GLU}}}{1 + \dfrac{C_{GLU}}{K_{GLU}} + \dfrac{C_{G6P}}{K_{G6P}}} \right) \quad \cdots (135)$$

$$K_{GLU} = 0.07$$

$$K_{G6P} = 0.253$$

$$K_{\frac{ATP}{ADP}} = 7.75$$

$$V_{GLU \to G6P} = 2.750297 \cdot HK(insulin)$$

In the formula, HK(insulin) is calculated in the insulin signaling block 222.

Furthermore, the G6P produced by the conversion reacts with ATP to give GA3P and ADP as shown in Formula (19). The rate of this reaction is represented by Formula (136) below.

$$f_{G6P \to GA3P} = V_{G6P \to GA3P} \left( \frac{\dfrac{C_{AMP}}{C_{ATP}}}{K_{\frac{AMP}{ATP}} + \dfrac{C_{AMP}}{C_{ATP}}} \right) \left( \frac{\dfrac{C_{G6P}}{K_{G6P}}}{1 + \dfrac{C_{G6P}}{K_{G6P}} + \dfrac{C_{GA3P}}{K_{GA3P}}} \right) \quad \cdots (136)$$

$$K_{G6P} = 0.253$$

$$K_{GA3P} = 0.08$$

$$K_{\frac{AMP}{ATP}} = 0.00645$$

$$V_{G6P \to GA3P} = 0.07365$$

The GA3P produced by the conversion is converted to 1,3-bisphosphoglycerate (BPG) according to Formula (137) below.

$$GA3P + Pi + NAD \to BPG + NADH \dots (137)$$

The reaction rate of the conversion to is represented by Formula (138) below.

$$f_{GA3P \to BPG} = V_{GA3P \to BPG} \left( \frac{\frac{C_{NAD}}{C_{NADH}}}{K_{\frac{NAD}{NADH}} + \frac{C_{NAD}}{C_{NADH}}} \right) \left( \frac{\frac{C_{GA3P}}{K_{GA3P}} \cdot \frac{C_{Pi}}{K_{Pi}}}{1 + \frac{C_{GA3P}}{K_{GA3P}} + \frac{C_{Pi}}{K_{Pi}} + \frac{C_{GA3P}}{K_{GA3P}} \cdot \frac{C_{Pi}}{K_{Pi}} + \frac{C_{BPG}}{K_{BPG}}} \right) \dots (138)$$

$$K_{GA3P} = 0.08$$

$$K_{Pi} = 27$$

$$K_{BPG} = 0.8$$

$$K_{\frac{NAD}{NADH}} = 0.09$$

$$V_{GA3P \to BPG} = 2.408042$$

The BPG produced by the conversion is converted to pyruvic acid (PYR) according to Formula (139) below.

$$BPG + 2ADP \to PYR + 2ATP \dots (139)$$

The reaction rate of the conversion to PYR is represented by Formula (140) below.

$$f_{BPG \to PYR} = V_{BPG \to PYR} \left( \frac{\frac{C_{ADP}}{C_{ATP}}}{K_{\frac{ADP}{ATP}} + \frac{C_{ADP}}{C_{ATP}}} \right) \left( \frac{\frac{C_{BPG}}{K_{BPG}}}{1 + \frac{C_{BPG}}{K_{BPG}} + \frac{C_{PYR}}{K_{PYR}}} \right) \dots (140)$$

$$K_{BPG} = 0.4$$

$$K_{PYR} = 0.238$$

$$K_{\frac{ADP}{ATP}} = 0.129$$

$$V_{BPG \to PYR} = 0.254724$$

The produced pyruvic acid (PYR) is converted to lactic acid (LAC) according to Formula (141) below.

$$PYR + NADH \to LAC + NAD \dots (141)$$

The reaction rate is represented by Formula (142) below.

$$f_{PYR \to LAC} = V_{PYR \to LAC} \left( \frac{\frac{C_{NADH}}{C_{NAD}}}{K_{\frac{NADH}{NAD}} + \frac{C_{NADH}}{C_{NAD}}} \right) \left( \frac{\frac{C_{PYR}}{K_{PYR}}}{1 + \frac{C_{PYR}}{K_{PYR}} + \frac{C_{LAC}}{K_{LAC}}} \right) \quad \cdots (142)$$

$$K_{PYR} = 0.0475$$

$$K_{LAC} = 1.75$$

$$K_{\frac{NADH}{NAD}} = 1.0$$

$$V_{PYR \to LAC} = 0.441$$

The lactic acid (LAC) is converted to pyruvic acid (PYR) according to Formula (143) below.

$$LAC + NAD \to PYR + NADH \dots (143)$$

The reaction rate is represented by Formula (144) below.

$$f_{LAC \to PYR} = V_{LAC \to PYR} \left( \frac{\frac{C_{NAD}}{C_{NADH}}}{K_{\frac{NAD}{NADH}} + \frac{C_{NAD}}{C_{NADH}}} \right) \left( \frac{\frac{C_{LAC}}{K_{LAC}}}{1 + \frac{C_{PYR}}{K_{PYR}} + \frac{C_{LAC}}{K_{LAC}}} \right) \quad \cdots (144)$$

$$K_{PYR} = 0.0475$$

$$K_{LAC} = 1.75$$

$$K_{\frac{NAD}{NADH}} = 9.0$$

$$V_{LAC \to PYR} = 0.0546$$

In the glycolysis block 223, the CPU 211a calculates the reaction rates $f_{GLU}$, $f_{LAC}$, $f_{PYR}$, $f_{GLU \to G6P}$, $f_{G6P \to GA3P}$, $f_{GA3P \to BPG}$, $f_{BPG \to PYR}$, $f_{PYR \to LAC}$, and $f_{LAC \to PYR}$ represented by Formulae (132), (133), (134), (135), (136), (138), (140), (142), and (144) above, respectively.

Furthermore, in the glycolysis block 223, the CPU 211a calculates the production rate of glucose represented by Formula (145) below, the production rate of G6P represented by Formula (146) below, the production rate of GA3P represented by Formula (147) below, the production rate of BPG represented by Formula (148) below, the production rate of pyruvic acid represented by Formula (149) below, and the production rate of lactic acid represented by Formula (150) below.

$$V \cdot \frac{dC_{GLU}}{dt} = f_{GLU} - f_{GLU \to G6P} \quad \cdots (145)$$

$$V \cdot \frac{dC_{G6P}}{dt} = f_{GLU \to G6P} - f_{G6P \to GA3P} - f_{G6P \to GLY} + f_{GLY \to G6P} \quad \cdots (146)$$

$$V \cdot \frac{dC_{GA3P}}{dt} = 2f_{G6P \to GA3P} - f_{GA3P \to DG} - f_{GA3P \to BPG} \quad \cdots (147)$$

$$V \cdot \frac{dC_{BPG}}{dt} = f_{GA3P \to BPG} - f_{BPG \to PYR} \quad \cdots (148)$$

$$V \cdot \frac{dC_{PYR}}{dt} = f_{BPG \to PYR} - f_{PYR \to ACoA} - f_{PYR \to ALA} + f_{LAC \to PYR} - f_{PYR \to LAC} + f_{PYR}$$

$$V \cdot \frac{dC_{LAC}}{dt} = f_{LAC} - f_{LAC \to PYR} + f_{PYR \to LAC} \quad \cdots (150)$$

In Formula (149), the reaction rate $f_{PYR \to ACoA}$ is represented by Formula (151) described later, and calculated in the mitochondria block 224. The reaction rate $f_{PYR \to ALA}$ is calculated in a supplemental calculation process. If no calculation has been performed in the mitochondria block 224 and the supplemental calculation process, the initial values of the reaction rates $f_{PYR \to ACoA}$ and $f_{PYR \to ALA}$ are used.

Furthermore, in the glycolysis block 223, the CPU 211a calculates the amounts of glucose, G6P, GA3P, BPG, pyruvic acid, and lactic acid produced in a specific period of time respectively from the thus obtained rates of glucose, G6P, GA3P, BPG, pyruvic acid, and lactic acid produced, and reflects these amounts on the intracellular glucose concentration, G6P concentration, GA3P concentration, BPG concentration, and pyruvic acid concentration at that time, thereby calculating the intracellular glucose concentration, G6P concentration, GA3P concentration, BPG concentration, and pyruvic acid concentration after the specific period of time.

Furthermore, as described later, the glycolysis block 223 is used both in the first glucose uptake rate estimating process and in the second glucose uptake rate estimating process. In the first glucose uptake rate estimating process, the glycolysis block 223 is used to perform a process that calculates the intracellular glucose concentration, G6P concentration, GA3P concentration, BPG concentration, pyruvic acid concentration, and lactic acid concentration in a fasted state. Furthermore, in the second glucose uptake rate calculating process, the glycolysis block 223 is used to perform a process that calculates the intracellular concentrations of the substances in a hyperinsulinemic state.

Mitochondria Block

The mitochondria block 224 is a functional block virtually reproducing a function of mitochondria in a living organ. The mitochondria produce acetyl coenzyme A (ACoA) by oxidizing the pyruvic acid (PYR) and the fatty acyl-coenzyme A complex (FAC), and convert the acetyl coenzyme A (ACoA) to $H_2O$ and $CO_2$ through metabolism in the TCA cycle. Fatty acid oxidization is suppressed according to the acetyl coenzyme A (ACoA) concentration. The mitochondria block 224 represents such a mitochondria function. Execution of the mitochondria block 224 by the CPU 211a allows the reaction rate $f_{PYR \to ACoA}$ of producing acetyl coenzyme A (ACoA) from the pyruvic acid obtained in the glycolysis block 223, the reaction rate $f_{FAC \to ACoA}$ of producing acetyl coenzyme A (ACoA) from the fatty acyl-coenzyme A complex (FAC) obtained in the fatty acid metabolism block 21, the reaction rates $f_{ACoA \to CIT}$, $f_{CIT \to aKG}$, $f_{aKG \to SCoA}$, $f_{SCoA \to SUC}$, $f_{SUC \to MAL}$, and $f_{MAL \to OXA}$ of producing citric acid (CIT) from acetyl coenzyme A (ACoA) and oxaloacetic acid (OXA), and sequentially metabolizing the resultant to produce α-ketoglutaric acid (aKG), succinyl-CoA (SCoA, succinic acid (SUC), maleic acid (MAL), and oxaloacetic acid (OXA) in this order, and the reaction rate $f_{O2 \to H2O}$ of consuming oxygen and producing water to be calculated. Furthermore, the intracellular concentrations of acetyl coenzyme A (ACoA), citric acid (CIT), α-ketoglutaric acid (aKG), succinyl-CoA (SCoA), succinic acid (SUC), malic acid (MAL), oxaloacetic acid (OXA), oxygen ($O_2$), and carbon dioxide ($CO_2$) after a specific period of time are calculated based on these reaction rates.

Hereinafter, a biochemical reaction relating to the mitochondria and a specific calculation process of the mitochondria block 224 based thereon will be described. Here, the intracellular concentrations of acetyl coenzyme A (ACoA), coenzyme A (CoA), oxygen ($O_2$), citric acid (CIT), α-ketoglutaric acid (aKG), succinyl-CoA (SCoA), succinic acid (SUC), malic acid (MAL), oxaloacetic acid (OXA), and carbon dioxide ($CO_2$) in the following formulae are each provided with predetermined initial values. The initial value is used in the first calculation, and an updated value is used in the subsequent calculations.

First, intracellular pyruvic acid (PYR) is oxidized to give acetyl coenzyme A (ACoA) (Formula (27)). The rate of this reaction is represented by Formula (151) below.

$$f_{PYR \to ACoA} = V_{PYR \to ACoA} \left( \frac{\dfrac{C_{NAD}}{C_{NADH}}}{K_{\frac{NAD}{NADH}} + \dfrac{C_{NAD}}{C_{NADH}}} \right) \left( \frac{\dfrac{C_{PYR}}{K_{PYR}} \cdot \dfrac{C_{CoA}}{K_{CoA}}}{1 + \dfrac{C_{PYR}}{K_{PYR}} + \dfrac{C_{CoA}}{K_{CoA}} + \dfrac{C_{PYR}}{K_{PYR}} \cdot \dfrac{C_{CoA}}{K_{CoA}} + \dfrac{C_{ACoA}}{K_{ACoA}} + \dfrac{C_{CO_2}}{K_{CO_2}} + \dfrac{C_{ACoA}}{K_{ACoA}} \dfrac{C_{CO_2}}{K_{CO_2}}} \right) \cdots (151)$$

$$K_{PYR} = 0.0475$$

$$K_{CoA} = 0.0255$$

$$K_{ACoA} = 0.0022$$

$$K_{CO2} = 23.6$$

$$K_{\frac{NAD}{NADH}} = 81.0$$

$$V_{PYR \to ACoA} = 1.320406 \cdot PDH(insulin)$$

In the formula, PDH(insulin) is calculated in the insulin signaling block 222.

Furthermore, the fatty acyl-coenzyme A complex (FAC) produced in the fatty acid metabolism block 221 is oxidized to give acetyl coenzyme A (ACoA) as shown in Formula (29) (β-oxidization). The rate of this reaction is represented by Formula (152) below.

$$f_{FAC \to ACoA} = V_{FAC \to ACoA} \left( \frac{\frac{C_{NAD}}{C_{NADH}}}{K_{\frac{NAD}{NADH}} + \frac{C_{NAD}}{C_{NADH}}} \right) \left( \frac{\frac{C_{FAC}}{K_{FAC}} \cdot \frac{C_{CoA}}{K_{CoA}}}{1 + \frac{C_{FAC}}{K_{FAC}} + \frac{C_{CoA}}{K_{CoA}} + \frac{C_{FAC}}{K_{FAC}} \cdot \frac{C_{CoA}}{K_{CoA}} + \frac{C_{ACoA}}{K_{ACoA}}} \right) \quad \cdots(152)$$

$$K_{CoA} = 0.0255$$

$$K_{FAC} = 0.0035$$

$$K_{ACoA} = 0.0023$$

$$K_{\frac{NAD}{NADH}} = 9.0$$

$$V_{FAC \to ACoA} = 0.048809 \cdot HAD(insulin)$$

In the formula, HAD (insulin) is calculated in the insulin signaling block 222.

The acetyl-coenzyme A (ACoA) complex produced from pyruvic acid (PYR) and fatty acyl-coenzyme A complex (FAC) is metabolized in the TCA cycle represented by the series of formulae shown below, and ATP and NADH are newly produced. In the TCA cycle, citric acid (CIT) is produced from oxaloacetic acid (OXA) and acetyl-CoA, and then (α-ketoglutaric acid (aKG), succinyl-CoA (SCoA), succinic acid (SUC), malic acid (MAL), and oxaloacetic acid (OXA) are produced and metabolized in this order.

First, ACoA and OXA are converted by citrate synthase to CIT and CoA (Formula (153)).

$$ACoA + OXA \to CIT + CoA \dots (153)$$

The rate of this reaction is represented by Formula (154) below.

$$f_{ACoA \to CIT} = V_{ACoA \to CIT} \left( \frac{\frac{C_{ACoA}}{K_{ACoA}} \cdot \frac{C_{OXA}}{K_{OXA}}}{1 + \frac{C_{ACoA}}{K_{ACoA}} + \frac{C_{OXA}}{K_{OXA}} + \frac{C_{ACoA}}{K_{ACoA}} \cdot \frac{C_{OXA}}{K_{OXA}} + \frac{C_{CIT}}{K_{CIT}} + \frac{C_{CoA}}{K_{CoA}} + \frac{C_{CIT}}{K_{CIT}} \cdot \frac{C_{CoA}}{K_{CoA}}} \right) \quad \cdots(154)$$

$$K_{ACoA} = 0.0022$$

$$K_{OXA} = 0.003$$

$$K_{CoA} = 0.0255$$

$$K_{CIT} = 0.103$$

$$V_{ACoA \to CIT} = 0.339011$$

The reaction process in which α-ketoglutaric acid (aKG) is produced from citric acid (CIT) via cis-aconitic acid, isocitric acid, and oxalosuccinic acid can be simply represented by the following formula.

$$CIT + NAD \to aKG + NADH + CO_2 \dots (155)$$

The rate of this reaction is represented by Formula (156) below.

$$f_{CIT \to aKG} = V_{CIT \to aKG} \left( \frac{\frac{C_{NAD}}{C_{NADH}}}{K_{\frac{NAD}{NADH}} + \frac{C_{NAD}}{C_{NADH}}} \right) \left( \frac{\frac{C_{CIT}}{K_{CIT}}}{1 + \frac{C_{CIT}}{K_{CIT}} + \frac{C_{aKG}}{K_{aKG}} + \frac{C_{CO_2}}{K_{CO_2}} + \frac{C_{aKG}}{K_{aKG}} \cdot \frac{C_{CO_2}}{K_{CO_2}}} \right) \cdots (156)$$

$$K_{CIT} = 0.103$$

$$K_{aKG} = 0.0125$$

$$K_{CO2} = 23.6$$

$$K_{\frac{NAD}{NADH}} = 9.0$$

$$V_{CIT \to aKG} = 0.489$$

The α-ketoglutaric acid (aKG) is oxidized to give succinyl-CoA (SCoA) and carbon dioxide ($CO_2$) (Formula (157)).

$$aKG + CoA + NAD \to SCoA + NADH + CO_2 \dots (157)$$

The rate of this reaction is represented by Formula (158) below.

$$f_{aKG \to SCoA} = V_{aKG \to SCoA} \left( \frac{\frac{C_{NAD}}{C_{NADH}}}{K_{\frac{NAD}{NADH}} + \frac{C_{NAD}}{C_{NADH}}} \right) \left( \frac{\frac{C_{aKG}}{K_{aKG}} \cdot \frac{C_{CoA}}{K_{CoA}}}{1 + \frac{C_{aKG}}{K_{aKG}} + \frac{C_{CoA}}{K_{CoA}} + \frac{C_{aKG}}{K_{aKG}} \cdot \frac{C_{CoA}}{K_{CoA}} + \frac{C_{SCoA}}{K_{SCoA}} + \frac{C_{CO_2}}{K_{CO_2}} + \frac{C_{SCoA}}{K_{SCoA}} \cdot \frac{C_{CO_2}}{K_{CO_2}}} \right) \cdots (158)$$

$$K_{aKG} = 0.0125$$

$$K_{CoA} = 0.0255$$

$$K_{SCoA} = 0.123$$

$$K_{CO2} = 23.6$$

$$K_{\frac{NAD}{NADH}} = 9.0$$

$$V_{aKG \to SCoA} = 0.6846$$

Succinic acid (SUC) is produced from the succinyl-CoA (SCoA) by succinyl-CoA synthetase (Formula (159)).

$$SCoA + ADP + Pi \to SUC + CoA + ATP \dots (159)$$

The rate of this reaction is represented by Formula (160) below.

$$f_{SCoA \to SUC} = V_{SCoA \to SUC} \left( \frac{\frac{C_{ADP}}{C_{ATP}}}{K_{\frac{ADP}{ATP}} + \frac{C_{ADP}}{C_{ATP}}} \right) \left( \frac{\frac{C_{SCoA}}{K_{SCoA}} \cdot \frac{C_{Pi}}{K_{Pi}}}{1 + \frac{C_{SCoA}}{K_{SCoA}} + \frac{C_{Pi}}{K_{Pi}} + \frac{C_{SCoA}}{K_{SCoA}} \cdot \frac{C_{Pi}}{K_{Pi}} + \frac{C_{SUC}}{K_{SUC}} + \frac{C_{CoA}}{K_{CoA}} + \frac{C_{SUC}}{K_{SUC}} \cdot \frac{C_{CoA}}{K_{CoA}}} \right) \cdots (160)$$

$$K_{SCoA} = 0.123$$

$$K_{Pi} = 2.7$$

$$K_{SUC} = 0.095$$

$$K_{CoA} = 0.0255$$

$$K_{\frac{ADP}{ATP}} = 0.129$$

$$V_{SCoA \rightarrow SUC} = 0.6846$$

Malic acid (MAL) is produced from the succinic acid (SUC) via fumaric acid (Formula (161)).

$$SUC + \frac{2}{3} NAD \rightarrow MAL + \frac{2}{3} NADH \quad \cdots (161)$$

The rate of this reaction is represented by Formula (162) below.

$$f_{SUC \rightarrow MAL} = V_{SUC \rightarrow MAL} \left( \frac{\dfrac{C_{NAD}}{C_{NADH}}}{K_{\frac{NAD}{NADH}} + \dfrac{C_{NAD}}{C_{NADH}}} \right) \left( \frac{\dfrac{C_{SUC}}{K_{SUC}}}{1 + \dfrac{C_{SUC}}{K_{SUC}} + \dfrac{C_{MAL}}{K_{MAL}}} \right) \quad \cdots (162)$$

$$K_{SUC} = 0.095$$

$$K_{MAL} = 0.0975$$

$$K_{\frac{NAD}{NADH}} = 9.0$$

$$V_{SUC \rightarrow MAL} = 0.2934$$

The malic acid (MAL) is oxidized by malate dehydrogenase to give oxaloacetic acid (OXA) (Formula (163)).

$$MAL + NAD \rightarrow OXA + NADH \ldots (163)$$

The rate of this reaction is represented by Formula (164) below.

$$f_{MAL \rightarrow OXA} = V_{MAL \rightarrow OXA} \left( \frac{\dfrac{C_{NAD}}{C_{NADH}}}{K_{\frac{NAD}{NADH}} + \dfrac{C_{NAD}}{C_{NADH}}} \right) \left( \frac{\dfrac{C_{MAL}}{K_{MAL}}}{1 + \dfrac{C_{MAL}}{K_{MAL}} + \dfrac{C_{OXA}}{K_{OXA}}} \right) \quad \cdots (164)$$

$$K_{MAL} = 0.0975$$

$$K_{OXA} = 0.003$$

$$K_{\frac{NAD}{NADH}} = 9.0$$

$$V_{MAL \to OXA} = 0.2934$$

Meanwhile, the relationship between the consumption of NADH, oxygen, and ADP and the synthesis of ATP in the mitochondria is represented by Formula (165) below.

$$O_2 + 5.64ADP + 5.64Pi + 1.88NADH \to 2H_2O + 5.64ATP + 1.88NAD \quad \cdots(165)$$

The rate of this reaction is represented by Formula (166) below.

$$f_{O_2 \to H_2O} = V_{O_2 \to H_2O} \left( \frac{\frac{C_{ADP}}{C_{ATP}}}{K_{\frac{ADP}{ATP}} + \frac{C_{ADP}}{C_{ATP}}} \right) \left( \frac{\frac{C_{O_2}}{K_{O_2}} \cdot \frac{C_{Pi}}{K_{Pi}} \cdot \frac{C_{NADH}}{K_{NADH}}}{1 + \frac{C_{O_2}}{K_{O_2}} + \frac{C_{Pi}}{K_{Pi}} + \frac{C_{NADH}}{K_{NADH}} + \frac{C_{O_2}}{K_{O_2}} \cdot \frac{C_{Pi}}{K_{Pi}} \cdot \frac{C_{NADH}}{K_{NADH}} + \frac{C_{NAD}}{K_{NAD}}} \right) \quad \cdots(166)$$

$$K_{O2} = 0.01$$

$$K_{NADH} = 0.07$$

$$K_{NAD} = 0.63$$

$$K_{Pi} = 3.8$$

$$K_{\frac{ADP}{ATP}} = 0.129$$

$$V_{O_2 \to H_2O} = 0.753529$$

The inflow rate $f_{O2}$ of oxygen into the cell can also be represented by Formula (167) below.

$$f_{O2} = Q \cdot \left( C_{O2,b} - \sigma_{O2} \cdot C_{O2} \right) \quad \cdots(167)$$

In the formula, $C_{O2,b}$ refers to the blood oxygen concentration (constant), $C_{O2}$ refers to the intracellular oxygen concentration, and $\sigma_{O2}$ refers to a distribution coefficient relating to oxygen.

In a similar manner, the rate $f_{CO2}$ of carbon dioxide produced in the cell can also be represented by Formula (168) below.

$$f_{CO2} = Q \cdot \left( C_{CO2,b} - \sigma_{CO2} \cdot C_{CO2} \right) \quad \cdots (168)$$

In the formula, $C_{CO2,b}$ refers to the blood carbon dioxide concentration (constant), $C_{CO2}$ refers to the intracellular carbon dioxide concentration, and $\sigma_{CO2}$ refers to a distribution coefficient relating to carbon dioxide.

In the mitochondria block 224, the CPU 211a calculates the reaction rates $f_{PYR \rightarrow ACoA}$, $f_{FAC \rightarrow ACoA}$, $f_{ACoA \rightarrow CIT}$, $f_{CIT \rightarrow aKG}$, $f_{aKG\text{-}SCoA}$, $f_{SCoA \rightarrow SUC}$, $f_{SUC \rightarrow MAL}$, $f_{MAL \rightarrow OXA}$, $f_{O2 \rightarrow H2O}$, $f_{O2}$, and $f_{CO2}$ represented by Formulae (151), (152), (154), (156), (158), (160), (162), (164), (166), (167), and (168) above, respectively.

Furthermore, in the mitochondria block 224, the CPU 211a calculates the production rate of acetyl coenzyme A (ACoA) represented by Formula (169) below, the production rate of citric acid (CIT) represented by Formula (170) below, the production rate of α-ketoglutaric acid (aKG) represented by Formula (171) below, the production rate of succinyl-CoA (SCoA) represented by Formula (172) below, the production rate of succinic acid (SUC) represented by Formula (173) below, the production rate of malic acid (MAL) represented by Formula (174) below, the production rate of oxaloacetic acid (OXA) represented by Formula (175) below, the production rate of oxygen ($O_2$) represented by Formula (176) below, and the production rate of carbon dioxide ($CO_2$) represented by Formula (177) below.

$$V \cdot \frac{dC_{ACoA}}{dt} = f_{PYR \rightarrow ACoA} + 8 f_{FAC \rightarrow ACoA} - f_{ACoA \rightarrow CIT} \quad \cdots (169)$$

$$V \cdot \frac{dC_{CIT}}{dt} = f_{ACoA \rightarrow CIT} - f_{CIT \rightarrow aKG} \quad \cdots (170)$$

$$V \cdot \frac{dC_{aKG}}{dt} = f_{CIT \rightarrow aKG} - f_{aKG \rightarrow SCoA} \quad \cdots (171)$$

$$V \cdot \frac{dC_{SCoA}}{dt} = f_{aKG \rightarrow SCoA} - f_{SCoA \rightarrow SUC} \quad \cdots (172)$$

$$V \cdot \frac{dC_{SUC}}{dt} = f_{SCoA \rightarrow SUC} - f_{SUC \rightarrow MAL} \quad \cdots (173)$$

$$V \cdot \frac{dC_{MAL}}{dt} = f_{SUC \rightarrow MAL} - f_{MAL \rightarrow OXA} \quad \cdots (174)$$

$$V \cdot \frac{dC_{OXA}}{dt} = f_{MAL \rightarrow OXA} - f_{ACoA \rightarrow CIT} \quad \cdots (175)$$

$$V \cdot \frac{dC_{O_2}}{dt} = f_{o2} - f_{O2 \to H2O} \quad \cdots(176)$$

$$V \cdot \frac{dC_{CO_2}}{dt} = f_{CO2} + f_{PYR \to ACoA} + f_{CIT \to AKG} + f_{aKG \to SCoA} \quad \cdots(177)$$

Furthermore, in the mitochondria block 224, the CPU 211a calculates the amounts of acetyl coenzyme A (ACoA), citric acid (CIT), $\alpha$-ketoglutaric acid (aKG), succinyl-CoA (SCoA), succinic acid (SUC), malic acid (MAL), oxaloacetic acid (OXA), oxygen ($O_2$), and carbon dioxide ($CO_2$) produced (consumed) in a specific period of time respectively from the thus obtained rates of acetyl coenzyme A (ACoA), citric acid (CIT), $\alpha$-ketoglutaric acid (aKG), succinyl-CoA (SCoA), succinic acid (SUC), malic acid (MAL), oxaloacetic acid (OXA), oxygen ($O_2$), and carbon dioxide ($CO_2$) produced, and calculates the intracellular acetyl coenzyme A (ACoA), citric acid (CIT), $\alpha$-ketoglutaric acid (aKG), succinyl-CoA (SCoA), succinic acid (SUC), malic acid (MAL), oxaloacetic acid (OXA), oxygen ($O_2$), and carbon dioxide ($CO_2$) after the specific period of time.

Furthermore, as described later, the mitochondria block 224 is used both in the first glucose uptake rate estimating process and in the second glucose uptake rate estimating process. In the first glucose uptake rate estimating process, the mitochondria block 224 is used to perform a process that calculates the intracellular acetyl coenzyme A (ACoA), citric acid (CIT), $\alpha$-ketoglutaric acid (aKG), succinyl-CoA (SCoA), succinic acid (SUC), malic acid (MAL), oxaloacetic acid (OXA), oxygen ($O_2$), and carbon dioxide ($CO_2$) in a fasted state. Furthermore, in the second glucose uptake rate calculating process, the mitochondria block 224 is used to perform a process that calculates the intracellular concentrations of the substances in a hyperinsulinemic state.

Supplemental Calculation Process

Furthermore, the CPU 211a performs a supplemental calculation process as described below.

When the ADP concentration is high, a phosphoric acid group is removed from phosphocreatine (PCr) to give creatine (Cr), and ATP is produced from ADP (Formula (39)). The rate of this reaction is represented by Formula (178) below.

$$f_{PCr \to Cr} = V_{PCr \to Cr} \left( \frac{\frac{C_{ADP}}{C_{ATP}}}{K_{\frac{ADP}{ATP}} + \frac{C_{ADP}}{C_{ATP}}} \right) \left( \frac{\frac{C_{PCr}}{K_{PCr}}}{1 + \frac{C_{PCr}}{K_{PCr}} + \frac{C_{Cr}}{K_{Cr}}} \right) \quad \cdots(178)$$

$$K_{PCr} = 20.0$$

$$K_{Cr} = 10.0$$

$$K_{\frac{ADP}{ATP}} = 0.129$$

$$V_{PCr \to Cr} = 20.1$$

Meanwhile, as the ATP concentration is increased, creatine and ATP react with each other to give phosphocreatine and ADP (Formula (41)). The rate of this reaction is represented by Formula (179) below.

$$f_{Cr \to PCr} = V_{Cr \to PCr} \left( \frac{\dfrac{C_{ATP}}{C_{ADP}}}{K_{\frac{ATP}{ADP}} + \dfrac{C_{ATP}}{C_{ADP}}} \right) \left( \frac{\dfrac{C_{Cr}}{K_{Cr}}}{1 + \dfrac{C_{Cr}}{K_{Cr}} + \dfrac{C_{PCr}}{K_{PCr}}} \right) \quad \cdots (179)$$

$$K_{PCr} = 20.0$$

$$K_{Cr} = 10.0$$

$$K_{\frac{ATP}{ADP}} = 7.75$$

$$V_{Cr \to PCr} = 20.1$$

Furthermore, ATP is hydrolyzed and converted to ADP according to Formula (43). The rate of this reaction is represented by Formula (180) below.

$$f_{ATP \to ADP} = V_{ATP \to ADP} \left( \frac{\dfrac{C_{ATP}}{K_{ATP}}}{1 + \dfrac{C_{ATP}}{K_{ATP}}} \right) \quad \cdots (180)$$

$$K_{ATP} = 0.063$$

$$V_{ATP \to ADP} = 0.765544$$

Furthermore, ADP is produced from AMP and ATP by adenylate kinase (Formula (181)).

$$AMP + ATP \to 2ADP \ldots (181)$$

The rate of this reaction is represented by Formula (182) below.

$$f_{AMP \to ADP} = V_{AMP \to ADP} \left( \frac{\dfrac{C_{AMP}}{K_{AMP}} \cdot \dfrac{C_{ATP}}{K_{ATP}}}{1 + \dfrac{C_{AMP}}{K_{AMP}} + \dfrac{C_{ATP}}{K_{ATP}} + \dfrac{C_{AMP}}{K_{AMP}} \cdot \dfrac{C_{ATP}}{K_{ATP}} + \dfrac{C_{ADP}}{K_{ADP}}} \right) \quad \cdots (182)$$

$$K_{AMP} = 0.04$$

$$K_{ATP} = 6.2$$

$$K_{ADP} = 0.8$$

$$V_{AMP \to ADP} = 12.5$$

AMP and ATP are produced from two ABPs by adenylate kinase (Formula (183)).

$$2ADP \to AMP + ATP \dots (183)$$

The rate of this reaction is represented by Formula (184) below.

$$f_{ADP \to AMP} = V_{ADP \to AMP} \left( \frac{\dfrac{C_{ADP}}{K_{ADP}}}{1 + \dfrac{C_{ADP}}{K_{ADP}} + \dfrac{C_{AMP}}{K_{AMP}} + \dfrac{C_{ATP}}{K_{ATP}} + \dfrac{C_{AMP}}{K_{AMP}} \cdot \dfrac{C_{ATP}}{K_{ATP}}} \right) \quad \cdots (184)$$

$$K_{AMP} = 0.04$$

$$K_{ATP} = 6.2$$

$$K_{ADP} = 0.8$$

$$V_{ADP \to AMP} = 12.5$$

The reaction formula in which G6P and ATP in the glycolysis block 223 are reacted via a plurality of processes to give glycogen (GLY) can be simply represented by the following formula.

$$G6P + ATP \to GLY + ADP + 2Pi \dots (185)$$

The rate of this reaction is represented by Formula (186) below.

$$f_{G6P \to GLY} = V_{G6P \to GLY} \left( \frac{\dfrac{C_{ATP}}{C_{ADP}}}{K_{\frac{ATP}{ADP}} + \dfrac{C_{ATP}}{C_{ADP}}} \right) \left( \frac{\dfrac{C_{G6P}}{K_{G6P}}}{1 + \dfrac{C_{G6P}}{K_{G6P}} + \dfrac{C_{GLY}}{K_{GLY}} + \dfrac{C_{Pi}}{K_{Pi}} + \dfrac{C_{GLY}}{K_{GLY}} \cdot \dfrac{C_{Pi}}{K_{Pi}}} \right) \quad \cdots (186)$$

$$K_{G6P} = 0.253$$

$$K_{GLY} = 95.0$$

$$K_{Pi} = 2.7$$

$$K_{\frac{ATP}{ADP}} = 7.75$$

$$V_{G6P \to GLY} = 0.806495 \cdot GS(insulin)$$

In the formula, GS (insulin) is calculated in the insulin signaling block 222.

The reaction formula in which glycogen (GLY) decomposes via a plurality of reactions to give G6P in the glycolysis block 223 can be simply represented by the following formula.

$$GLY \to G6P + Pi \ldots (187)$$

The rate of this reaction is represented by Formula (188) below.

$$f_{GLY \to G6P} = V_{GLY \to G6P} \left( \frac{\dfrac{C_{AMP}}{C_{ATP}}}{K_{\frac{AMP}{ATP}} + \dfrac{C_{AMP}}{C_{ATP}}} \right) \left( \frac{\dfrac{C_{GLY}}{K_{GLY}} \cdot \dfrac{C_{Pi}}{K_{Pi}}}{1 + \dfrac{C_{GLY}}{K_{GLY}} + \dfrac{C_{Pi}}{K_{Pi}} + \dfrac{C_{GLY}}{K_{GLY}} \cdot \dfrac{C_{Pi}}{K_{Pi}} + \dfrac{C_{G6P}}{K_{G6P}}} \right) \quad \cdots (188)$$

$$K_{G6P} = 0.253$$

$$K_{GLY} = 95.0$$

$$K_{Pi} = 2.7$$

$$K_{\frac{AMP}{ATP}} = 0.00645$$

$$V_{GLY \to G6P} = 0.129$$

The reaction formula in which alanine (ALA) is produced from the pyruvic acid (PYR) produced in the glycolysis block 223 and glutamic acid can be simply represented by the following formula.

$$PYR \to ALA \ldots (189)$$

The rate of this reaction is represented by Formula (190) below.

$$f_{PYR \to ALA} = V_{PYR \to ALA} \left( \frac{\dfrac{C_{PYR}}{K_{PYR}}}{1 + \dfrac{C_{PYR}}{K_{PYR}} + \dfrac{C_{ALA}}{K_{ALA}}} \right) \quad \cdots (190)$$

$$K_{PYR} = 0.0475$$

$$K_{ALA} = 1.3$$

$$V_{PYR \to ALA} = 0.006$$

Blood alanine (ALA) is taken up into the cell. The uptake rate $f_{ALA}$ is represented by Formula (191) below.

$$f_{ALA} = Q \cdot \left( C_{ALA,b} - \sigma_{ALA} \cdot C_{ALA} \right) \quad \cdots (191)$$

In the formula, $C_{ALA,b}$ refers to the input blood fatty acid concentration, $C_{ALA}$ refers to the intracellular fatty acid concentration, and $\sigma_{ALA}$ refers to a distribution coefficient relating to fatty acid.

In the supplemental calculation process, the CPU 211a calculates the reaction rates $f_{PCr \to Cr}$, $f_{Cr \to PCr}$, $f_{ATP \to ADP}$, $f_{AMP \to ADP}$, $f_{ADP \to AMP}$, $f_{G6P \to GLY}$, $f_{GLY \to G6P}$, $f_{PYR \to ALA}$, and $f_{ALA}$ represented by Formulae (178), (179), (180), (182), (184), (186), (188), (190), and (191) above, respectively.

Furthermore, the CPU 211a calculates the production rates of substances GLY, ALA, NAD, NADH, ATP, ADP, Pi, PCr, Cr, and CoA according to Formulae (192) to (202) below.

$$V \cdot \frac{dC_{GLY}}{dt} = f_{G6P \to GLY} - f_{GLY \to G6P} \quad \cdots (192)$$

$$V \cdot \frac{dC_{ALA}}{dt} = f_{ALA} + f_{PYR \to ALA} \quad \cdots (193)$$

$$V \cdot \frac{dC_{Cr}}{dt} = f_{PCr \to Cr} - f_{Cr \to PCr} \quad \cdots(194)$$

$$V \cdot \frac{dC_{PCr}}{dt} = f_{Cr \to PCr} - f_{PCr \to Cr} \quad \cdots(195)$$

$$V \cdot \frac{dC_{CoA}}{dt} = f_{ACoA \to CIT} + f_{SCoA \to SUC} - f_{PYR \to ACoA} - f_{FFA \to FAC} + 2 f_{GA3P \to DG} + f_{DG \to TG} \\ - 7 f_{FAC \to ACoA} - f_{aKG \to SCoA} \quad \cdots(196)$$

$$V \cdot \frac{dC_{Pi}}{dt} = 2 f_{G6P \to GLY} + f_{GA3P \to DG} + 2 f_{FFA \to FAC} + f_{ATP \to ADP} - f_{GLY \to G6P} - f_{GA3P \to BPG} \\ - f_{SCoA \to SUC} - 5.64 f_{O2 \to H2O} \quad \cdots(197)$$

$$V \cdot \frac{dC_{ATP}}{dt} = 2 f_{BPG \to PYR} + f_{SCoA \to SUC} + 5.64 f_{O2 \to H2O} + f_{PCr \to Cr} + f_{ADP \to AMP} - f_{GLU \to G6P} \\ - f_{G6P \to GA3P} - 2 f_{FFA \to FAC} - f_{Cr \to PCr} - f_{ATP \to ADP} - f_{AMP \to ADP} \quad \cdots(198)$$

$$V \cdot \frac{dC_{ADP}}{dt} = f_{GLU \to G6P} + f_{G6P \to GLY} + f_{G6P \to GA3P} + 2 f_{FFA \to FAC} + f_{Cr \to PCr} + f_{ATP \to ADP} \\ + 2 f_{AMP \to ADP} - 2 f_{BPG \to PYR} - f_{SCoA \to SUC} - 5.64 f_{O2 \to H2O} - f_{PCr \to Cr} - 2 f_{ADP \to AMP} \quad \cdots(199)$$

$$V \cdot \frac{dC_{AMP}}{dt} = f_{ADP \to AMP} - f_{AMP \to ADP} \quad \cdots(200)$$

$$V \cdot \frac{dC_{NADH}}{dt} = f_{GA3P \to BPG} + f_{LAC \to PYR} + f_{PYR \to ACoA} + \frac{35}{3} f_{FAC \to ACoA} + f_{aKG \to SCoA} + \frac{2}{3} f_{SUC \to MAL} \\ - f_{PYR \to LAC} - 1.87794 f_{O2 \to H2O} - f_{GA3P \to DG} \quad \cdots(201)$$

$$V \cdot \frac{dC_{NAD}}{dt} = f_{PYR \to LAC} + 1.87794 f_{O2 \to H2O} + f_{GA3P \to DG} - f_{GA3P \to BPG} - f_{LAC \to PYR} - f_{PYR \to ACoA} \\ - \frac{35}{3} f_{FAC \to ACoA} - f_{aKG \to SCoA} - \frac{2}{3} f_{SUC \to MAL} \quad \cdots(202)$$

Furthermore, the CPU 21a calculates the amounts of GLY, ALA, NAD, NADH, ATP, ADP, AMP, Pi, PCr, Cr, and CoA produced in a specific period of time respectively from the thus obtained rates of GLY, ALA, NAD, NADH, ATP, ADP, AMP, Pi, PCr, Cr, and CoA produced, and reflects these amounts on the intracellular concentrations of GLY, ALA, NAD, NADH, ATP, ADP, AMP, Pi, PCr, Cr, and CoA at that time, thereby calculating the intracellular concentrations of GLY, ALA, NAD, NADH, ATP, ADP, AMP, Pi, PCr, Cr, and CoA after the specific period of time.

Furthermore, as described later, the supplemental calculation process is used both in the first glucose uptake rate estimating process and in the second glucose uptake rate estimating process. In the first glucose uptake rate estimating process, the supplemental calculation process is executed to perform a process that calculates the intracellular concentrations of GLY, ALA, NAD, NADH, ATP, ADP, AMP, Pi, PCr, Cr, and CoA in a fasted state. Furthermore, in the second glucose uptake rate calculating process, the supplemental calculation process is executed to perform a process that calculates the intracellular concentrations of the substances in a hyperinsulinemic state.

The fatty acid metabolism block 221, the insulin signaling block 222, the glycolysis block 223, and the mitochondria block 224 according to this embodiment were formed with reference to the following documents.

1. A computational model of skeletal muscle metabolism linking cellular adaptations induced by altered loading states to metabolic responses during exercise. Dash RK, Dibella JA 2nd, Cabrera ME. Biorned Eng Online. 2007 Apr 20;6:14. PMID: 17448235

2. Metabolic dynamics in skeletal muscle during acute reduction in blood flow and oxygen supply to mitochondria: in-silico studies using a multi-scale, top-down integrated model.

Dash RK, Li Y, Kim J, Beard DA, Saidel GM, Cabrera ME. PLoS ONE. 2008 Sep 9;3(9):e3168. PMID: 18779864

3. A mathematical model of metabolic insulin signaling pathways.

Sedaghat AR, Sherman A, Quon MJ. Am J Physiol Endocrinol Metab. 2002 Nov;283(5):E1084-101. PMID: 12376338

Operation of the Insulin Resistance Evaluation Supporting System

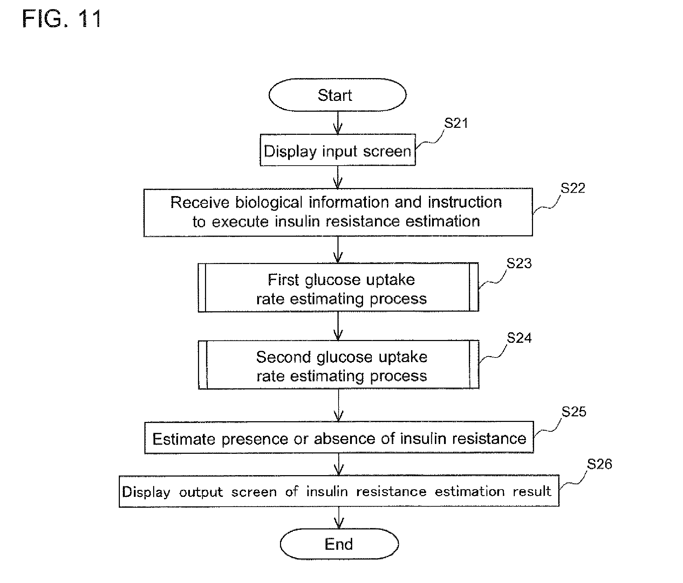

Next, an operation of the insulin resistance evaluation supporting system 201 according to this embodiment will be described. FIG. 11 is a flowchart illustrating a processing flow of the insulin resistance evaluation supporting program according to this embodiment. The computer 201a operates as below by executing the insulin resistance evaluation supporting program 214a. First, the body weight of a subject is weighed in advance with a scale, the skeletal muscle percentage is measured with a body composition monitor, and a blood test is performed. Thus, the biological information of the subject including the body weight, the skeletal muscle percentage, the blood glucose concentration, the plasma insulin concentration, and the blood free fatty acid concentration is acquired. Here, the blood glucose concentration, the plasma insulin concentration, and the blood free fatty acid concentration all measured in a fasted state are used. The biological information is given in advance to a user such as a doctor or an operator who uses the insulin resistance evaluation supporting system.

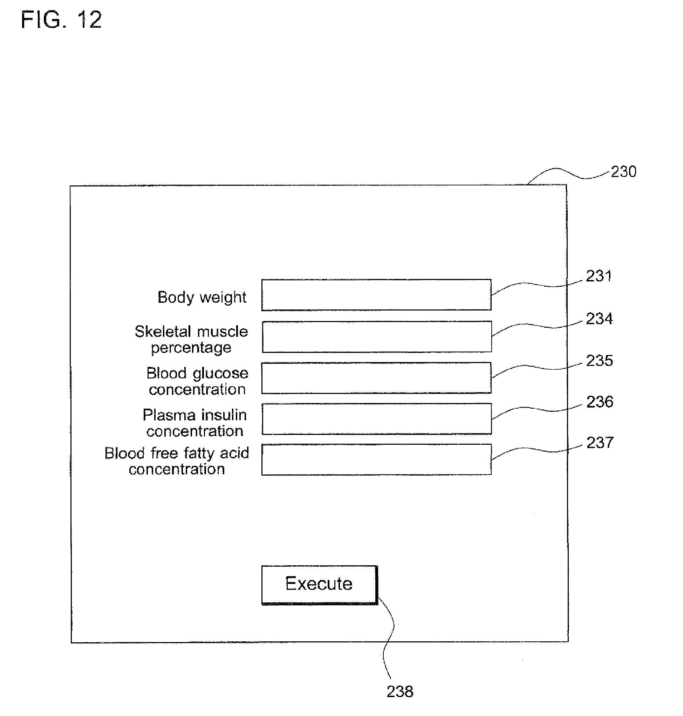

After the insulin resistance evaluation supporting program 214a is started, first, the CPU 211a displays an input screen for prompting the user to input the body weight, the skeletal muscle percentage, the blood glucose concentration, the plasma insulin concentration, and the blood free fatty acid concentration of the subject (step S21). FIG. 12 is a schematic diagram showing an exemplary input screen in the insulin resistance evaluation supporting system 201 according to Embodiment 2. As shown in FIG. 12, an input screen 230 includes input areas 231 and 234 to 237 in which the body weight, the skeletal muscle percentage, the blood glucose concentration, the plasma insulin concentration, and the blood free fatty acid concentration of the subject are to be input, and an Execute button 238 with which an instruction is to be given to execute estimation of the insulin resistance after the biological information is input to the input areas 231 and 234 to 237. The user operates the input portion 213 to input the biological information including the body weight, the skeletal muscle percentage, the blood glucose concentration, the plasma insulin concentration, and the blood free fatty acid concentration of the subject to the input areas 231 and 234 to 237, and selects (clicks on) the Execute button 238 to give an instruction to execute estimation of the insulin resistance. The CPU 211a receives input of the biological information and the execution instruction from the user (step S22). When such biological information is input, the CPU 211a is interrupted, and processes in step S23 and subsequent steps are called.

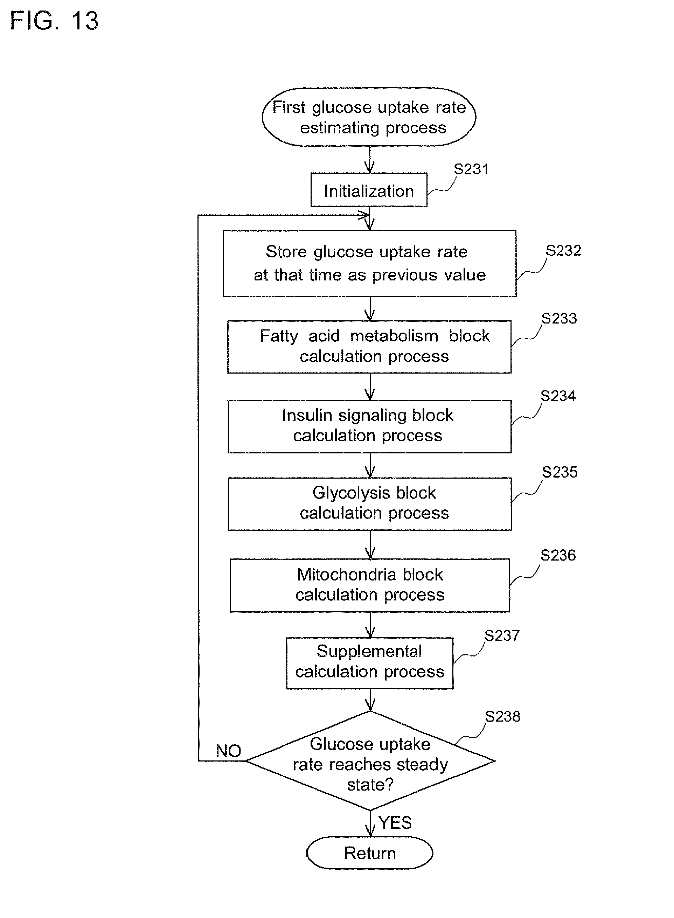

Once input of the biological information and the execution instruction is received from the user, the CPU 211a executes a first glucose uptake rate estimating process (step S23). FIG. 13 is a flowchart illustrating the procedure of the first glucose uptake rate estimating process according to Embodiment 2. The first glucose uptake rate estimating process is a process that sequentially repeats steps S231 to S238, thereby estimating the fasting glucose uptake rate. First, the CPU 211a sets (initializes) the initial value to a variable (step S231). In this process, the initial values of the intracellular concentrations and the reaction rates of the above-described substances in a fasted state are stored in the RAM 211c, and the initial value 0 is stored in the RAM 211c as the glucose uptake rate. Next, the CPU 211a stores the glucose uptake rate at that time in the RAM 211c as the previous glucose uptake rate (step S232). Then, the CPU 211a sequentially executes a fatty acid metabolism block calculation process that is the calculation process in the fatty acid metabolism block 221 (step S233), an insulin signaling block calculation process that is the calculation process in the insulin signaling block 222 (step S234), a glycolysis block calculation process that is the calculation process in the glycolysis block 223 (step S235), a mitochondria block calculation process that is the calculation process in the mitochondria block 224 (step S236), and a supplemental calculation process (step S237). The substance concentrations, the reaction rates, the value according to the GLUT4 appearance amount, and the glucose uptake rate obtained in steps S233 to 237 are values in a fasted state.

Next, the CPU 211a determines whether or not the fasting glucose uptake rate obtained in the above-described process reaches a steady state (step S238). In this embodiment, this process is performed by obtaining a difference between the glucose uptake rate obtained in the current calculation (turn) and the glucose uptake rate in the previous calculation (turn) stored in the RAM 211c, and determining whether or not the difference is less than a first reference value for determining whether or not the glucose uptake rate reaches a steady state. If the difference between the current glucose uptake rate and the previous glucose uptake rate is less than the first reference value (YES in step S238), the CPU 211a returns the process to the call address of the first glucose uptake rate estimating process in the main routine, and, if the difference is at least the first reference value (NO in step S238), the CPU 211a repeats the processes in step S232 and subsequent steps.

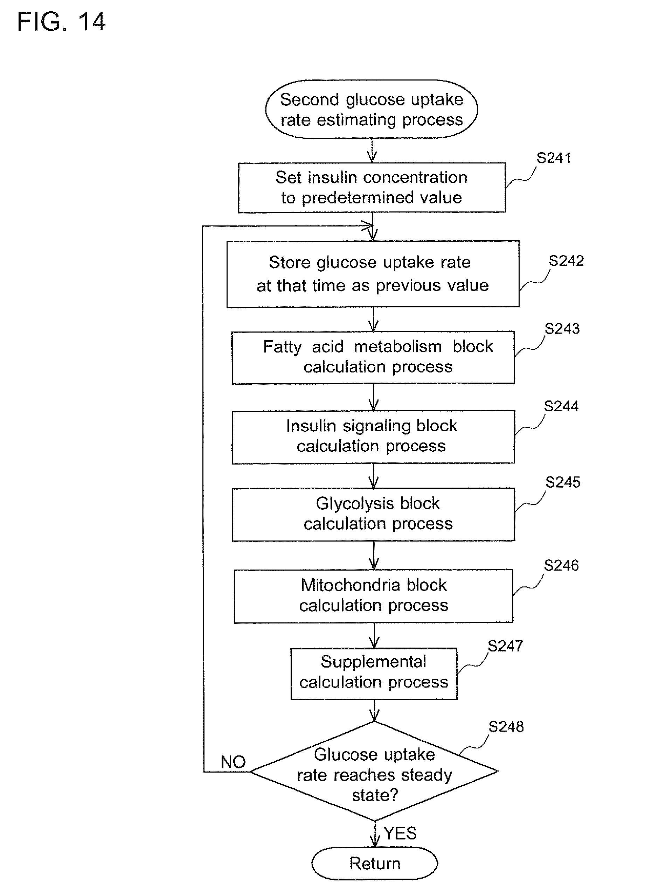

Next, the CPU 211a executes a second glucose uptake rate estimating process (step S24), FIG. 14 is a flowchart illustrating the procedure of the second glucose uptake rate estimating process according to Embodiment 2. The second glucose uptake rate estimating process is a process that sequentially repeats steps S241 to S248, thereby estimating the glucose uptake rate in a hyperinsulinemic state. First, the CPU 211a sets the insulin concentration to a predetermined value (insulin concentration in a hyperinsulinemic state) (step S241). Here, in the second glucose uptake rate estimating process, as initial values of the fasting intracellular substance concentrations other than the insulin concentration, the reaction rates, and the glucose uptake (GLUT), the values finally obtained in the first glucose uptake rate estimating process, that is, the substance concentrations, the reaction rates, and the glucose uptake when the glucose uptake rate reaches the steady state are used as they are. As for the blood glucose concentration, the fasting glucose concentration used in the first glucose uptake rate estimating process is used as it is. Furthermore, the initial value 0 is stored in the RAM 211c as the glucose uptake rate. Next, the CPU 211a stores the glucose uptake rate at that time in the RAM 211c as the previous glucose uptake rate (step S242). Then, the CPU 211a sequentially executes a fatty acid metabolism block calculation process that is the calculation process in the fatty acid metabolism block 221 (step S243), an insulin signaling block calculation process that is the calculation process in the insulin signaling block 222 (step S244), a glycolysis block calculation process that is the calculation process in the glycolysis block 223 (step S245), a mitochondria block calculation process that is the calculation process in the mitochondria block 224 (step S246), and a supplemental calculation process (step S247). The substance concentrations, the reaction rates, the value according to the GLUT4 appearance amount, and the glucose uptake rate obtained in steps S243 to 247 are values in a hyperinsulinemic state.

Next, the CPU 211a determines whether or not the glucose uptake rate in a hyperinsulinemic state obtained in the above-described process reaches a steady state (step S248). In this embodiment, this process is performed by obtaining a difference between the glucose uptake rate obtained in the current calculation (turn) and the glucose uptake rate in the previous calculation (turn) stored in the RAM 211c, and determining whether or not the difference is less than the first reference value for determining whether or not the glucose uptake rate reaches a steady state. Here, the configuration is adopted in which, in the first glucose uptake rate estimating process and the second glucose uptake rate estimating process, the same first reference value is used to determine whether or not the glucose uptake rate reaches the steady state, but this is not a limitation, and different reference values may be respectively used in these processes. Then, if the difference between the current glucose uptake rate and the previous glucose uptake rate is less than the first reference value (YES in step S248), the CPU 211a returns the process to the call address of the second glucose uptake rate estimating process in the main routine, and, if the difference is at least the first reference value (NO in step S248), the CPU 211a repeats the processes in step S242 and subsequent steps.

Next, the CPU 211a estimates the presence or absence of the insulin resistance (step S25). This process is performed by determining whether or not the glucose uptake rate (estimated value) per unit muscle amount obtained in step S248 is at least a second reference value (e.g., 12.0 mg/kg/min if the insulin concentration during the glucose clamp is approximately 3480 pM, although it varies by race) for estimating the presence or absence of the insulin resistance. Accordingly, if the estimated glucose uptake rate is at least the second reference value, it is possible to estimate that the insulin resistance is not present, that is, the insulin sensitivity is present. Furthermore, if the estimated glucose uptake rate is less than the second reference value, it is possible to estimate that the insulin resistance is present, that is, the insulin sensitivity is not present. In this manner, the CPU 211a estimates the insulin resistance. Here, the second reference value is assumed to be 12.0 mg/kg/min based on the following documents.
Critical evaluation or adult treatment panel III criteria in identifying insulin resistance with dyslipidemia.
Liao Y, Kwon S, Shaughnessy S, Wallace P, Hutto A, Jenkins AJ, Klein RL, Garvey WT. Diabetes Care. 2004 Apr;27 (4):978-83.PMID:15047659

Next, the CPU 11a displays an output screen for outputting the estimation results of the insulin resistance (step S26). This output screen includes an insulin resistance estimation result obtained in the above-described process and a finally obtained estimated glucose uptake rate per unit muscle amount. With the output screen, the user is notified of the insulin resistance estimation result and the estimated glucose uptake rate. As the insulin resistance estimation result and the estimated glucose uptake rate are provided to the user in this manner, the user can use the information to perform evaluation of the insulin resistance. Furthermore, the display screen can be changed from this screen to a simulation result screen as described later. The configuration of the simulation result screen will be described later.

With this sort of configuration, it is possible to estimate the presence or absence of the insulin resistance using the biological information including "body weight", "skeletal muscle percentage", "fasting blood glucose concentration", "fasting plasma insulin concentration", and "fasting blood free fatty acid concentration" that can be obtained with a simple test without placing a heavy burden on the subject, instead of requiring test results of a glucose clamp test and an oral glucose tolerance test that place a heavy burden on the subject. Among these pieces of input information, regarding the fasting blood glucose concentration and plasma insulin concentration, test values easily obtained with a blood test can be used as described above, but test values obtained with a glucose clamp test or an oral glucose tolerance test may also be used as the input information. However, this system is useful in that results of tests such as a glucose clamp test and an oral glucose tolerance test that place a heavy burden on the subject are not always required and results of a simple blood test can also be used instead of such test results, and in that information used as the input information can be freely obtained from any of the blood test, the glucose clamp test, and the oral glucose tolerance test.

Here, in Embodiments 1 and 2 above, the configuration is adopted in which, in the second glucose uptake rate estimating process, the insulin concentration in a hyperinsulinemic state is set in order to reproduce the physical condition of the subject during a glucose clamp test, the glucose concentration is not changed from that in the first glucose uptake rate estimating process, and the fasting glucose concentration is used as it is, but this is not a limitation. A glucose clamp test that evaluates the insulin resistance is normally performed at a normal blood glucose level and a high insulin state. In a glucose clamp test, for example, there are cases in which insulin and glucose are administered to the subject such that the fasting blood glucose concentration is maintained or in which insulin and glucose are administered to the subject such that the blood glucose concentration is kept at 100 mg/dL. The latter case can be reproduced by not only setting an insulin concentration as found in a hyperinsulinemic state but also by setting a blood glucose concentration to be 100 mg/dL in the second glucose uptake rate estimating process.

Furthermore, in Embodiment 2, the configuration is adopted in which the glucose uptake rate per unit muscle amount is estimated and output, but the configuration may be adopted in which the glucose uptake rate per unit weight is obtained and output as in Embodiment 1.

Furthermore, in Embodiments 1 and 2, the configuration is adopted in which, until the glucose uptake rate reaches a steady state, the processes from the first, metabolism amount calculation process to the supplemental calculation process are repeated, and the reaction rate, the production rate, and the intracellular concentration of each substance are repeatedly updated. Accordingly, when the glucose uptake rate reaches a steady state, the intracellular concentration of each substance is expected to be in a steady state, and the concentration of each substance in this steady state can be considered to reflect the physical condition of the subject. Accordingly, with this sort of configuration, it is possible to estimate the glucose uptake rate precisely reflecting the physical condition of the subject.

Performance Evaluation Experiment of the Insulin Resistance Evaluation Supporting System

A performance evaluation experiment of the insulin resistance evaluation supporting system 201 according to Embodiment 2 was performed. One of the documents reporting result of glucose clamp tests is a report by Basu et al. (Basu et al., Obesity and Type 2 Diabetes Impair Insulin-Induced Suppression of Glycogenolysis as well as gluconeogenesis, Diabetes 54:1942-1948, 2005). The document by Basu et al. uses a glucose clamp test to perform a comparison between 10 non-diabetic lean-type subjects (Lean), 10 non-diabetic obese subjects (Obese), and 11 subjects suffering from type 2 diabetes (DM2), and mainly discusses the liver insulin resistance. In this experiment, data in the document was used, and a difference between the glucose appearance rate and the endogeneous glucose production as a GIR value.

Table 1 below shows a summary of Lean, Obese, and DM2 measurement data shown in the above-described document.

Table 1

| | Lean (n=10) | Obese (n=10) | DM2 (n=11) |
|---|---|---|---|
| Blood glucose level (basal, mM) | $5.2 \pm 0,1$ | $5.4 \pm 0.1$ | $10.4 \pm 0.8$ |
| Blood insulin concentration (basal, pM) | $19 \pm 2$ | $47 \pm 9$ | $52 \pm 9$ |
| Blood free fatty acid concentration (basal, mM) | $0.39 \pm 0.03$ | $0.35 \pm 0.02$ | $0.41 \pm 0.03$ |
| Blood glucose level (clamp, mM) | $5 \pm 0.1$ | $5.1 \pm 0.1$ | $5.5 \pm 0.3$ |
| Blood insulin concentration (clamp, pM) | $138 \pm 8$ | $139 \pm 10$ | $144 \pm 4$ |
| Blood free fatty acid concentration (clamp, mM) | $0.03 \pm 0.00$ | $0.05 \pm 0.01$ | $0.09 \pm 0.02$ |
| Glucose appearance rate (clamp, umol/kg LBM/min) | $26.1 \pm 2.2$ | $19.2 \pm 0.6$ | $17,5 \pm 2.5$ |
| Glucose production rate (clamp, umol/kg LBM/min) | -1.6:t 1.1 | $1.9 \pm 0.5$ | $5.4 \pm 1.0$ |
| Glucose uptake rate (umol/kg LBM/min) | 27.7 | 17.3 | 12.2 |

In this experiment, first, the fasting blood glucose level, insulin level, and free fatty acid concentration of Lean, Obese, and DM2 measurement data shown in Table 1 above were input to the insulin resistance evaluation supporting system 201, and the concentration and the reaction rate of each substance in a steady state were obtained following the procedure of the first glucose uptake rate estimating process. Tables 2 to 4 show initial values used in the first glucose uptake rate estimating process, and variables (variables of the concentration, the reaction rate, etc.) obtained in the steady state after the estimating process (hereinafter, referred to as "estimated values"). Table 2 shows initial values and estimated values of the concentrations of the substances in the insulin resistance evaluation supporting system 201, Table 3 shows initial values and estimated values of parameters (variables of items other than the concentration and the reaction rate of the substances) in the insulin signaling block 222, and Table 4 shows initial values and estimated values of the reaction rates of the substances in the insulin resistance evaluation supporting system 201.

Table 2

| Concentration (mM) | Initial value | Lean | Obese | DM2 |
|---|---|---|---|---|
| GLU | 0.525 | 0.649983 | 0.674035 | 1.310537 |
| G6P | 0.253 | 0.53887 | 1.1.02946 | 1.050117 |
| GA3P | 0.15 | 0.134342 | 0.210629 | 0.202406 |
| BPG | 0.08 | 0.13321 | 0.196438 | 0.189717 |
| PYR | 0.0475 | 0.062306 | 0.081776 | 0.082193 |
| GLY | 95 | 194.1144 | 395.4255 | 363.173 |
| ALA | 1.3 | 1.343075 | 1.388607 | 1.389474 |
| LAC | 1.75 | 1.846256 | 1.973701 | 1.991309 |
| FFA | 0.57 | 0.35733 | 0.32887 | 0.392824 |
| FAC | 0.00348 | 0.001979 | 0.001459 | 0.001682 |
| DG | 0.329 | 0.269927 | 0.315285 | 0.369725 |
| TGL | 14.8 | 6.764592 | 6.2884 | 8.740677 |
| GLR | 0.062 | 0.061591 | 0.065435 | 0.06878 |
| PKCtheta (dimensionless) | 0 | 0.39338 | 0.626815 | 1.0108 |
| ACoA | 0.00223 | 0.001449 | 0.00127 | 0.001418 |
| CIT | 0.103 | 0.088031 | 0.082903 | 0.087278 |
| aKG | 0.0125 | 0.010256 | 0.009479 | 0.010114 |
| SCoA | 0.123 | 0.1218 | 0.120945 | 0.121784 |
| SUC | 0.095 | 0.095387 | 0.095162 | 0.095358 |
| MAL | 0.0975 | 0.113954 | 0.12026 | 0.114803 |
| OXA | 0.003 | 0.004573 | 0.00525 | 0.004663 |
| AMP | 0.04 | 0.042285 | 0.045986 | 0.045489 |
| ADP | 0.8 | 0.839936 | 0.903477 | 0.895026 |
| ATP | 6.2 | 6.157779 | 6.090537 | 6.099485 |
| NAD | 0.45 | 0.452236 | 0.452843 | 0.452018 |
| NADH | 0.05 | 0.047764 | 0.047157 | 0.047982 |
| $O_2$ | 3 | 3.018377 | 3.018418 | 3.011265 |
| $CO_2$ | 23.6 | 23.67135 | 23.74773 | 23.73378 |
| CoA | 0.0255 | 0.028983 | 0.030536 | 0.029326 |
| Cr | 10 | 10.37368 | 10.95022 | 10.87478 |
| PCr | 20 | 19.62632 | 19.04978 | 19.12522 |
| Pi | 2.7 | 2.67155 | 2.552223 | 2.541827 |

Table 3

| Variable | Initial value | Lean | Obese | DM2 |
|---|---|---|---|---|
| Insulin | 0 | 1.90E-11 | 4.70E-11 | 5.20E-11 |
| $X_2$ | 9.00E-13 | 2.14E-12 | 2.12E-12 | 2.11E-12 |
| $X_3$ | 0 | 9.77E-19 | 2.39E-18 | 2.63E-18 |
| $X_4$ | 0 | 6.97E-19 | 4.25E-18 | 5.17E-18 |
| $X_5$ | 0 | 1.22E-14 | 3.02E-14 | 3.31E-14 |
| $X_6$ | 1.00E-13 | 2.52E-13 | 2.61E-13 | 2.63E-13 |
| $X_7$ | 0 | 3.22E-21 | 1.98E-20 | 2.40E-20 |
| $X_8$ | 0 | 5.64E-17 | 1.41E-16 | 1.54E-16 |
| $X_9$ | 1.00E-12 | 3.80E-13 | 2.74E-13 | 1.92E-13 |
| $X_{10}$ | 0 | 1.56E-14 | 2.82E-14 | 2.15E-14 |
| $X_{10a}$ | 0 | 6.05E-13 | 6.97E-13 | 7.86E-13 |
| $X_{11}$ | 1.00E-13 | 9.99E-14 | 9.98E-14 | 9.98E-14 |
| $X_{12}$ | 0 | 1.10E-16 | 1.98E-16 | 1.52E-16 |
| $X_{13}$ | 0.31 | 0.437403 | 0.538864 | 0.485206 |
| $X_{14}$ | 99.4 | 99.15341 | 98.95704 | 99.06089 |
| $X_{15}$ | 0.29 | 0.409183 | 0.504099 | 0.453902 |
| $X_{16}$ | 100 | 99.54543 | 99.18637 | 99.37594 |
| $X_{17}$ | 0 | 0.454566 | 0.813628 | 0.624059 |
| $X_{18}$ | 100 | 99.54543 | 99.18637 | 99.37594 |
| $X_{19}$ | 0 | 0.454566 | 0.813628 | 0.624059 |
| $X_{20}$ | 96 | 95.99364 | 95.99429 | 95.99659 |
| $X_{21}$ | 4 | 6.999628 | 9.369347 | 8.118478 |
| $X_{22}$ | 80 | 1.904779 | 1.835724 | 1.871538 |
| $X_{23}$ | 20 | 0.095221 | 0.164276 | 0.128462 |
| PI3K | 5.00E-15 | 2.55E-15 | 2.55E-15 | 2.55E-15 |
| PKC | 0 | 0.39338 | 0.627237 | 1.010946 |
| PTEN | 1 | 1 | 1 | 1 |
| SHIP | 1 | 1 | 1 | 1 |
| PTP | 1 | 0.987499 | 0.977625 | 0.982838 |

Table 4

| Reaction rate (mmol/kg/min) | Initial value | Lean | Obese | DM2 |
|---|---|---|---|---|
| $f_{GLU \to G6P}$ | 0.012275 | 0.018296 | 0.025946 | 0.024912 |
| $f_{G6P \to GA3P}$ | 0.012275 | 0.016818 | 0.021663 | 0.02134 |
| $f_{GA3P \to BPG}$ | 0.0182 | 0.027334 | 0.036465 | 0.035329 |
| $f_{BPG \to PYR}$ | 0.0182 | 0.027334 | 0.036464 | 0.035329 |
| $f_{G6P \to GLY}$ | 0.0129 | 0.017905 | 0.02328 | 0.021986 |

(continued)

| Reaction rate (mmol/kg/min) | Initial value | Lean | Obese | DM2 |
|---|---|---|---|---|
| $f_{GLY \to G6P}$ | 0.0129 | 0.01643 | 0.019004 | 0.01842 |
| $f_{PYR \to ALA}$ | 0.002 | 0.002353 | 0.002726 | 0.002733 |
| $f_{PYR \to LAC}$ | 0.0147 | 0.016413 | 0.018602 | 0.018931 |
| $f_{LAC \to PYR}$ | 0.0091 | 0.008772 | 0.008258 | 0.008214 |
| $F_{FFA \to FAC}$ | 0.0238 | 0.02063 | 0.020506 | 0.022433 |
| $f_{GA3P \to DG}$ | 0.00635 | 0.006301 | 0.006859 | 0.00735 |
| $f_{DG \to TG}$ | 0.00635 | 0.004175 | 0.003639 | 0.004336 |
| $f_{TG \to DG}$ | 0.00635 | 0.004165 | 0.003631 | 0.004327 |
| $F_{DG \to GLR}$ | 0.00635 | 0.00629 | 0.006852 | 0.00734 |
| $f_{PYR \to ACoA}$ | 0.0109 | 0.016779 | 0.021703 | 0.020163 |
| $f_{FAC \to ACoA}$ | 0.00475 | 0.003853 | 0.003149 | 0.003398 |
| $f_{ACoA \to CIT}$ | 0.0489 | 0.047601 | 0.046895 | 0.047345 |
| $f_{CIT \to aKG}$ | 0.0489 | 0.047601 | 0.046895 | 0.047345 |
| $f_{aKG \to SCoA}$ | 0.0489 | 0.047601 | 0.046895 | 0.047345 |
| $f_{SCoA \to SUC}$ | 0.0489 | 0.047601 | 0.046895 | 0.047345 |
| $f_{SUC \to MAL}$ | 0.0489 | 0.047601 | 0.046895 | 0.047345 |
| $f_{MAL \to OXA}$ | 0.0489 | 0.047601 | 0.046895 | 0.047345 |
| $f_{O2 \to H2O}$ | 0.134325 | 0.132943 | 0.132939 | 0.133477 |
| $f_{ATP \to ADP}$ | 0.757843 | 0.757791 | 0.757706 | 0.757717 |
| $f_{AMP \to ADP}$ | 2.5 | 2.548276 | 2.618747 | 2.609796 |
| $f_{ADP \to AMP}$ | 2.5 | 2.548276 | 2.618747 | 2.609796 |
| $f_{PCr \to Cr}$ | 33.35 | 33.42742 | 33.4471 | 33.45116 |
| $f_{Cr \to PCr}$ | 33.35 | 33.42742 | 33.44709 | 33.45116 |

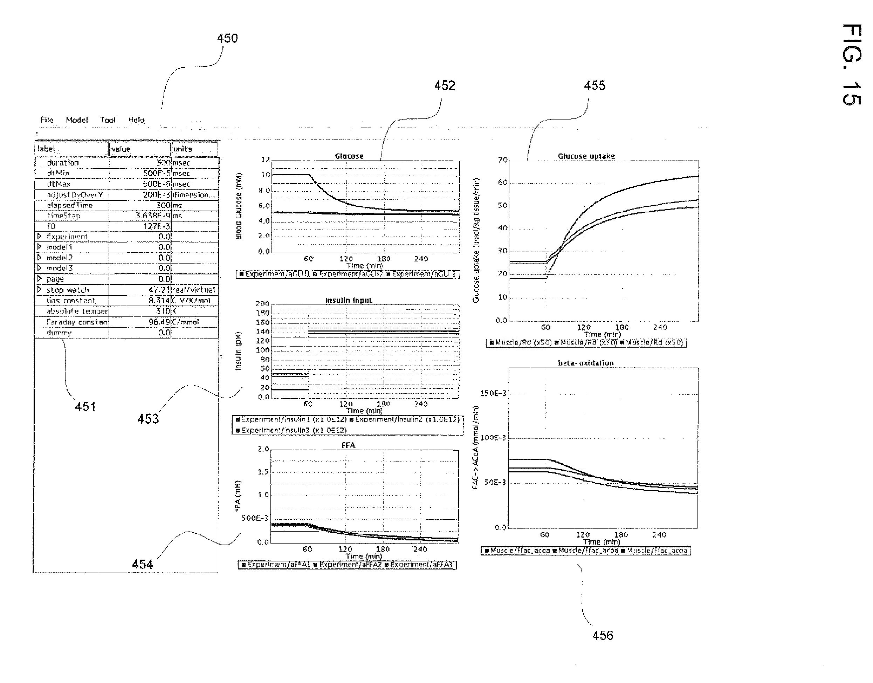

A glucose clamp test simulation was performed by a second glucose uptake rate estimating means using, as initial values, the estimated values of the variables after the first glucose uptake rate estimating process shown in Tables 2 to 4. In this glucose clamp test simulation, estimated values in Table 1 were used as the concentrations of blood glucose, blood insulin, and blood fatty acid during the clamp (60 minutes before insulin injection), and a 300-minute process from 60 minutes before the insulin injection to 240 minutes after the injection was simulated. FIG. 15 is a diagram showing a simulation result display screen in this evaluation experiment.
As shown in FIG. 15, a simulation result display screen 450 includes a parameter display area 451 that displays set values of various parameters used in a simulation and graphs 452 to 455 that show simulation results. The graph 452 shows a change over time in the blood glucose concentration, the graph 453 shows a change over time in the blood insulin concentration, the graph 454 shows a change over time in the blood fatty acid concentration, the graph 455 shows a change over time in the glucose uptake rate, and the graph 456 shows a change over time in the blood fatty acid oxidization rate.

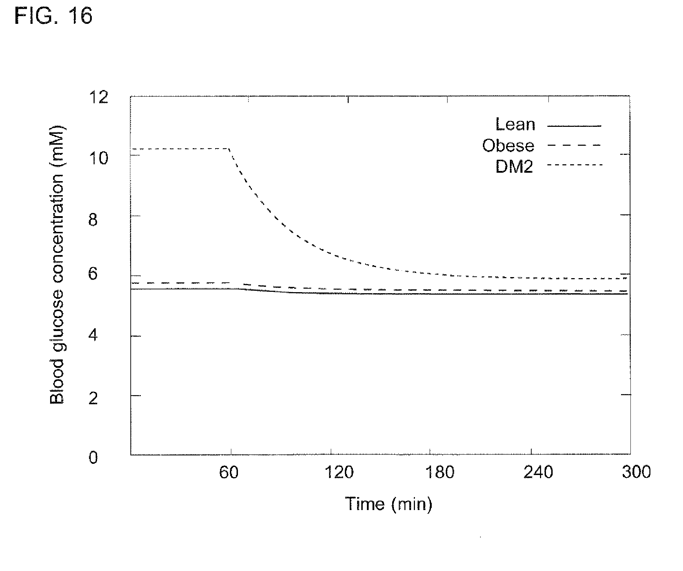

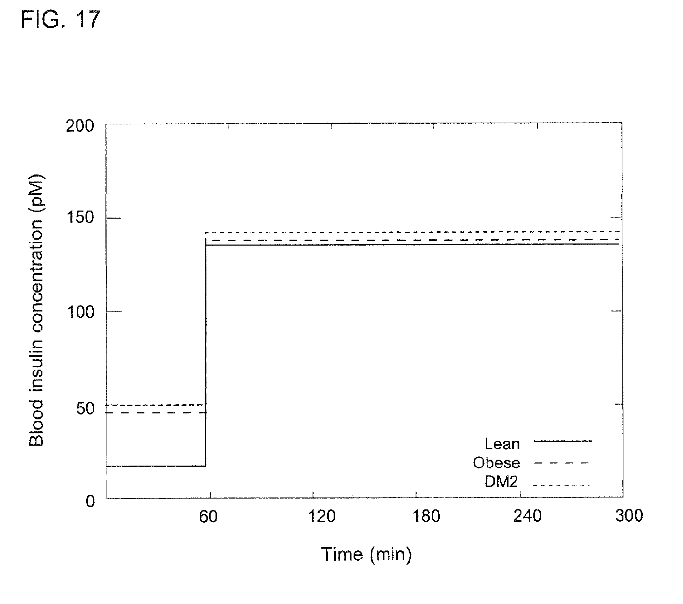

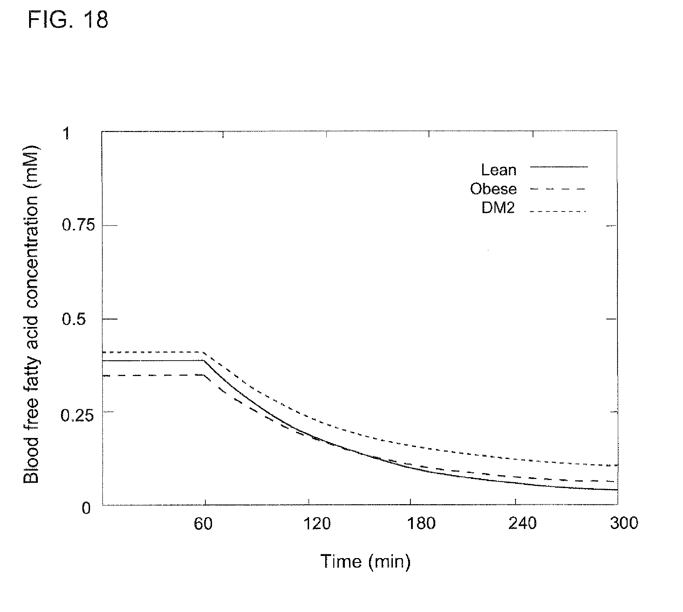

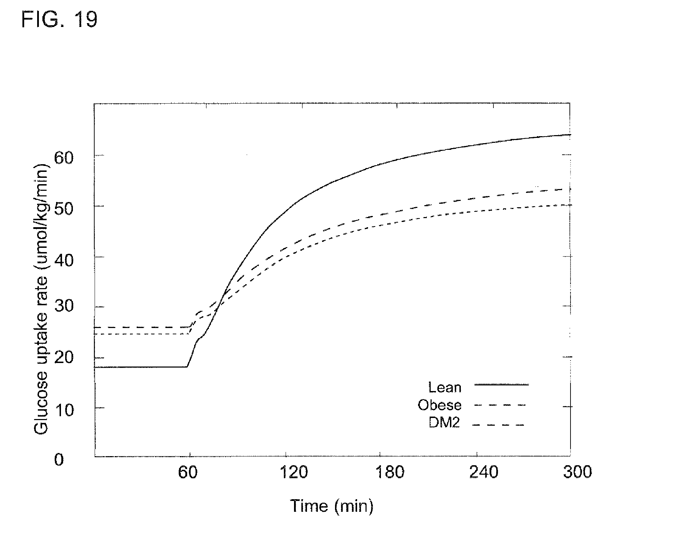

FIGS. 16 to 19 show the simulation results in more detail. FIG. 16 is a graph showing simulation results of the blood glucose concentration, FIG. 17 is a graph showing simulation results of the blood insulin concentration, FIG. 18 is a graph showing simulation results of the blood fatty acid concentration, and FIG. 19 is a graph showing simulation results of the glucose uptake rate.

As shown in FIG. 16, the blood glucose concentrations of Lean and Obese were substantially constant at approximately 5 mM throughout the simulation period. Meanwhile, the blood glucose concentration of DM2 was constant at approximately 10 mM for 60 minutes before the insulin injection, and decreased after the insulin injection. As shown in FIG. 17, the blood insulin concentration of Lean was constant at approximately 20 pM for 60 minutes before the insulin injection, and the blood insulin concentrations of Obese and DM2 were constant at approximately 45 to 50 pM in the same period. Furthermore, the blood insulin concentrations of Lean, Obese, and DM2 were all constant at approximately 140 pM in the period after the insulin injection. As shown in FIG. 18, the blood fatty acid concentrations of Lean, Obese, and DM2 were constant at approximately 0.3 to 0.4 mM for 60 minutes before the insulin injection. Furthermore, the concentrations of Lean, Obese, and DM2 were all decreased after the insulin injection, and the extents or decrease were diminished over time. As shown in FIG. 19, the glucose uptake rate of Lean was constant at approximately 18 umol/kg/min for 60 minutes before the insulin injection, and increased after the insulin injection. Furthermore, the extent of increase was diminished over time. The glucose uptake rates of Obese and DM2 were constant at approximately 25 umol/kg/min for 60 minutes before the insulin injection, and increased after the insulin injection. As a result of the simulation, the glucose uptake rates of Lean, Obese, and DM2 at 240 minutes after the insulin injection were respectively 63.3 umol/kg/min, 53.1 umol/kg/min, and 49.82 umol/kg/min.

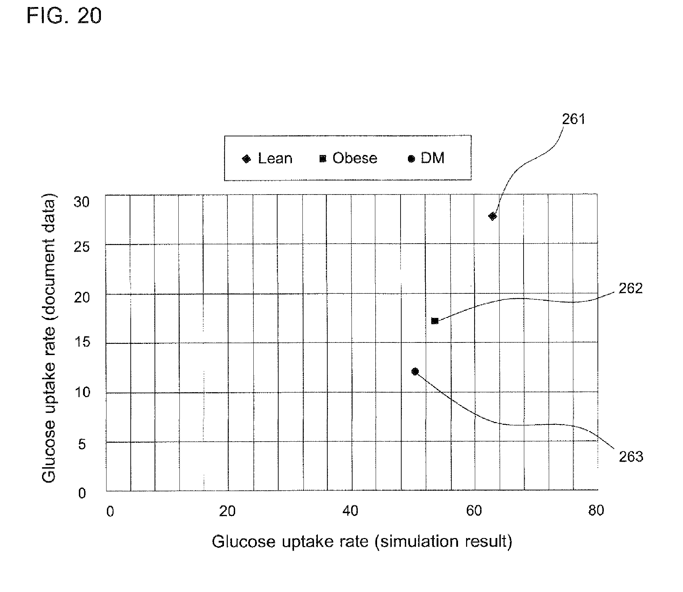

FIG. 20 is a graph showing a result of comparison between measured values provided in a document and simulation results. In FIG. 20, the vertical axis indicates the glucose uptake rate shown in the document, and the horizontal axis indicates the glucose uptake rate estimated by the insulin resistance evaluation supporting system 201. As shown in FIG. 20, a corresponding point 261 between measured glucose uptake rates of Lean shown in the document and estimated glucose uptake rates of Lean by the insulin resistance evaluation supporting system 201, a corresponding point 262 between measured glucose uptake rates of Obese shown in the document and estimated glucose uptake rates of Obese by the insulin resistance evaluation supporting system 201, and a corresponding point 263 between measured glucose uptake rates of DM2 shown in the document and estimated glucose uptake rates of DM2 by the insulin resistance evaluation supporting system 201 are arranged on a straight line. Accordingly, the size relationship of measured glucose uptake rates of Lean, Obese, and DM2 and the size relationship of estimated glucose uptake rates do not contradict each other, but sufficiently correspond to each other. Thus, it can be seen that the insulin resistance evaluation supporting system 241 precisely estimates the glucose uptake rate. Furthermore, if the glucose uptake rate is more precisely estimated, the regression line has to pass through the origin. This can be achieved by adjusting the coefficients and the like in the above-stated formulae.

Other Embodiments

In Embodiments 1 and 2 above, the configuration is adopted in which the glucose uptake rate is estimated, the presence or absence of the insulin resistance is estimated based on the estimation result, and the insulin resistance estimation result and the estimated glucose uptake rate are displayed on an output screen, but this is not a limitation. As another embodiment, the configuration may be adopted in which a screen showing only the insulin resistance estimation result (e.g., a message indicating "it is estimated that the insulin resistance is present" or "it is estimated that the insulin resistance is not present") is displayed. Furthermore, the configuration may be adopted in which the process in step S7 that estimates the presence or absence of the insulin resistance is not performed, and an output screen showing the estimated glucose uptake rate per unit weight is displayed when the calculated glucose uptake rate in a hyperinsulinemic state reaches a steady state. In this case, information that supports evaluation of insulin resistance (e.g., a message indicating "it is estimated that the insulin resistance is not present if the glucose uptake rate is 12 mg/kg/min or more") may be simultaneously displayed.

Furthermore, in Embodiment 1 above, the configuration is adopted in which the input information includes the measured body weight and skeletal muscle percentage, the muscle amount is obtained from the body weight and the skeletal muscle percentage, and the glucose uptake rate per unit weight is obtained from this muscle amount, but this is not a limitation. As another embodiment, the configuration may be adopted in which the input information includes the measured muscle amount, and the glucose uptake rate per unit weight is obtained from this muscle amount.

Furthermore, in Embodiment 1 above, the configuration is adopted in which the rate of acetyl-CoA produced in the β-oxidization is adjusted due to the malonyl-CoA concentration, but this is not a limitation. As another embodiment, the configuration may be adopted in which the rate of acetyl-CoA produced in the β-oxidization is obtained without consideration of suppression due to malonyl-CoA.

Furthermore, in Embodiments 1 and 2 above, the configuration is adopted in which the input information includes the free fatty acid concentration, but this is not a limitation. As another embodiment, the configuration may be adopted in which the input information includes the triglyceride concentration instead of the free fatty acid concentration, and the free fatty acid concentration is calculated from this triglyceride concentration.

Furthermore, in Embodiment 1 above, the configuration is adopted in which the input information includes the amount of oxygen consumed and the amount of carbon dioxide produced per unit time in the skeletal muscle and the skeletal muscle percentage, but this is not a limitation. As another embodiment, the input information may include the amount of oxygen consumed and the amount of carbon dioxide produced per unit time in the whole body and the body fat percentage. In this case, the configuration is adopted in which the rate of oxygen consumed in the cell is obtained from the amount of oxygen consumed per unit time in the whole body and the body fat percentage, and the amount of carbon dioxide produced in the cell is obtained from the amount of carbon dioxide produced per unit time in the whole body and the body fat percentage.

Furthermore, in Embodiments 1 and 2 above, it is assumed that the ATP hydrolysis rate does not depend on the insulin concentration, but it is known that glycogen synthesis and sodium-potassium pump including ATP hydrolysis are activated actually depending on the insulin concentration. Accordingly, the present invention is not limited to the above-described configuration, and the ATP hydrolysis rate may change according to the insulin concentration.

Furthermore, in Embodiments 1 and 2 above, the configuration is adopted in which a value according to the GLUT4 appearance amount is calculated using the insulin amount and the fatty acyl-coenzyme A complex concentration without consideration of the diacylglycerol concentration, but this is not a limitation. It is known that the GLUT4 appearance amount is affected not only by the insulin amount and the fatty acyl-coenzyme A complex concentration but also by the diacylglycerol concentration. Thus, as another embodiment, the configuration may be adopted in which a value GLUT according to the GLUT4 appearance amount is obtained also using the diacylglycerol concentration.

Furthermore, in Embodiments 1 and 2 above, the configuration was described in which one computer 1a or 201a is caused to function as the insulin resistance evaluation supporting system 1 or 201 by causing the CPU 11a or 21a of the computer 1a or 201a to execute the insulin resistance evaluation supporting program 14a or 214a, but this is not a limitation, and the insulin resistance evaluation supporting system may be configured from a dedicated hardware circuit for executing a process substantially the same as that of the insulin resistance evaluation supporting program 14a or 214a.

Furthermore, in Embodiments 1 and 2 above, the configuration was described in which all processes in the insulin resistance evaluation supporting program are executed by a single computer 1a or 201a, but this is not a limitation, and a distributed system may be adopted in which a process similar to that of the above-described insulin resistance evaluation supporting program is executed by a plurality of apparatuses (computers) in a distributed manner.

Industrial Applicability

The insulin resistance evaluation supporting system, the insulin resistance evaluation supporting method, and the computer program of the present invention are useful, for example, as an insulin resistance evaluation supporting system, an insulin resistance evaluation supporting method, and a computer program, respectively, that support evaluation of the insulin resistance of a subject.

Description of Reference Numerals

| | |
|---|---|
| 1, 201 | Insulin resistance evaluation supporting system |
| 1a, 201a | Computer |
| 11, 211 | Main unit |
| 12, 212 | Image display portion |
| 13, 213 | Input portion |
| 11d, 211d | Hard disk |
| 11g, 211g | Image output interface |
| 11e, 211e | Reading device |
| 11f, 211f | Input/output interface |
| 14, 214 | Portable storage medium |
| 14a, 214a | Insulin resistance evaluation supporting program |
| 21, 221 | Fatty acid metabolism block |
| 22, 222 | Insulin signaling block |
| 23, 223 | Glycolysis block |
| 24, 224 | Mitochondria block |
| 3, 230 | Input screen |
| 31 to 37, 231, 234 to 237 | Input area |
| 38, 238 | OK button |
| 4 | Output screen |
| 41 | Insulin resistance estimation result |
| 42 | Estimated glucose uptake rate |

**Claims**

1. An insulin resistance evaluation supporting system for supporting evaluation of insulin resistance of a subject, comprising:

   an input means that receives input of information relating to a glucose concentration, an insulin concentration, and a free fatty acid concentration in blood obtained by measuring the subject; and
   an estimating means that estimates a glucose uptake rate of the subject based on the information relating to the glucose concentration, the insulin concentration, and the free fatty acid concentration in the blood whose input has been received by the input means.

2. The insulin resistance evaluation supporting system according to claim 1, wherein the estimating means comprises:

   a first estimating means that estimates a value according to a glucose transporter appearance amount based on the information relating to the insulin concentration and the free fatty acid concentration in the blood; and
   a second estimating means that estimates a glucose uptake rate based on the value according to the glucose transporter appearance amount estimated by the first estimating means and the glucose concentration in the blood.

3. The insulin resistance evaluation supporting system according to claim 2, wherein the first estimating means comprises:

   a third estimating means that estimates a fatty acyl-coenzyme A complex concentration based on the information relating to the free fatty acid concentration in the blood; and
   a fourth estimating means that estimates the value according to the glucose transporter appearance amount based on the insulin concentration in the blood and the fatty acyl-coenzyxne A complex concentration estimated by the third estimating means.

4. The insulin resistance evaluation supporting system according to claim 3, wherein the estimating means further comprises:

   a fifth estimating means that estimates an intracellular pyruvic acid concentration based on the glucose uptake rate estimated by the second estimating means;
   a sixth estimating means that estimates an intracellular acetyl coenzyme concentration based on the fatty acyl-coenzyme A complex concentration estimated by the third estimating means and the pyruvic acid concentration estimated by the fifth estimating means; and
   an adjusting means that adjusts a production rate of an acetyl coenzyme produced from a fatty acyl-coenzyme A complex based on the acetyl coenzyme concentration estimated by the sixth estimating means; and

   the sixth estimating means again estimates the acetyl coenzyme concentration based on the production rate of the acetyl coenzyme after the adjustment by the adjusting means.

5. The insulin resistance evaluation supporting system according to any one of claims 2 to 4, wherein the input means further receives input of information relating to a muscle amount of the subject, the second estimating means estimates a glucose uptake rate per unit muscle amount of the subject based on the value according to the glucose transporter appearance amount estimated by the first estimating means and the glucose concentration in the blood, and the estimating means estimates a glucose uptake rate per unit weight of the subject based on the glucose uptake rate per unit muscle amount of the subject estimated by the second estimating means and the information relating to the muscle amount of the subject whose input has been received by the input means.

6. The insulin resistance evaluation supporting system according to any one of claims 1 to 5, wherein the estimating means repeats a process that, in production of a plurality or substances relating to a change from glucose to pyruvic acid in a living body, acquires amounts of the substances produced in a specific period of time based on concentrations of the substances before the production, acquires concentrations of the substances after the specific period of time by reflecting the amounts of the respective substances produced on the concentrations of the substances before the production, and estimates the glucose uptake rate based on the acquired concentrations of the substances.

7. The insulin resistance evaluation supporting system according to claim 6, wherein the estimating means determines whether or not the glucose uptake rate reaches a steady state, and repeats the process that acquires the concentrations of the substances until it is determined that the glucose uptake rate reaches a steady state.

8. The insulin resistance evaluation supporting system according to claim 6 or 7,
wherein the input means receives input of information relating to a glucose concentration, an insulin concentration, and a free fatty acid concentration in blood in a fasted state of the subject, and
the estimating means comprises:

a first glucose uptake rate estimating means that acquires concentrations of the substances in the fasted state based on the information relating to the glucose concentration, the insulin concentration, and the free fatty acid concentration in the blood whose input has been received by the input means, and estimates a glucose uptake rate in the fasted state based on the acquired concentrations of the substances; and
a second glucose uptake rate estimating means that acquires concentrations of the substances when the insulin concentration is at a predetermined value, based on the concentrations of the substances in the fasted state acquired by the first glucose uptake rate estimating means, and estimates a glucose uptake rate when the insulin concentration is at the predetermined value, based on the acquired concentrations of the substances.

9. The insulin resistance evaluation supporting system according to any one of claims 1 to 8, further comprising an output means that outputs the glucose uptake rate estimated by the estimating means.

10. The insulin resistance evaluation supporting system according to any one of claims 1 to 8, further comprising:

an insulin resistance estimating means that estimates insulin, resistance of the subject based on information relating to the glucose uptake rate estimated by the estimating means; and
an output means that outputs a result of the estimation by the insulin resistance estimating means.

11. An insulin resistance evaluation supporting method for supporting evaluation of insulin resistance of a subject using a computer provided with an input device, comprising the steps of:

receiving, using the input device, input of information relating to a glucose concentration, an insulin concentration, and a free fatty acid concentration in blood obtained by measuring the subject; and
estimating, using the computer, a glucose uptake rate of the subject based on the information relating to the glucose concentration, the insulin concentration, and the free fatty acid concentration in the blood whose input has been received using the input device.

12. The insulin resistance evaluation supporting method according to claim 11, wherein the step of estimating a glucose uptake rate of the subject comprises the steps of:

estimating, using the calculating portion, a value according to a glucose transporter appearance amount based on the information relating to the insulin concentration and the free fatty acid concentration in the blood; and
estimating, using the computer, a glucose uptake rate based on the estimated value according to the glucose transporter appearance amount and the glucose concentration in the blood.

13. A computer program for causing a computer provided with an input device to execute the steps of:

receiving, using the input device, input of information relating to a glucose concentration, an insulin concentration, and a free fatty acid concentration in blood obtained by measuring a subject; and
estimating a glucose uptake rate of the subject based on the glucose concentration, the insulin concentration, and the free fatty acid concentration in the blood whose input has been received using the input means and the glucose concentration in the blood of the subject.

14. The computer program according to claim 13, wherein, in the step of estimating a glucose uptake rate of the subject, the computer is further caused to execute the steps of:

estimating a value according to a glucose transporter appearance amount based on the information relating to the insulin concentration and the free fatty acid concentration in the blood; and
estimating a glucose uptake rate based on the estimated value according to the glucose transporter appearance amount and the glucose concentration in the blood.

FIG. 1

FIG. 2

Glucose <Blood>          Insulin <Blood>

23

**GLUT4 Glycolysis system**

Input glucose to glycolysis block according to glucose transporter (GLUT4) appearance amount
Cause glucose to decompose to give pyruvic acid, and output pyruvic acid to mitochondria in glycolysis system

Insulin receptor

**Insulin signaling**

Adjust glucose transporter (GLUT4) appearance amount according to amount of insulin binding to insulin receptor and concentration of metabolite (FAC) of fatty acid metabolism

22

14a

**Mitochondria**

Oxidize pyruvic acid and FAC to give ACoA, and consume ACoA in TCA cycle
Suppress fatty acid oxidization according to ACoA concentration

**Fatty acid metabolism**

Produce fatty acyl-coenzyme A complex (FAC) from free fatty acid (FFA)
Produce TG via DAG from free fatty acid (FFA)
Cause TG to decompose to give FFA
Output FAC to mitochondria

21

Free fatty acid <Blood>

24

Amount of oxygen consumed in skeletal muscle or whole body
(expired gas analysis or body composition monitor)

EP 2 335 599 A1

FIG. 3

Glucose <Blood>

Insulin <Blood>

GLUT4

Insulin receptor

Insulin signaling

Glucose

Adjust GLUT4 appearance amount
according to amount of insulin binding
to insulin receptor and concentration
of FAC in fatty acid metabolism

Pyruvic acid

Pyruvic acid

TG

ACoA ← FAC ←

FAC    DAG

TCA cycle

Free fatty acid

Free fatty acid
<Blood>

23

22

14a

21

24

Amount of oxygen consumed in skeletal muscle or whole body
(expired gas analysis or body composition monitor)

EP 2 335 599 A1

## FIG. 4

```
              ┌──────────────┐
              │    Start     │
              └──────┬───────┘
                     │
                     ▼           S1
              ┌──────────────────┐
              │Display input screen│
              └──────┬───────────┘
                     │
                     ▼                        S2
       ┌─────────────────────────────────────┐
       │ Receive biological information and instruction│
       │   to execute insulin resistance estimation    │
       └──────────────────┬──────────────────┘
                          │
                          ▼            S3
          ┌─────────────────────────┐
          │     First glucose uptake │
          │    rate estimating process│
          └───────────┬─────────────┘
                      │
                      ▼               S4
          ┌─────────────────────────┐
          │    Second glucose uptake │
          │   rate estimating process│
          └───────────┬─────────────┘
                      │
                      ▼               S5
          ┌─────────────────────────┐
          │ Calculate muscle amount of subject│
          └───────────┬─────────────┘
                      │
                      ▼                  S6
       ┌───────────────────────────────────┐
       │ Calculate glucose uptake rate per unit weight│
       └──────────────┬────────────────────┘
                      │
                      ▼                    S7
     ┌───────────────────────────────────────┐
     │ Estimate presence or absence of insulin resistance│
     └──────────────┬────────────────────────┘
                    │
                    ▼                        S8
   ┌─────────────────────────────────────────────┐
   │Display output screen of insulin resistance estimation result│
   └─────────────────┬───────────────────────────┘
                     │
                     ▼
              ┌──────────────┐
              │     End      │
              └──────────────┘
```

FIG. 5

```
        ┌─────────────────────────┐
        │  First glucose uptake rate │
        │    estimating process      │
        └─────────────────────────┘
                    │
                    ▼           S301
              ┌──────────────┐
              │ Initialization │
              └──────────────┘
                    │
    ┌──────────────▶│
    │         ┌──────────────────────────┐   S302
    │         │  Store glucose uptake rate │
    │         │ at that time as previous value │
    │         └──────────────────────────┘
    │                 │
    │         ┌──────────────────────────┐   S303
    │         │ Fatty acid metabolism block │
    │         │    calculation process     │
    │         └──────────────────────────┘
    │                 │
    │         ┌──────────────────────────┐   S304
    │         │  Insulin signaling block   │
    │         │    calculation process     │
    │         └──────────────────────────┘
    │                 │
    │         ┌──────────────────────────┐   S305
    │         │     Glycolysis block       │
    │         │    calculation process     │
    │         └──────────────────────────┘
    │                 │
    │         ┌──────────────────────────┐   S306
    │         │   Mitochondria block       │
    │         │    calculation process     │
    │         └──────────────────────────┘
    │                 │
    │         ┌──────────────────────────┐   S307
    │         │      Supplemental          │
    │         │   calculation process      │
    │         └──────────────────────────┘
    │                 │
    │                 ▼            S308
    │         ╱─────────────────╲
    │  NO    ╱   Glucose uptake   ╲
    └───────╱ rate reaches steady  ╲
            ╲      state?          ╱
             ╲───────────────────╱
                    │ YES
                    ▼
              ┌──────────┐
              │  Return  │
              └──────────┘
```

# FIG. 6

```
      ┌─────────────────────────┐
     ( Second glucose uptake     )
      \ rate estimating process /
       └───────────┬───────────┘
                   ▼
      ┌─────────────────────────┐      S401
      │ Set insulin concentration│    ╱
      │ to predetermined value   │
      └───────────┬─────────────┘
    ┌────────────►│
    │             ▼
    │  ┌─────────────────────────┐     S402
    │  │ Store glucose uptake rate│   ╱
    │  │ at that time as previous │
    │  │ value                    │
    │  └───────────┬─────────────┘
    │              ▼
    │  ┌─────────────────────────┐     S403
    │  │ Fatty acid metabolism    │   ╱
    │  │ block calculation process│
    │  └───────────┬─────────────┘
    │              ▼
    │  ┌─────────────────────────┐     S404
    │  │ Insulin signaling block  │   ╱
    │  │ calculation process      │
    │  └───────────┬─────────────┘
    │              ▼
    │  ┌─────────────────────────┐     S405
    │  │ Glycolysis block         │   ╱
    │  │ calculation process      │
    │  └───────────┬─────────────┘
    │              ▼
    │  ┌─────────────────────────┐     S406
    │  │ Mitochondria block       │   ╱
    │  │ calculation process      │
    │  └───────────┬─────────────┘
    │              ▼
    │     ┌──────────────────┐         S407
    │     │ Supplemental     │        ╱
    │     │ calculation      │
    │     │ process          │
    │     └────────┬─────────┘
    │              ▼
    │            ◇◇◇◇◇                 S408
    │  NO    ◇◇ Glucose ◇◇            ╱
    └───────◇ uptake rate  ◇
            ◇ reaches steady ◇
             ◇◇  state?  ◇◇
                   │YES
                   ▼
            ┌─────────────┐
           (   Return      )
            └─────────────┘
```

FIG. 7

FIG. 8

4

Insulin resistance estimation result — 41

It is estimated that the insulin resistance is present.

Estimated glucose uptake rate: 13.2mg/kg/min

42

FIG. 9

EP 2 335 599 A1

FIG. 10

EP 2 335 599 A1

## FIG. 11

Start

Display input screen — S21

Receive biological information and instruction to execute insulin resistance estimation — S22

First glucose uptake rate estimating process — S23

Second glucose uptake rate estimating process — S24

Estimate presence or absence of insulin resistance — S25

Display output screen of insulin resistance estimation result — S26

End

FIG. 12

FIG. 13

```
         ┌─────────────────────────┐
         │ First glucose uptake rate│
         │   estimating process     │
         └─────────────────────────┘
                     │
                     ▼           S231
              ┌──────────────┐
              │Initialization│
              └──────────────┘
                     │
      ┌──────────────┤
      │              ▼
      │   ┌────────────────────────┐
      │   │  Store glucose uptake  │  S232
      │   │ rate at that time as   │
      │   │    previous value      │
      │   └────────────────────────┘
      │              │
      │              ▼
      │   ┌────────────────────────┐
      │   │ Fatty acid metabolism  │  S233
      │   │ block calculation      │
      │   │      process           │
      │   └────────────────────────┘
      │              │
      │              ▼
      │   ┌────────────────────────┐
      │   │ Insulin signaling block│  S234
      │   │  calculation process   │
      │   └────────────────────────┘
      │              │
      │              ▼
      │   ┌────────────────────────┐
      │   │   Glycolysis block     │  S235
      │   │  calculation process   │
      │   └────────────────────────┘
      │              │
      │              ▼
      │   ┌────────────────────────┐
      │   │  Mitochondria block    │  S236
      │   │  calculation process   │
      │   └────────────────────────┘
      │              │
      │              ▼
      │   ┌────────────────────────┐
      │   │    Supplemental        │  S237
      │   │ calculation process    │
      │   └────────────────────────┘
      │              │
      │              ▼         S238
      │         ╱──────────╲
      │  NO    ╱Glucose uptake╲
      └───────◄ rate reaches   ►
              ╲steady state?  ╱
               ╲──────────╱
                    │ YES
                    ▼
              ┌──────────┐
              │  Return  │
              └──────────┘
```

# FIG. 14

Second glucose uptake
rate estimating process

Set insulin concentration
to predetermined value — S241

Store glucose uptake rate
at that time as previous value — S242

Fatty acid metabolism block
calculation process — S243

Insulin signaling block
calculation process — S244

Glycolysis block
calculation process — S245

Mitochondria block
calculation process — S246

Supplemental
calculation process — S247

Glucose uptake
rate reaches steady
state? — S248

NO

YES

Return

FIG. 15

FIG. 16

FIG. 17

FIG. 18

FIG. 19

FIG. 20

## INTERNATIONAL SEARCH REPORT

| International application No. |
|---|
| PCT/JP2009/003002 |

A. CLASSIFICATION OF SUBJECT MATTER
*A61B10/00*(2006.01)i, *A61B5/00*(2006.01)i, *G06Q50/00*(2006.01)i

According to International Patent Classification (IPC) or to both national classification and IPC

B. FIELDS SEARCHED

Minimum documentation searched (classification system followed by classification symbols)
A61B10/00, A61B5/00, G06Q50/00

Documentation searched other than minimum documentation to the extent that such documents are included in the fields searched
Jitsuyo Shinan Koho        1922-1996   Jitsuyo Shinan Toroku Koho   1996-2009
Kokai Jitsuyo Shinan Koho   1971-2009   Toroku Jitsuyo Shinan Koho   1994-2009

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X | JP 2006-318162 A  (Sysmex Corp.), 24 November, 2006 (24.11.06), Full text; all drawings & US 2006/0265136 A1    & EP 1724698 A2 & CN 1862551 A | 1,9,10,11,13 |
| X | JP 2007-200093 A  (Sysmex Corp.), 09 August, 2007 (09.08.07), Full text; all drawings & US 2007/0179771 A1    & EP 1830333 A1 & CN 101008965 A | 1,9,10,11,13 |
| X | JP 2006-318128 A  (Sysmex Corp.), 24 November, 2006 (24.11.06), Full text; all drawings & US 2006/0282245 A1    & EP 1722344 A1 & CN 1860990 A | 1,9,10,11,13 |

☒ Further documents are listed in the continuation of Box C.          ☐ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 06 August, 2009 (06.08.09) | 25 August, 2009 (25.08.09) |

| Name and mailing address of the ISA/ Japanese Patent Office | Authorized officer |
|---|---|
| Facsimile No. | Telephone No. |

Form PCT/ISA/210 (second sheet) (April 2007)

**INTERNATIONAL SEARCH REPORT**

International application No.

PCT/JP2009/003002

| C (Continuation).  DOCUMENTS CONSIDERED TO BE RELEVANT | | |
|---|---|---|
| Category* | Citation of document, with indication, where appropriate, of the relevant passages | Relevant to claim No. |
| A | JP 2007-136009 A  (Sysmex Corp.),<br>07 June, 2007 (07.06.07),<br>Full text; all drawings<br>& US 2007/0118347 A1    & EP 1788540 A1<br>& CN 101015445 A | 1-14 |
| A | WO 2006/132899 A2  (MEDTRONIC MINIMED, INC.),<br>14 December, 2006 (14.12.06),<br>Full text; full text<br>& JP 2008-545489 A      & US 2006/0272652 A1<br>& US 2007/0033074 A1    & US 2008/0071580 A1<br>& EP 1886241 A | 1-14 |
| A | WO 2007/005170 A2  (MEDTRONIC MINIMED, INC.),<br>11 January, 2007 (11.01.07),<br>Full text; all drawings<br>& JP 2009-500744 A      & US 2007/0016449 A1<br>& US 2009/0018779 A1    & US 2009/0030733 A1<br>& US 2009/0150186 A      & EP 1899879 A | 1-14 |

Form PCT/ISA/210 (continuation of second sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2006304833 A

**Non-patent literature cited in the description**

- **Dash RK ; Dibella JA 2nd ; Cabrera ME.** *Biorned Eng Online,* 20 April 2007, vol. 6, 14
- **Dash RK ; Li Y ; Kim J ; Beard DA ; Saidel GM ; Cabrera ME.** *PLoS ONE,* 09 September 2008, vol. 3 (9), e3168
- **Sedaghat AR ; Sherman A ; Quon MJ.** *Am J Physiol Endocrinol Metab.,* November 2002, vol. 283 (5), E1084-101
- **Liao Y ; Kwon S ; Shaughnessy S ; Wallace P ; Hutto A ; Jenkins AJ ; Klein RL ; Garvey WT.** *Diabetes Care,* April 2004, vol. 27 (4), 978-83
- **Basu et al.** Obesity and Type 2 Diabetes Impair Insulin-Induced Suppression of Glycogenolysis as well as gluconeogenesis. *Diabetes,* 2005, vol. 54, 1942-1948